# EUROPEAN PATENT SPECIFICATION

(11) **EP 1 901 618 B1**
(45) Date of publication and mention of the grant of the patent: **17.06.2009**
(21) Application number: 06777471.1
(22) Date of filing: 27.06.2006
(51) Int. Cl.: A23L 1/226, C07C 49/00

(54) **HYDROXYPHENYLALKADIONES AND THEIR USE FOR MASKING BITTER TASTE AND/OR FOR INTENSIFYING SWEET TASTE**
HYDROXYPHENYLALKADIONE UND DEREN VERWENDUNG ZUR ÜBERDECKUNG VON BITTEREM GESCHMACK UND/ODER ZUR VERSTÄRKUNG VON SÜSSEM GESCHMACK
HYDROXYPHÉNYLALCADIONES ET LEUR UTILISATION POUR MASQUER UN GOÛT AMER ET/OU POUR INTENSIFIER UN GOÛT SUCRÉ

(30) Priority: 05.07.2005 US 696670 P
(43) Date of publication of application: 26.03.2008
(73) Proprietor: Symrise GmbH & Co. KG, 37603 Holzminden (DE)
(72) Inventor: LEY, Jakob, 37603 Holzminden (DE); BERTRAM, Heinz-Jürgen, 37603 Holzminden (DE); KRAMMER, Gerhard, 37603 Holzminden (DE); KINDEL, Günter, 37671 Höxter (DE)
(74) Representative: Eisenführ, Speiser & Partner
(86) International application number: PCT/EP2006/063576
(87) International publication number: WO 2007/003527

(56) References cited:
- EP-A1- 0 245 825
- WO-A1-20/06024587
- DE-A1- 2 161 528
- US-A- 5 336 513
- US-A- 5 637 618
- PATENT ABSTRACTS OF JAPAN & JP 2003 052331 A (OGAWA & CO LTD), 25 February 2003 (2003-02-25)

## Description

The invention relates to the use of certain hydroxyphenylalkadione derivatives of the general formula (I), salts thereof and mixtures thereof for modifying, masking or reducing unpleasant taste impressions, in particular bitter, astringent and/or metallic taste impressions, and/or for intensifying the sweet taste of sweet-tasting substances or the sweet odour impression of aroma substances which cause a sweet odour impression. The invention furthermore relates to certain formulations which comprise an active content of the hydroxyphenylalkadione derivatives described, salts thereof or mixtures thereof.

The invention was developed in two stages. Due to this development history, the present text is divided into two parts. Part **A** describes the invention before the further development, and part **B** is a supplementary description of the invention after the further development had taken place. However, aspects described only in part **A** of course are also valid in respect of the invention after the further development had taken place. Aspects from part **B** can also be combined with aspects from part **A,** in particular in respect of preferred embodiments of the invention.

### Part A:

The invention relates to the use of certain hydroxyphenylalkadione derivatives, salts thereof and mixtures thereof for masking or reducing unpleasant taste impressions, in particular bitter, astringent and/or metallic taste impressions. The invention furthermore relates to certain formulations which comprise an active content of the hydroxyphenylalkadione derivatives described, salts thereof or mixtures thereof.

Foodstuffs or compositions for consumption for pleasure often comprise various bitter principles, which indeed on the one hand are desirable and characteristic in moderation (e.g. caffeine in tea or coffee, quinine in so-called bitter lemon drinks, hop extracts in beer), but on the other hand can also greatly reduce the value (e.g. flavonoid glycosides and limonoids in citrus juices, the bitter after-taste of many artificial sweeteners, such as aspartame or saccharin, hydrophobic amino acids and/or peptides in cheese).

A subsequent treatment is therefore often necessary to lower the natural content of bitter principles, for example by extraction, as in the decaffeination of tea or coffee, or enzymatically, e.g. treatment of orange juice with a glycosidase to destroy the bitter naringin or the use of specific peptidases in the maturation of cheese. This treatment is a burden for the product, generates waste and e.g. also causes solvent residues and other residues (enzymes) in the products.

It is therefore desirable to discover substances which can effectively suppress or at least reduce unpleasant taste impressions, in particular, bitter, astringent and/or metallic taste impressions.

Suppression of the bitter taste is particularly important in the case of many pharmaceutical active compoundss, since the willingness of the patient, in particular in the case of patients sensitive to bitterness, such as children, to take the formulation orally can thereby be increased significantly. Many pharmaceutical active compounds, for example aspirin, salicin, paracetamol, ambroxol or quinine, to name only a very small selection for illustration, have a pronounced bitter, astringent and/or metallic taste and/or after-taste.

Some substances which can partly suppress bitter taste are indeed known, but many show severe limitations in use.

In US 5,637,618, a bitter taste is reduced with the aid of lactisol [20-(4-methoxyphenyl)lactic acid]. However, this inhibitor at the same time shows a marked inhibition of the sweet taste impression (cf. US 5,045,336), which severely restricts usability.

2,4-Dihydroxybenzoic acid potassium salt is described in US 5,643,941 (Table column 3, line 18) as a masking agent for the bitter taste of potassium chloride, but cannot suppress e.g. the taste of caffeine.

According to GB 2,380,936, suppression of the taste of bitter pharmaceuticals is achieved with ginger extract. Needless to say, the strong aroma impression and/or the piquancy of ginger extracts which is often to be found therein or constituents therefrom which have a piquant action is not suitable for a large number of uses.

Neohesperidin dihydrochalcone also shows a bitter-reducing effect, but is above all a sweetener (cf. Manufacturing Chemist 2000, July issue, p. 16-17), which also causes trouble in non-sweet uses.

Taste-modifying properties are indeed described for some flavones (2-phenylchrom-2-en-4-ones) in US-A 5,580,545, but a bitter-reducing or -suppressing action was not found.

US 2002 177,576 describes the suppression of a bitter taste by nucleotides, for example cytidine 5'-monophosphates (CMP). However, the strongly polar compounds, which therefore can be used only in strongly polar solvents, can be used to only a very limited degree in many fat-containing foodstuffs. Furthermore, the availability of such substances is severely limited because of their expensive chemical synthesis.

US 2002 188,019 describes hydroxyflavanones as active bitterness maskers which, however, are accessible by synthesis only with difficulty and are not available inexpensively in larger amounts.

The sodium salts sodium chloride, sodium citrate, sodium acetate and sodium lactate show a bitterness-masking effect against many bitter principles (e.g. Nature, 1997, vol. 387, p. 563); needless to say, intake of relatively large amounts of sodium ions can lead e.g. to cardiovascular diseases. Furthermore, a significant bitterness-masking action disadvantageously occurs only at relatively high sodium concentrations (from approx. 0.1 M), which corresponds e.g. to an as a rule unacceptably high content of approx. 0.6 wt.% NaCl in the end use (cf. R.S.J. Keast, P.A.S. Breslin and G.K. Beauchamp, Chimia 2001, 55(5), 441-447).

WO 00/21390 describes polyglutamic acid as a bitterness-masking agent; relatively high concentrations in the region of about 1 wt.% are required here.

A lipoprotein comprising β-lactoglobulin and phosphatidic acid also shows a bitterness-masking effect (EP-A 635 218). Needless to say, such polymers are difficult to characterize and standardize and show a decidedly soapy secondary taste.

The flavone glycoside neodiosmin [5,7-dihydroxy-2-(4-methoxy-3-hydroxyphenyl)-7-O-neohesperidoxyl-chrom-2-en-4-one] likewise shows a bitterness-masking action (US-A 4,154,862), but is distinguished by a disaccharide radical which makes the preparation or isolation and usability of the substance very difficult.

It was the primary object of the present invention to discover (a) substances which are suitable for masking or reducing the unpleasant taste impression of unpleasantly tasting substances (and preferably in particular show a bitterness-masking effect against a large number of bitter principles), (b) can be used widely and (c) are readily accessible.

According to the invention, the object described is achieved by the use of a compound of the formula (I) 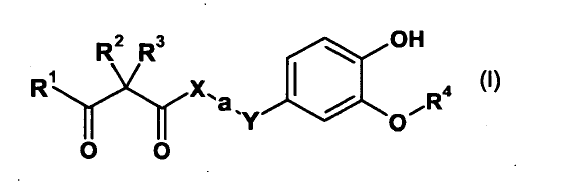 that is to say a hydroxyphenylalkadione derivative,
wherein, for X, a and Y:
X is a -CH₂-, -NH- or -O- group,
Y a is a -CH₂- group and
a is a single bond
or
X and Y are in each case a -CH- group and
a is a double bond in the Z or E configuration,
wherein, for R¹, R² and R³
- R¹: denotes hydrogen, an aliphatic radical having 1 to 4 C atoms or a -O-R⁵, -S-R⁵ or -NR⁵R⁶ group and
- R² and R³: independently of one another denote hydrogen or an alkyl having 1, 2, 3, 4 or 5 C atoms which is optionally substituted by one or more oxo groups or R⁷-O- groups
or
- R¹ and R²: together form an aliphatic ring which contains a total of 4 to 10 carbon atoms and 0 or 1 oxygen atom, wherein the ring constituent represented by R¹ and R² is optionally substituted by one or more oxo groups and/or R⁷-O- groups and/or aliphatic radicals having 1 to 4 C atoms and
- R³: denotes hydrogen or an alkyl having 1, 2, 3, 4 or 5 C atoms which is optionally substituted by one or more oxo groups or R⁷-O- groups
or
- R² and R³: together form an aliphatic ring which contains a total of 4 to 10 carbon atoms and 0 or 1 oxygen atom, wherein the ring constituent represented by R² and R³ is optionally substituted by one or more oxo groups and/or R⁷-O- groups and/or aliphatic radicals having 1 to 4 C atoms and
- R¹: denotes hydrogen, an aliphatic radical having 1 to 4 C atoms or a -O-R⁵, -S-R⁵ or -NR⁵R⁶ group,
wherein, for R⁴_{:}
- R⁴: denotes hydrogen, methyl or ethyl
and wherein, for R⁵, R⁶ and R⁷:
- R⁵ R⁶ and R⁷: independently of one another denote hydrogen or an aliphatic radical having 1 to 4 C atoms,
salts thereof and mixtures thereof for masking or reducing the unpleasant taste impression of an unpleasantly tasting substance, that is to say as a flavour correctant.

The aliphatic radical having 1 to 4 C atoms which is optionally present in R¹, R⁵, R⁶ and/or R⁷ preferably denotes methyl, ethyl, 1-propyl or 2-propyl. If an aliphatic radical having 1 to 4 C atoms is present in several radicals R¹, R⁵, R⁶ and/or R⁷, each of these aliphatic radicals is chosen independently of the others.

If an alkyl having 1, 2, 3, 4 or 5 C atoms which is optionally substituted by one or more oxo groups or R⁷-O- groups is present in the radicals R² and/or R³, this is preferably chosen from the group consisting of: methyl, ethyl, 1-propyl, 2-propyl, 1-oxomethyl, 1-oxoethyl, 1-methyloxymethyl, 1-methyloxyethyl, 1-oxopropyl. If corresponding radicals are present in both groups R² and R³, these are chosen independently of one another.

If R¹ and R² together form an aliphatic ring which is five-, six- or seven-membered in total, this preferably contains 4 to 7 carbon atoms and 0 or 1 oxygen atom. In this context, the ring constituent represented by R¹ and R² is optionally substituted by one or more oxo groups and/or R⁷-O- groups and/or aliphatic radicals having 1 to 4 C atoms. Particularity preferred (part) structures can be seen from the following figure: 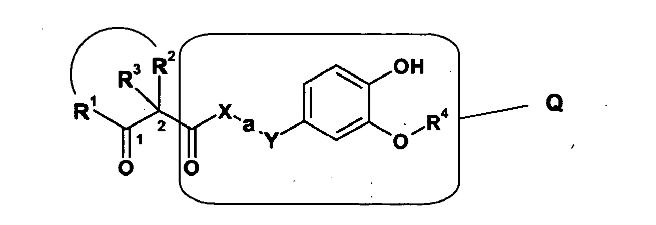 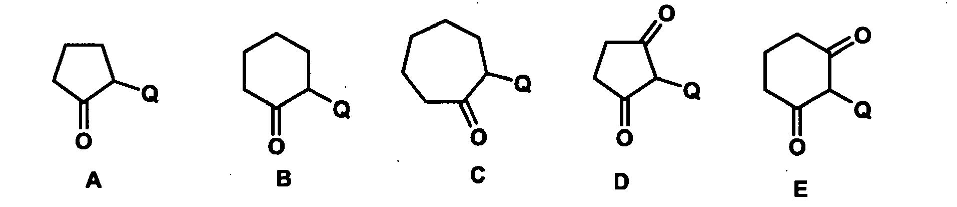 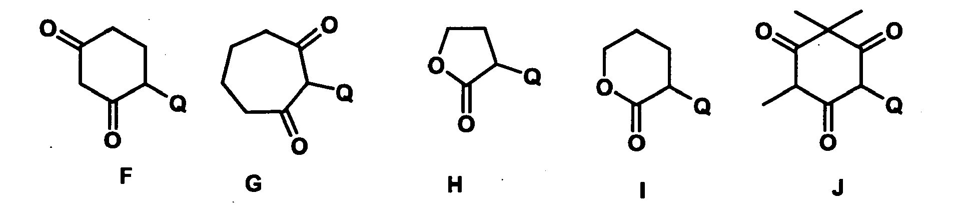 (Part) structures A to J are groupings which are in each case linked with the structural constituent Q via the 2 position. The meaning of the radicals R¹, R² and R³ of the formula (I) within the particular (part) structure A to J can be seen, in particular, by comparison of the particular (part) structure with the formula representation shown above.

The following (part) structures are thus particularly preferred:
- Cyclopentan-1-one (A): R³ = H; R¹ and R² together form a ring of 5 carbon atoms having 5 members in total, wherein the ring constituent represented by R¹ and R² is not substituted.
- Cyclohexan-1-one (B): R¹ and R² together form a an aliphatic ring which contains 6 carbon atoms and has 6 members in total, wherein the ring constituent represented by R¹ and R² is not substituted.
- Cycloheptan-1-one (C): R¹ and R² together form a ring which contains 7 carbon atoms and has 7 members in total, wherein the ring constituent represented by R¹ and R² is not substituted.
- Cyclopentane-1,3-dione (D): R¹ and R² together form an aliphatic ring which contains 5 carbon atoms and has 6 members in total, wherein the ring constituent represented by R¹ and R² is substituted by an oxo group.
- Cyclohexa-1,3-dione (E): R¹ and R² together form an aliphatic ring which contains 6 carbon atoms and has 6 members in total, wherein the ring constituent represented by R¹ and R² is substituted by an oxo group.
- Cyclohexa-1,5-dione (F): R¹ and R² together form an aliphatic ring which contains 6 carbon atoms and has 6 members in total, wherein the ring constituent represented by R¹ and R² is substituted by an oxo group.
- Cycloheptane-1,3-dione (G): R¹ and R² together form a ring which contains 7 carbon atoms and has 7 members in total, wherein the ring constituent represented by R¹ and R² is substituted by an oxo group.
- 5-Oxacyclopentan-1-one (H): R¹ and R² together form a ring which contains 4 carbon atoms and 1 oxygen atom and has 5 members in total, wherein the ring constituent represented by R¹ and R² is not substituted.
- 6-Oxa-cyclohexan-1-one (I): R¹ and R² together form a ring which contains 5 carbon atoms and 1 oxygen atom and has 6 members in total, wherein the ring constituent represented by R¹ and R² is not substituted.
- 4,6,6-Trimethyl-cyclohexane-1,3,5-trione (J): R¹ and R² together form a ring which contains 6 carbon atoms and has 6 members in total, wherein the ring constituent represented by R¹ and R² is substituted by 2 oxo groups and a total of 3 methyl radicals.

The oxo group present in position 1 of the particular (part) structure does not belong to the ring constituent represented by R¹ and R² and is present in every compound of the formula (I) to be employed according to the invention.

Unpleasantly tasting substances in the context of the invention are:
(a) substances which taste bitter, astringent, cardboardy, dusty, dry, floury, rancid and/or metallic and
(b) substances which have a bitter, astringent, cardboardy, dusty, dry, floury, rancid or metallic after-taste.

The abovementioned unpleasantly tasting substances can have still further as a rule not unpleasant taste and/or odour qualities. As further taste qualities which are not unpleasant in the sense of the present invention there may be mentioned e.g. the spicy, umami, sweet, salty, acid, piquant, cooling, warming, burning or tingling impressions.

Substances which taste bitter, astringent, cardboardy, dusty, dry, floury, rancid and/or metallic are, for example: xanthine alkaloids, xanthines (caffeine, theobromine, theophylline), alkaloids (quinines, brucine, strychnine, nicotine), phenolic glycosides (e.g. salicin, arbutin), flavonoid glycosides (e.g. hesperidin, naringin), chalcones or chalcone glycosides, hydrolysable tannins (gallic or ellagic acid esters of carbohydrates, e.g. pentagalloylglucose), non-hydrolysable tannins (optionally galloylated catechols or epicatechols and oligomers thereof, e.g. proanthyocyanidines or procyanidines, thearubigin), flavones (e.g. quercetin, taxifolin, myricetin), other polyphenols (y-oryzanol, caffeic acid or esters thereof), terpenoid bitter principles (e.g. limonoids, such as limonin or nomilin from citrus fruits, lupolones and humolones from hops, iridoids, secoiridoids), absinthe from wormwood, amarogentin from gentian, metallic salts (potassium chloride, sodium sulfate, magnesium sulfate), pharmaceutical active compounds (e.g. fluoroquinolone antibiotics, paracetamol, aspirin, β-lactam antibiotics, ambroxol, propylthiouracil [PROP], guaifenesin), vitamins (for example vitamin H, vitamins from the B series, such as vitamin B1, B2, B6, B12, niacin, pantothenic acid), denatonium benzoate, sucralose octaacetate, potassium chloride, magnesium salts, iron salts, aluminium salts, zinc salts, urea, unsaturated fatty acids, in particular unsaturated fatty acids in emulsions, amino acids (e.g. leucine, isoleucine, valine, tryptophan, proline, histidine, tyrosine, lysine or phenylalanine), peptides (in particular peptides with an amino acid from the group consisting of leucine, isoleucine, valine, tryptophan, proline or phenylalanine on the N or C terminus).

Substances which have a bitter, astringent, cardboardy, chalky, dusty, dry, floury, rancid or metallic after-taste can be aroma or flavouring substances having a not unpleasant primary taste (for example sweet, salty, spicy, acid) and/or odour and belong e.g. to the group consisting of sweeteners, sugar substitutes or the aroma substances. Examples which may be mentioned are: aspartame, neotame, superaspartame, saccharin, sucralose, tagatose, monellin, stevioside, rebaudioside, hemandulcin, thaumatin, miraculin, glycyrrhizin, glycyrrhetic acid or derivatives thereof, cyclamate or the pharmaceutically acceptable salts of the abovementioned compounds.

A particularly preferred use according to the invention of a compound of the formula (I) is that
wherein, for X, a and Y:
X is a -CH₂- or -NH- group,
Y is a -CH₂- group and
a is a single bond
or
X and Y are in each case a -CH- group and
a is a double bond in the E configuration,
wherein, for R¹, R² and R³
- R¹: denotes an aliphatic radical having 1 to 4 C atoms or a -O-R⁵ group,
- R²: denotes hydrogen or an alkyl having 1, 2, 3, 4 or 5 C atoms which is optionally substituted by one or more oxo groups or R⁷-O- groups and
- R³: denotes hydrogen,
or
- R¹ and R²: together form an aliphatic ring which contains a total of 5 to 7 carbon atoms and 0 or 1 oxygen atom, wherein the ring constituent represented by R¹ and R² is optionally substituted by one or more oxo groups and/or R⁷-O- groups and
- R³: denotes hydrogen
wherein, for R⁴:
- R⁴: denotes hydrogen, methyl or ethyl
and wherein, for R⁵ and R⁷:
- R⁵ and R⁷: independently of one another denote hydrogen or an aliphatic radical having 1 to 4 C atoms.

Corresponding statements of course also apply to the corresponding salts and mixtures.

A particularly preferred use of compounds of the formula (I) is that
wherein, for X, a and Y:
X is a -CH₂- group,
Y is a -CH₂- group and
a is a single bond
or
X and Y are in each case a -CH- group and
a is a double bond in the E configuration,
wherein, for R¹, R² and R³
- R¹: denotes methyl, ethyl, 1-propyl, 2-propyl or a -O-R⁵ group and
- R²: denotes hydrogen or an alkyl having 1, 2, 3, 4 or 5 C atoms which is optionally substituted by one or more oxo groups and
- R³: denotes hydrogen,
or
- R¹ and R²: together form an aliphatic ring which contains a total of 5 to 7 carbon atoms and 0 or 1 oxygen atom, wherein the ring constituent represented by R¹ and R² is optionally substituted by one or more oxo groups and
- R³: denotes hydrogen.
wherein, for R⁴:
- R⁴.: denotes hydrogen or methyl
and wherein, for R⁵:
- R⁵: denotes hydrogen, methyl or ethyl.

Compounds which are particularly preferably used are
(5*E*)-6-(4-hydroxy-3-methoxyphenyl)hex-5-ene-2,4-dione (compound 1)
(6*E*)-7-(4-hydroxy-3-methoxyphenyl)hept-6-ene-3,5-dione (compound 2)
(5*E*)-6-(3,4-dihydroxyphenyl)hex-5-ene-2,4-dione ("hispolone", compound 3)
6-(4-hydroxy-3-methoxyphenyl)hexane-2,4-dione (compound 4)
7-(4-hydroxy-3-methoxyphenyl)heptane-3,5-dione (compound 5)
5-(4-hydroxy-3-methoxyphenyl)pentan-3-onoic acid (compound 6)
5-(4-hydroxy-3-methoxyphenyl)pentan-3-onoic acid ethyl ester (compound 7)
2-acetyl-5-(4-hydroxy-3-methoxyphenyl)pentan-3-onoic acid ethyl ester (compound 8)
2-(3-[4-hydroxy-3-methoxyphenyl]propionyl)-cyclopentane-1,3-dione (compound 9),
salts thereof and mixtures thereof for masking or reducing the unpleasant taste impression of an unpleasantly tasting substance.

The structures of the preferred compounds (1) - (9) are given below for illustration.

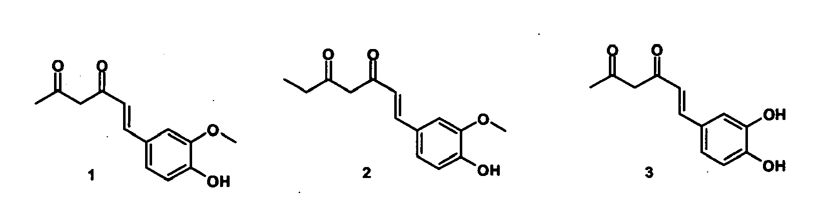

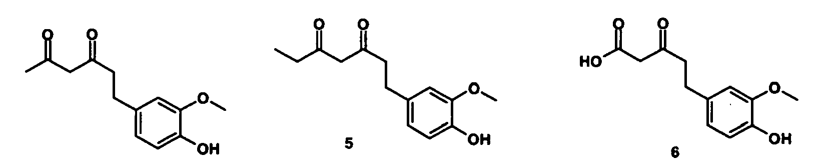

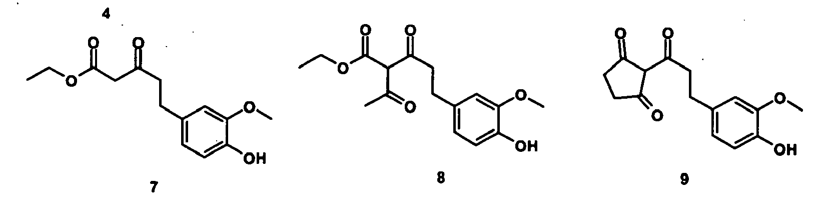

In salts of a compound of the above formula (I) (wherein that stated above continues to apply in respect of the preferred meanings of the radicals and variables) to be used according to the invention, one, several or all of the hydroxyl groups of compound (I) are deprotonated. A corresponding amount of counter-cations is then present, these preferably being chosen from the group consisting of: singly positively charged cations of the first main and sub-group, ammonium ions, trialkylammonium ions, doubly positively charged cations of the second main and sub-group and triply positively charged cations of the third main and sub-group, and mixtures thereof.

It goes without saying that the number of hydroxyl groups in the underlying hydroxyphenylalkadione derivative is decisive for the maximum degree of deprotonation and thus also for the amount of counter-cations present. For example, if two hydroxyl groups in total are present in the underlying hydroxyphenylalkadione derivative, in the case of complete deprotonation of the hydroxyl groups a doubly negatively charged anion is present, so that a corresponding number of positive charges must be provided by the countercation(s).

Particularly preferred cations are Na⁺, K⁺, NH₄⁺, Ca²⁺, Mg²⁺, Al³⁺ and Zn²⁺.

The various hydroxyphenylalkadione derivatives according to the invention and salts thereof can of course in each case be used according to the invention by themselves or as mixtures.

A preferred use according to the invention is also that in which, in addition to a compound of the formula (I), a corresponding salt or a corresponding mixture, a further substance for modifying, masking or reducing the unpleasant taste impression of an unpleasantly tasting substance is present. The combination of at least two flavour correctants is then present.

According to further particularly preferred embodiment of the invention, the hydroxyphenylalkadione derivatives of the formula (I) to be used according to the invention, their salts or mixtures are used in combination with at least one sweet-tasting substance. The sweet-tasting substance can also be a plant extract.

Sweet-tasting substances (including plant extracts) can be, for example, sweet-tasting carbohydrates (e.g. sucrose, trehalose, lactose, maltose, melicitose, raffinose, palatinose, lactulose, D-fructose, D-glucose, D-galactose, L-rhamnose, D-sorbose, D-mannose, D-tagatose, D-arabinose, L-arabinose, D-ribose, D-glyceraldehyde) sugar alcohols (e.g. erythritol, threitol, arabitol, ribitol, xylitol, sorbitol, mannitol, dulcitol, lactitol), proteins (e.g. miraculin, monellin, thaumatin, curculin, brazzein), sweeteners (magap, sodium cyclamate, acesulfame K, neohesperidin dihydrochalcone, saccharin sodium salt, aspartame, superaspartame, neotame, sucralose, stevioside, rebaudioside, lugduname, carrelame, sucrononate, sucrooctate), certain sweet-tasting amino acids (glycine, D-leucine, D-threonine, D-asparagine, D-phenylalanine, D-tryptophan, L-proline), other sweet-tasting low molecular weight substances (e.g. hernandulcin, dihydrochalcone glycosides, glycyrrhetic acid derivatives), extracts from liquorice *(Glycyrrhizza glabra ssp.),* sugar beet *(Beta vulgaris ssp.),* sugar cane *(Saccharum officinarium ssp.)* or *Stevia ssp.* (e.g. *Stevia rebaudiana).*

The compounds of the formula (I) (hydroxyphenylalkadione derivatives) to be used according to the invention can be, in the case where at least one of the radicals R² or R³ represents a hydrogen atom, in the form of their tautomers, the ratio of amounts of dione tautomer to keto-enol tautomer being determined by intrinsic and extrinsic physicochemical parameters. The possible tautomeric forms are illustrated by an example where R³ = H: 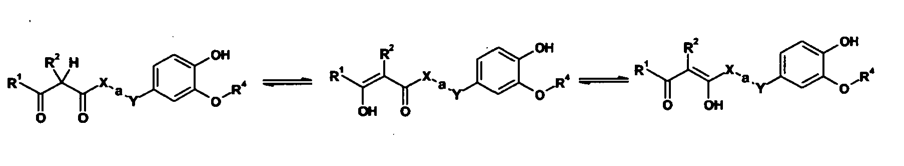 In the context of the present text, a "compound of the formula (I)" is also to be understood as meaning the particular tautomers.

The use according to the invention of the compounds of the formula (I) (hydroxyphenylalkadione derivatives) is novel.

(5*E*)-6-(4-Hydroxy-3-methoxyphenyl)hex-5-ene-2,4-dione (compound 1) was described as an intermediate product for a synthesis in J. Nat. Prod., vol. 61, year 1998, 609 et seq.

The synthesis and the antioxidative action of hispolone (compound 3) from a parasitic fungus was described in Indian Journal of Chemistry, vol. 41B. April 2002, 875 et seq.

6-(4-Hydroxy-3-methoxyphenyl)hexane-2,4-dione (compound 4), (6*E*)-7-(4-hydroxy-3-methoxyphenyl)hept-6-ene-3,5-dione (compound 2), 7-(4-hydroxy-3-methoxyphenyl)heptane-3,5-dione (compound 5) were mentioned in EP 245,825 as an intermediate product for the synthesis of lipoxygenase inhibitors.

The synthesis of 2-(3-[4-hydroxy-3-methoxyphenyl]propionyl)-cyclopentane-1,3-dione (compound 9), which was found to be a weak antihypertensive agent, was described in DE 2 161,528.

A use of the abovementioned compounds as an aroma or flavouring substance or for influencing aroma or taste was not described or suggested in any of the cases mentioned.

The present invention also relates to the novel compounds of the formula (I), as described above, that stated above in respect of the preferred meaning of the radicals and variables applying accordingly. In this respect, the present invention relates in particular to the compounds
5-(4-hydroxy-3-methoxyphenyl)pentan-3-onoic acid (compound 6),
5-(4-hydroxy-3-methoxyphenyl)pentan-3-onoic acid ethyl ester (compound 7),
2-acetyl-5-(4-hydroxy-3-methoxyphenyl)pentan-3-onoic acid ethyl ester (compound 8),
salts thereof and mixtures thereof.

It has been found, surprisingly, that the hydroxyphenylalkadione derivatives of the formula (I) used according to the invention can also reduce or even completely suppress the unpleasant taste impression, in particular, the bitter taste impression, of a large number of substances, in particular of methylxanthines, such as e.g. caffeine, alkaloids, such as e.g. quinine, flavonoids, such as e.g. naringin, phenols, such as e.g. salicin, pharmaceutical active compounds, such as e.g. denatonium benzoate or β-lactam antibiotics, in very low concentrations, it being particularly advantageous that the hydroxyphenylalkadione derivatives used according to the invention have virtually no intrinsic taste and do not adversely influence the further as a rule not unpleasant taste qualities, in particular even positively influence the sweet taste of sweet substances.

As already mentioned, one aspect of the present invention is the use of a hydroxyphenylalkadione derivative of the formula (I) or of a corresponding salt or mixture for masking or reducing the unpleasant taste impression of an unpleasantly tasting substance, i.e. as a flavour correctant. Preferably, the hydroxyphenylalkadione derivative of the formula (I) to be used according to the invention, the salt or the mixture is employed in a formulation for nutrition, oral care or consumption for pleasure or oral pharmaceutical formulation or cosmetic formulation for application in the region of the head, the formulation conventionally comprising one or more unpleasantly tasting substances.

A further aspect of the present invention relates to such formulations. Formulations according to the invention for nutrition, oral care or consumption for pleasure or cosmetic formulations according to the invention for application in the region of the head preferably comprise 0.000001 wt.% to 95 wt.%, based on the total weight of the formulation, of a compound of the formula (I) to be used according to the invention (see above), a corresponding salt or mixture. In addition, an unpleasantly tasting substance is conventionally present.

An oral pharmaceutical formulation according to the invention preferably comprises 0.000001 wt.% to 10 wt.%, based on the total weight of the formulation, of a compound of the formula (I) to be used according to the invention (see above), a corresponding salt or mixture and at least one unpleasantly tasting substance (see the definition given above).

Formulations according to the invention which comprise at least one unpleasantly tasting substance, the amount of the unpleasantly tasting substance being sufficient to be perceived as an unpleasant taste in a comparison formulation which comprises no compound of the formula (I) according to the invention or corresponding salt or mixture but is of otherwise identical composition, and the amount of the compound of the formula (I), salt or mixture in the formulation being sufficient to mask sensorially the unpleasant taste impression of the unpleasantly tasting substance or to reduce it in comparison with the comparison formulation, are particularly relevant.

Formulations according to the invention can be in the form of semi-finished goods, in the form of an odoriferous, aroma or flavouring substance composition or in the form of a spice mixture.

In the context of the invention, the formulations for nutrition or consumption for pleasure are e.g. baked goods (e.g. bread, dry biscuits, cakes, other baked products), confectionery (e.g. chocolate, chocolate bar products, other bar products, fruit gum, hard and soft caramels, chewing gum), alcoholic or non-alcoholic drinks (e.g. coffee, tea, wine, wine-containing drinks, beer, beer-containing drinks, liqueurs, schnapps, brandies, fruit-containing carbonated drinks, isotonic drinks, refreshing drinks, nectars, fruit and vegetable juices, fruit or vegetable juice formulations), instant drinks (e.g. instant cocoa drinks, instant tea drinks, instant coffee drinks), meat products (e.g. ham, fresh sausage or uncooked sausage formulations, seasoned or marinated fresh or salted meat products), eggs or egg products (dried egg, egg white, egg yolk), cereal products (e.g. breakfast cereals, muesli bars, precooked ready-made rice products), dairy products (e.g. milk drinks, milk ice, yoghurt, kefir, fresh cheese, soft cheese, hard cheese, dried milk powder, whey, butter, buttermilk, products containing partly or completely hydrolysed milk protein), products from soya protein or other soya bean fractions (e.g. soya milk and products produced therefrom, soya lecithin-containing formulations, fermented products, such as tofu or tempe or products produced therefrom), fruit formulations (e.g. preserves, fruit-flavoured ice-cream, fruit sauces, fruit fillings), vegetable formulations (e.g. ketchup, sauces, dried vegetables, frozen vegetables, precooked vegetables, cooked vegetables), nibbles (e.g. baked or fried potato crisps or potato paste products, extrudates based on maize or peanuts), fat- and oil-based products or emulsions thereof (e.g. mayonnaise, remoulade, dressings), other ready-made dishes and soups (e.g. dried soups, instant soups, precooked soups), spices, spice mixtures and, in particular, sprinkling spices ( seasonings), which are used, for example, in the snacks sector. The formulations in the context of the invention can also be used as semi-finished goods for the preparation of further formulations for nutrition or consumption for pleasure. The formulations in the context of the invention can also be in the form of capsules, tablets (non-coated and coated tablets, e.g. coatings which are resistant to gastric juice), lacquered tablets, granules, pellets, solid mixtures, dispersions in liquid phases, emulsions, powders, solutions, pastes or other formulations as food supplements which can be swallowed or chewed.

In the context of the invention, formulations for oral care are, in particular, oral and/or dental care compositions, such as toothpastes, tooth gels, tooth powders, mouthwashes, chewing gums and other oral care compositions.

Oral pharmaceutical formulations in the context of the invention are formulations which e.g. are in the form of capsules, tablets (non-coated and coated tablets, e.g. coatings which are resistant to gastric juice), lacquered tablets, granules, pellets, solid mixtures, dispersions in liquid phases, emulsions, powders, solutions, pastes or other formulations which can be swallowed or chewed, and are used as medicaments which are available only on prescription or are available only from pharmacies or other medicaments or as food supplements.

Cosmetic formulations for application in the region of the head are, in particular, those which include an unpleasantly tasting substance and may come into contact with the oral cavity even when applied properly to the skin, that is to say, for example - as already mentioned, cosmetic formulations for application in the region of the head, such as soaps, other cleansing or care compositions for the face region, face creams or lotions or ointments, sunscreen agents, beard cleansing or care compositions, shaving foams, soaps or gels, lipsticks or other lip cosmetics or lip care compositions.

Further conventional active compounds, base substances, auxiliary substances and additives for formulations for nutrition, oral care or consumption for pleasure or oral pharmaceutical formulations can be present in amounts of from 5 to 99.999999 wt.%, preferably 10 to 80 wt.%, based on the total weight of the formulation. The formulations can furthermore contain water in an amount of up to 99.999999 wt.%, preferably 5 to 80 wt.%, based on the total weight of the formulation.

The formulations according to the invention comprising one or more of the compounds of the formula (I) according to the invention or their salts or mixtures are prepared according to a preferred embodiment by incorporating the compounds of the formula (I) or their salts or mixtures as solids, as a solution or in the form of a mixture with a solid or liquid carrier substance, into a base formulation for nutrition, oral care or consumption for pleasure or an oral pharmaceutical base formulation. Advantageously, formulations according to the invention in the form of a solution can also be converted into a solid formulation by spray drying.

According to a further preferred embodiment, for the preparation of formulations according to the invention, the compounds of the formula (I) according to the invention or their salts or mixtures and optionally other constituents of the formulation according to the invention can also be incorporated beforehand into emulsions, into liposomes, e.g. starting from phosphatidylcholine, into microspheres, into nanospheres or also into capsules, granules or extrudates of a matrix which is suitable for foodstuffs and compositions for consumption for pleasure, e.g. of starch, starch derivatives, cellulose or cellulose derivatives (e.g. hydroxypropylcellulose), other polysaccharides (e.g. alginate), natural fats, natural waxes (e.g. beeswax, carnauba wax) or of proteins, e.g. gelatine.

In a further preferred preparation process, the hydroxyphenylalkadione derivatives (compound of the formula (I)) or their salts or mixtures are complexed beforehand with one or more suitable complexing agents, for example with cycloglycans, e.g. cyclofructans, cyclodextrins or cyclodextrin derivatives, preferably α-, γ- and β-cyclodextrin, and employed in this complexed form.

A formulation according to the invention in which the matrix is chosen such that hydroxyphenylalkadione derivatives are released from the matrix in delayed form, so that a long-lasting aroma action is obtained, is particularly preferred.

Further constituents which can be used for formulations according to the invention for nutrition or consumption for pleasure are conventional base substances, auxiliary substances and additives for foodstuffs or compositions for consumption for pleasure, e.g. water, mixtures of fresh or processed, plant or animal base substances or raw materials (e.g. raw, roasted, dried, fermented, smoked and/or boiled meat, bone, cartilage, fish, vegetables, fruit, herbs, nuts, vegetable or fruit juices or pastes or mixtures thereof), digestible or non-digestible carbohydrates (e.g. sucrose, maltose, fructose, glucose, dextrins, amylose, amylopectin, inulin, xylans, cellulose), sugar alcohols (e.g. sorbitol), natural or hydrogenated' fats (e.g. tallow, lard, palm fat, coconut fat, hydrogenated plant fat), oils (e.g. sunflower oil, groundnut oil, maize germ oil, olive oil, fish oil, soya oil, sesame oil), fatty acids or salts thereof (e.g. potassium stearate), proteinogenic or non-proteinogenic amino acids and related compounds (e.g. taurine), peptides, native or processed proteins (e.g. gelatine), enzymes (e.g. peptidases), nucleic acids, nucleotides, flavour correctants for unpleasant taste impressions, flavour correctants for further as a rule not unpleasant taste impressions, taste-modulating substances (e.g. inositol phosphate, nucleotides, such as guanosine monophosphate, adenosine monophosphate or other substances, such as sodium glutamate or 2-phenoxypropionic acid), emulsifiers (e.g. lecithins, diacylglycerols), stabilizers (e.g. carrageenan, alginate), preservatives (e.g. benzoic acid, sorbic acid), antioxidants (e.g. tocopherol, ascorbic acid), chelating agents (e.g. citric acid), organic or inorganic acidifying agents (e.g. malic acid, acetic acid, citric acid, tartaric acid, phosphoric acid), additional bitter principles (e.g. quinine, caffeine, limonin, amarogentin, humolones, lupolones, catechols, tannins), sweeteners (e.g. saccharin, cyclamate, aspartame, neotame), mineral salts (e.g. sodium chloride, potassium chloride, magnesium chloride, sodium phosphates), substances which prevent enzymatic browning (e.g. sulfite, ascorbic acid), essential oils, plant extracts, natural or synthetic dyestuffs or coloured pigments (e.g. carotenoids, flavonoids, anthocyans, chlorophyll and derivatives thereof), spices, substances having a trigeminal action (that is to say cause piquant, pungent, prickling, tickling, astringent, warm or cold effects) or plant extracts containing such substances having a trigeminal action, synthetic, natural or nature-identical aroma substances or odoriferous substances as well as flavour correctants.

Dental care compositions (formulations which serve as a basis for oral care) which comprise the compounds of the formula (I) to be used according to the invention; their salts or mixtures in general comprise an abrasive system (abrasive or polishing agent), such as e.g. silicas, calcium carbonates, calcium phosphates, aluminium oxides and/or hydroxyapatites, surface-active substances, such as e.g. sodium lauryl sulfate, sodium lauryl sarcosinate and/or cocamidopropylbetaine, moisture-retaining agents, such as e.g. glycerol and/or sorbitol, thickeners, such as e.g. carboxymethylcellulose, polyethylene glycols, carrageenan and/or Laponite^{®}, sweeteners, such as e.g. saccharin, flavour correctants for unpleasant taste impressions, flavour correctants for further, as a rule not unpleasant taste impressions, taste-modulating substances (e.g. inositol phosphate, nucleotides, such as guanosine monophosphate, adenosine monophosphate or other substances, such as sodium glutamate or 2-phenoxypropionic acid), cooling active compounds, such as e.g. menthol, menthol derivatives (e.g. L-menthol, L-menthyl lactate, L-menthyl alkyl carbonates, menthone ketals, menthanecarboxylic acid amides), 2,2,2-trialkylacetic acid amides (e.g. 2,2-diisopropylpropionic acid methylamide), icilin derivatives, stabilizers and active compounds, such as e.g. sodium fluoride, sodium monofluorophosphate, tin difluoride, quaternary ammonium fluorides, zinc citrate, zinc sulfate, tin pyrophosphate, tin dichloride, mixtures of various pyrophosphates, triclosan, cetylpyridinium chloride, aluminium lactate, potassium citrate, potassium nitrate, potassium chloride, strontium chloride, hydrogen peroxide, aromas and/or sodium bicarbonate, or odour correctants.

Chewing gums (as a further example of formulations for oral care) which comprise hydroxyphenylalkadione derivatives to be used according to the invention, their salts or mixtures in general comprise a chewing gum base, i.e. a chewing compositions which becomes plastic on chewing, various types of sugars, sugar substitutes, sweeteners, sugar alcohols, flavour correctants for unpleasant taste impressions, flavour correctants for further, as a rule not unpleasant taste impressions, taste-modulating substances (e.g. inositol phosphate, nucleotides, such as guanosine monophosphate, adenosine monophosphate or other substances, such as sodium glutamate or 2-phenoxypropionic acid), the cooling active compounds, humectants, thickeners, emulsifiers, aromas and stabilizers or odour correctants mentioned in the preceding section.

All the conventional further active compounds, base substances, auxiliary substances and additives for oral pharmaceutical formulations can be used as constituents for oral pharmaceutical formulations according to the invention. Unpleasantly tasting pharmaceutical active compounds which can be formulated for oral use can also be used, in particular, as active compounds. The active compounds, base substances, auxiliary substances and additives can be converted into the oral administration forms in a manner known per se. This is regularly effected using inert, non-toxic, pharmaceutically suitable auxiliary substances. These include, inter alia, carrier substances (e.g. microcrystalline cellulose), solvents (e.g. liquid polyethylene glycols), emulsifiers (e.g. sodium dodecyl sulfate), dispersing agents (e.g. polyvinylpyrrolidone), synthetic and natural biopolymers (e.g. albumin), stabilizers (e.g. antioxidants, such as ascorbic acid), dyestuffs (e.g. inorganic pigments, such as iron oxides) and odour correctants as well as flavour correctants which do not relate to the bitter taste.

Preferably, the formulations according to the invention can also comprise an aroma composition in order to round off and refine the taste and/or odour of the formulation. Suitable aromatic compositions comprise e.g. synthetic, natural or nature-identical aroma, odoriferous and flavouring substances as well as suitable auxiliary and carrier substances. In this context, it is regarded as particularly advantageous that a bitter or metallic taste impression which arises from aroma or odoriferous substances contained in the formulations according to the invention can be masked or reduced and therefore the overall aroma or taste profile is improved.

Formulations according to the invention which are in the form of semi-finished goods can serve to mask or reduce the unpleasant taste impression of finished goods formulations which are prepared using the semi-finished goods formulations.

Formulations according to the invention which preferably serve as semi-finished goods as a rule comprise 0.0001 wt.% to 95 wt.%, preferably 0.001 to 80 wt.%, but in particular 0.01 wt.% to 50 wt.%, based on the total weight of the formulation, of compounds of the formula (I) to be used according to the invention, salts thereof or mixtures thereof. Formulations according to the invention which are in the form of semi-finished goods can serve to mask or reduce the unpleasant taste impression of finished goods formulations which are prepared using the semi-finished goods formulation.

In a particularly preferred embodiment of the invention, the hydroxyphenylalkadione derivatives according to the invention, their salts or mixtures are used in the formulations according to the invention in combination with at least one further substance for modifying, masking or reducing the unpleasant taste impression of an unpleasantly tasting substance. A particularly effective masking can be achieved in this manner. In particular, the combination of the hydroxyphenylalkadione derivatives to be employed according to the invention with other flavour correctants for unpleasant, in particular bitter taste impressions is preferred.

The further flavour correctants can be chosen from the following list, without limiting the invention therewith. Nucleotides (e.g. adenosine 5`-monophosphate, cytidine 5'-monophosphate) or pharmaceutically acceptable salts thereof, lactisols, sodium salts (e.g. sodium chloride, sodium lactate, sodium citrate, sodium acetate, disodium gluconate), hydroxyflavanones (e.g. eriodidyol, homoeriodictyol or sodium salts thereof), in particular according to EP 1 258 200, hydroxybenzoic acid amides (e.g. 2,4-dihydroxybenzoic acid vanillylamide, 2,4-dihydroxybenzoic acid *N*-(4-hydroxy-3-methoxybenzyl)amide, 2,4,6-trihydroxybenzoic acid *N*-(4-hydroxy-3-methoxybenzyl)amide, 2 -hydroxy-benzoic acid *N*-4-(hydroxy-3-methoxybenzyl)amide, 4-hydroxybenzoic acid *N*-(4-hydroxy-3-methoxybenzyl)amide, 2,4-dihydroxybenzoic acid *N*-(4-hydroxy-3-methoxybenzyl)amide monosodium salt, 2,4-dihydroxybenzoic acid *N*-2-(4-hydroxy-3-methoxyphenyl)ethylamide, 2,4-dihydroxybenzoic acid *N*-(4-hydroxy-3-ethoxybenzyl)amide, 2,4-dihydroxybenzoic acid *N*-(3,4-dihydroxybenzyl)amide and 2-hydroxy-5-methoxy-N-[2-(4-hydroxy-3-methoxyphenyl)ethyl]amide (aduncamide), 4-hydroxybenzoic acid vanillylamide), hydroxydeoxybenzoins (e.g. 2-(4-hydroxy-3-methoxyphenyl)-1-(2,4,6-trihydroxyphenyl)ethanone, 1-(2,4-dihydroxyphenyl)-2-(4-hydroxy-3-methoxyphenyl)ethanone, 1-(2-hydroxy-4-methoxyphenyl)-2-(4-hydroxy-3-methoxyphenyl)ethanone), amino acids (e.g. gamma-aminobutyric acid) or mixtures of whey proteins with lecithins.

### Examples

Examples 1 and 2 and Use Examples 1 to 10, which are merely for illustration of the invention, without thereby limiting the invention, are to be found in part B of the experimental part.

Some more embodiments of the use or formulations or compounds according to the invention which are preferred are described in the following sections, which are also decisive for the further development in part B:
A. Use of a compound of the formula (I) 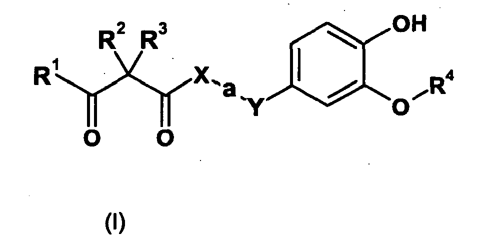 wherein, for X, a and Y:
   X is a -CH₂-, -NH- or -O- group,
   Y ais a -CH₂- group and
   a is a single bond or
      X and Y are in each case a -CH- group and
      a is a double bond in the Z or E configuration,
   wherein, for R¹, R² and R³
   - R¹: denotes hydrogen, an aliphatic radical having 1 to 4 C atoms or a -O-R⁵, -S-R⁵ or -NR⁵R⁶ group and
   - R² and R³: independently of one another denote hydrogen or an alkyl having 1, 2, 3, 4 or 5 C atoms which is optionally substituted by one or more oxo groups or R⁷-O- groups
   or
   - R¹ and R²: together form an aliphatic ring which contains a total of 4 to 10 carbon atoms and 0 or 1 oxygen atom, wherein the ring constituent represented by R¹ and R² is optionally substituted by one or more oxo groups and/or R⁷-O- groups and/or aliphatic radicals having 1 to 4 C atoms and
   - R³: denotes hydrogen or an alkyl having 1, 2, 3, 4 or 5 C atoms which is optionally substituted by one or more oxo groups or R⁷-O- groups
   or
   - R² and R³: together form an aliphatic ring which contains a total of 4 to 10 carbon atoms and 0 or 1 oxygen atom, wherein the ring constituent represented by R² and R³ is optionally substituted by one or more oxo groups and/or R⁷-O- groups and/or aliphatic radicals having 1 to 4 C atoms and
   - R¹: denotes hydrogen, an aliphatic radical having 1 to 4 C atoms or a -O-R⁵, -S-R⁵ or -NR⁵R⁶ group,
   wherein, for R⁴:
   - R⁴: denotes hydrogen, methyl or ethyl
   and wherein, for R⁵, R⁶ and R⁷:
   - R⁵ R⁶ and R⁷: independently of one another denote hydrogen or an aliphatic radical having 1 to 4 C atoms,
   salts thereof and mixtures thereof for masking or reducing the unpleasant taste impression of an unpleasantly tasting substance.
B. Use as defined in section A, wherein the aliphatic radical having 1 to 4 C atoms which is optionally present in R¹, R⁵, R⁶ and/or R⁷ denotes methyl, ethyl, 1-propyl or 2-propyl.
C. Use as defined in the preceding sections, wherein the alkyl having 1, 2, 3, 4 or 5 C atoms which is optionally substituted by one or more oxo groups or R⁷ -O-groups is chosen from the group consisting of: methyl, ethyl, 1-propyl, 2-propyl, 1-oxomethyl, 1-oxoethyl, 1-methyloxymethyl, 1-methyloxyethyl, 1-oxopropyl.
D. Use as defined in the preceding sections, wherein
   - R¹ and R²: together form an aliphatic ring which is five-, six- or seven-membered in total and contains 4 to 7 carbon atoms and 0 or 1 oxygen atom, wherein the ring constituent represented by R¹ and R² is optionally substituted by one or more oxo groups and/or R⁷-O- groups and/or aliphatic radicals having 1 to 4 C atoms.
E. Use as defined in the preceding sections,
   wherein, for X, a and Y:
   X is a -CH₂- or -NH- group,
   Y is a -CH₂- group and
   a is a single bond
   or
   X and Y are in each case a -CH- group and
   a is a double bond in the E configuration,
   wherein, for R¹, R² and R³
   - R¹: denotes an aliphatic radical having 1 to 4 C atoms or a -O-R⁵ group,
   - R²: denotes hydrogen or an alkyl having 1, 2, 3, 4 or 5 C atoms which is optionally substituted by one or more oxo groups or R⁷-O- groups and
   - R³: denotes hydrogen,
   or
   - R¹ and R²: together form an aliphatic ring which contains a total of 5 to 7 carbon atoms and 0 or 1 oxygen atom, wherein the ring constituent represented by R¹ and R² is optionally substituted by one or more oxo groups and/or R⁷-O- groups and
   - R³: denotes hydrogen.
   wherein, for R⁴:
   - R⁴: denotes hydrogen, methyl or ethyl
   and wherein, for R⁵ and R⁷:
   - R⁵ and R⁷: independently of one another denote hydrogen or an aliphatic radical having 1 to 4 C atoms.
F. Use as defined in the preceding sections,
   wherein, for X, a and Y:
   X is a -CH₂- group,
   Y is a -CH₂- group and
   a is a single bond
   or
   X and Y are in each case a -CH- group and
   a is a double bond in the E configuration,
   wherein, for R¹, R² and R³
   - R¹: denotes methyl, ethyl, 1-propyl, 2-propyl or a -O-R⁵ group and
   - R²: denotes hydrogen or an alkyl having 1, 2, 3, 4 or 5 C atoms which is optionally substituted by one or more oxo groups and
   - R³: denotes hydrogen,
   or
   - R¹ and R²: together form an aliphatic ring which contains a total of 5 to 7 carbon atoms and 0 or 1 oxygen atom, wherein the ring constituent represented by R¹ and R² is optionally substituted by one or more oxo groups and
   - R³: denotes hydrogen.
   wherein, for R⁴:
   - R⁴: denotes hydrogen or methyl
   and wherein, for R⁵:
   - R⁵: denotes hydrogen, methyl or ethyl.
G. Use of
   (5*E*)-6-(4-hydroxy-3-methoxyphenyl)hex-5-ene-2,4-dione,
   (6*E*)-7-(4-hydroxy-3-methoxyphenyl)hept-6-ene-3,5-dione,
   (5*E*)-6-(3,4-dihydroxyphenyl)hex-5-ene-2,4-dione,
   6-(4-hydroxy-3-methoxyphenyl)hexane-2,4-dione,
   7-(4-hydroxy-3-methoxyphenyl)heptane-3.5-dione,
   5-(4-hydroxy-3-methoxyphenyl)pentan-3-onoic acid,
   5-(4-hydroxy-3-methoxyphenyl)pentan-3-onoic acid ethyl ester,
   2-acetyl-5-(4-hydroxy-3-methoxyphenyl)pentan-3-onoic acid ethyl ester,
   2-(3-[4-hydroxy-3-methoxyphenyl]propionyl)-cyclopentane-1,3-dione,
   salts thereof and mixtures thereof for masking or reducing the unpleasant taste impression of an unpleasantly tasting substance.
H. Use of a salt of a compound of the formula (I) as defined in the receding sections, wherein one, several or all of the hydroxyl groups of the compound of the formula (I) are deprotonated and a corresponding amount of counter-cations is present, which are chosen from the group consisting of: singly positively charged cations of the first main and sub-group, ammonium ions, trialkylammonium ions, doubly positively charged cations of the second main and sub-group and triply positively charged cations of the third main and sub-group, and mixtures thereof.
I. Use as defined in the preceding sections, in combination with at least one further substance for modifying, masking or reducing the unpleasant taste impression of an unpleasantly tasting substance.
J. Use as defined in the preceding sections, in combination with at least one sweet-tasting substance.
K. Use as defined in the preceding sections, in a formulation for nutrition, oral care or consumption for pleasure or an oral pharmaceutical formulation.
L. Formulation for nutrition, oral care or consumption for pleasure or cosmetic formulation for application in the region of the head, comprising:
   0.000001 wt.% to 95 wt.%, based on the total weight of the formulation, of a compound of the formula (I) 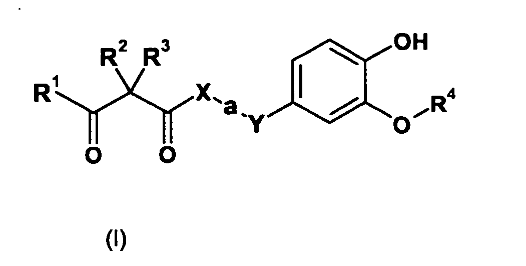 wherein, for X, a and Y:
      X is a -CH₂-, -NH- or -O- group,
      Y is a -CH₂- group and
      a is a single bond
      or
      X and Y are in each case a -CH- group and
      a is a double bond in the Z or E configuration,
      wherein, for R¹, R² and R³

   - R¹: denotes hydrogen, an aliphatic radical having 1 to 4 C atoms or a -O-R⁵, -S-R⁵ or -NR⁵R⁶ group and
   - R² and R³: independently of one another denote hydrogen or an alkyl having 1, 2, 3, 4 or 5 C atoms which is optionally substituted by one or more oxo groups or R⁷-O- groups
   or
   - R¹ and R²: together form an aliphatic ring which contains a total of 4 to 10 carbon atoms and 0 or 1 oxygen atom, wherein the ring constituent represented by R¹ and R² is optionally substituted by one or more oxo groups and/or R⁷-O- groups and/or. aliphatic radicals having 1 to 4 C atoms and
   - R³: denotes hydrogen or an alkyl having 1, 2, 3, 4 or 5 C atoms which is optionally substituted by one or more oxo groups or R⁷-O- groups
   or
   - R² and R³: together form an aliphatic ring which contains a total of 4 to 10 carbon atoms and 0 or 1 oxygen atom, wherein the ring constituent represented by R² and R³ is optionally substituted by one or more oxo groups and/or R⁷-O- groups and/or aliphatic radicals having 1 to 4 C atoms and
   - R¹: denotes hydrogen, an aliphatic radical having 1 to 4 C atoms or a -O-R⁵, -S-R⁵ or -NR⁵R⁶ group,
   wherein, for R⁴:
   - R⁴: denotes hydrogen, methyl or ethyl
   and wherein, for R⁵, R⁶ and R⁷:
   R⁵ R⁶ and R⁷ independently of one another denote hydrogen or an aliphatic radical having 1 to 4 C atoms or
   one or more of the salts or a mixture of such compounds, as well as
   optionally an unpleasantly tasting substance.
M. Oral pharmaceutical compositions, comprising
   at least one unpleasantly tasting substance and
   0.000001 wt.% to 10 wt.%, based on the total weight of the formulation, of a compound of the formula (I) 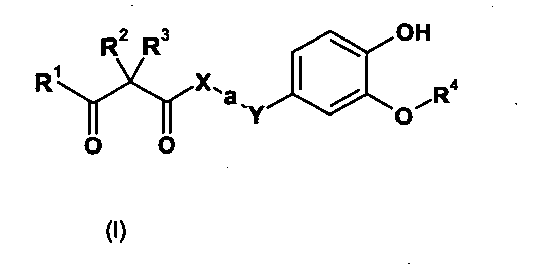 wherein, for X, a and Y:
   X is a -CH₂-, -NH- or -O- group,
   Y is a -CH₂- group and
   a is a single bond
   or
   X and Y are in each case a -CH- group and
   a is a double bond in the Z or E configuration,
   wherein, for R¹, R² and R³
   - R¹: denotes hydrogen, an aliphatic radical having 1 to 4 C atoms or a -O-R⁵, -S-R⁵ or -NR⁵R⁶ group and
   - R² and R³: independently of one another denote hydrogen or an alkyl having 1, 2, 3, 4 or 5 C atoms which is optionally substituted by one or more oxo groups or R⁷-O- groups
   - R¹ and R²: together form an aliphatic ring which contains a total of 4 to 10 carbon atoms and 0 or 1 oxygen atom, wherein the ring constituent represented by R¹ and R² is optionally substituted by one or more oxo groups and/or R⁷-O- and/or aliphatic radicals having 1 to 4 C atoms and
   - R³: denotes hydrogen or an alkyl having 1, 2, 3, 4 or 5 C atoms which is optionally substituted by one or more oxo groups or R⁷-O- groups
   or
   - R² and R³: together form an aliphatic ring which contains a total of 4 to 10 carbon atoms and 0 or 1 oxygen atom, wherein the ring constituent represented by R² and R³ is optionally substituted by one or more oxo groups and/or R⁷-O- groups and/or aliphatic radicals having 1 to 4 C atoms and
   - R¹: denotes hydrogen, an aliphatic radical having 1 to 4 C atoms or a -O-R⁵, -S-R⁵ or -NR⁵ R⁶group,
   wherein, for R⁴:
   - R⁴: denotes hydrogen, methyl or ethyl
   and wherein, for R⁵, R⁶ and R⁷:
   R⁵ R⁶ and R⁷ independently of one another denote hydrogen or an aliphatic radical having 1 to 4 C atoms,
      one or more of the salts or a mixture of such compounds.
N. Formulation as defined in section L or M, comprising at least one unpleasantly tasting substance, the amount of the unpleasantly tasting substance being sufficient to be perceived as an unpleasant taste in a comparison formulation which comprises no compound of the formula (I), salt or mixture, as defined in sections A to H, but is of otherwise identical composition, and the amount of the compound of the formula (I), salt or mixture as defined in sections A to H in the formulation being sufficient to mask sensorially the unpleasant taste impression of the unpleasantly tasting substance or to reduce it in comparison with the comparison formulation.
O. Formulation as defined in sections L to N, characterized in that they are in the form of semi-finished goods, in the form of an odoriferous, aroma or flavouring substance composition or in the form of a spice mixture.
P. Formulation as defined in sections L to O, furthermore comprising at least one further substance for modifying, masking or reducing the unpleasant taste impression of an unpleasantly tasting substance.
Q. Cosmetic formulations comprising a compound of the formula (I), a salt or a mixture, as defined in section A to H.
R. 5-(4-hydroxy-3-methoxyphenyl)pentan-3-onoic acid,
   5-(4-hydroxy-3-methoxyphenyl)pentan-3-onoic acid ethyl ester,
   2-acetyl-5-(4-hydroxy-3-methoxyphenyl)pentan-3-onoic acid ethyl ester, salts thereof and mixtures thereof.

### Part B:

The invention relates to the use of certain hydroxyphenylalkadione derivatives of the general formula (I), salts thereof and mixtures thereof for modifying, masking or reducing unpleasant taste impressions, in particular bitter, astringent and/or metallic taste impressions, and/or for intensifying the sweet taste of sweet-tasting substances or the sweet odour impression of aroma substances which cause a sweet odour impression. The invention furthermore relates to certain formulations which comprise an active content of the hydroxyphenylalkadione derivatives described, salts thereof or mixtures thereof.

Foodstuffs or compositions for consumption for pleasure often comprise various bitter principles, which indeed on the one hand are desirable and characteristic in moderation (e.g. caffeine in tea or coffee, quinine in so-called bitter lemon drinks, hop extracts in beer), but on the other hand can also greatly reduce the value (e.g. flavonoid glycosides and limonoids in citrus juices, the bitter after-taste of many artificial sweeteners; such as aspartame or saccharin, hydrophobic amino acids and/or peptides in cheese).

A subsequent treatment is therefore often necessary to lower the natural content of bitter principles, for example by extraction, as in the decaffeination of tea or coffee, or enzymatically, e.g. treatment of orange juice with a glycosidase to destroy the bitter naringin or the use of specific peptidases in the maturation of cheese. This treatment is a burden for the product, generates waste and e.g. also causes solvent residues and other residues (enzymes) in the products.

It is therefore desirable to discover substances which can effectively suppress or at least reduce unpleasant taste impressions, in particular bitter, astringent and/or metallic taste impressions.

Suppression of the bitter taste is particularly important in the case of many pharmaceutical active compoundss, since the willingness of the patient, in particular in the case of patients sensitive to bitterness, such as children, to take the formulation orally can thereby be increased significantly. Many pharmaceutical active compounds, for example aspirin, salicin, paracetamol, ambroxol or quinine, to name only a very small selection for illustration, have a pronounced bitter, astringent and/or metallic taste and/or after-taste.

Some substances which can partly suppress bitter taste are indeed known, but many show severe limitations in use.

In US 5,637,618, a bitter taste is reduced with the aid of lactisol [20-(4-methoxyphenyl)lactic acid]. However, this inhibitor at the same time shows a marked inhibition of the sweet taste impression (cf. US 5,045,336), which severely restricts usability.

2,4-Dihydroxybenzoic acid potassium salt is described in US 5,643,941 (Table column 3, line 18) as a masking agent for the bitter taste of potassium chloride, but cannot suppress e.g. the taste of caffeine.

According to GB 2,380,936, suppression of the taste of bitter pharmaceuticals is achieved with ginger extract. Needless to say, the strong aroma impression and/or the piquancy of ginger extracts which is often to be found therein or constituents therefrom which have a piquant action is not suitable for a large number of uses.

Neohesperidin dihydrochalcone also shows a bitter-reducing effect, but is above all a sweetener (cf. Manufacturing Chemist 2000, July issue, p. 16-17), which also causes trouble in non-sweet uses.

Taste-modifying properties are indeed described for some flavones (2-phenylchrom-2-en-4-ones) in US-A 5,580,545, but a bitter-reducing or -suppressing action was not found.

US 2002 177,576 describes the suppression of a bitter taste by nucleotides, for example cytidine 5'-monophosphates (CMP). However, the strongly polar compounds, which therefore can be used only in strongly polar solvents, can be used to only a very limited degree in many fat-containing foodstuffs. Furthermore, the availability of such substances is severely limited because of their expensive chemical synthesis.

US 2002 188,019 describes hydroxyflavanones as active bitterness maskers which, however, are accessible by synthesis only with difficulty and are not available inexpensively in larger amounts.

The sodium salts sodium chloride, sodium citrate, sodium acetate and sodium lactate show a bitterness-masking effect against many bitter principles (e.g. Nature, 1997, vol. 387, p. 563); needless to say, intake of relatively large amounts of sodium ions can lead e.g. to cardiovascular diseases. Furthermore, a significant bitterness-masking action disadvantageously occurs only at relatively high sodium concentrations (from approx. 0.1 M), which corresponds e.g. to an as a rule unacceptably high content of approx. 0.6 wt.% NaCl in the end use (cf. R.S.J. Keast, P.A.S. Breslin and G.K. Beauchamp, Chimia 2001, 55(5), 441-447).

WO 00/21390 describes polyglutamic acid as a bitterness-masking agent; relatively high concentrations in the region of about 1 wt.% are required here.

A lipoprotein comprising β-lactoglobulin and phosphatidic acid also shows a bitterness-masking effect (EP-A 635 218). Needless to say, such polymers are difficult to characterize and standardize and show a decidedly soapy secondary taste.

The flavone glycoside neodiosmin [5,7-dihydroxy-2-(4-methoxy-3-hydroxyphenyl)-7-O-neohesperidoxyl-chrom-2-en-4-one] likewise shows a bitterness-masking action (US-A 4,154,862), but is distinguished by a disaccharide radical which makes the preparation or isolation and usability of the substance very difficult.

Foodstuffs or compositions for consumption for pleasure which have a high sugar content (above all sucrose (= saccharose), lactose, glucose or fructose or mixtures thereof) are as a rule greatly preferred by consumers because of the sweetness. On the other hand, it is generally known that a high content of readily metabolizable carbohydrates makes the blood sugar level rise severely, leads to the formation of fat deposits and in the end can lead to health problems, such as overweight, obesity, insulin resistance, adult-onset diabetes and secondary diseases thereof. In particular, the situation is made difficult by the fact that many of the abovementioned carbohydrates can additionally impair dental health, since they are broken down by certain varieties of bacteria in the oral cavity to give, for example, lactic acid and can attack the tooth enamel of juvenile or adult teeth (caries).

It has therefore long been an aim to reduced the sugar content of foodstuffs and/or compositions for consumption for pleasure to the absolutely necessary degree or below. An appropriate measure consists of the use of sweeteners: these are chemically uniform substances which themselves have no or only a very low caloric fuel value and at the same time cause a strong sweet taste impression; the substances are as a rule non-cariogenic (an overview is to be found e.g. in the Journal of the American Dietetic Associations 2004, 104 (2), 255-275). The so-called bulk sweeteners, such as sorbitol, mannitol or other sugar alcohols, indeed in some cases are excellent sweeteners and can also partly replace the other foodstuffs properties of sugars, but if ingested too often in one sector of the population lead to osmosis-related digestion problems. The non-nutritious, highly intensive sweeteners are indeed particularly suitable, due to their low use concentration, for introducing sweetness into foodstuffs, but often show taste problems due to dissimilar time/intensity profiles (e.g. sucralose, stevioside, cyclamate) compared with sugars, a bitter and/or astringent after-taste (e.g. acesulfame K, saccharin) or pronounced additional aroma impressions (e.g. glycyrrhizic acid ammonium salt). Some of the sweeteners are not particularly stable to heat (e.g. thaumatin, brazzein, monellin), are not stable in all uses (e.g. aspartame) and are in some cases very persistent in their sweet action (strong sweet after-taste, e.g. saccharin).

An improvement in the flavouring properties in particular the after-taste problem, of non-nutritional, highly intensive sweeteners can be achieved by the use of tannic acid, e.g. as described in WO 98/20753, or phenol acids, as in US 3,924,017. Needless to say, such substances are not particularly stable in uses because of their catechol units.

Another possibility - without the use of non-nutritional sweeteners - comprises lowering the sugar content of foodstuffs and/or compositions for consumption for pleasure and adding substances which are weakly perceptible or imperceptible sensorially and intensify the sweetness indirectly or directly, as described e.g. in WO 2005/041684. However, the substances described in WO 2005/041684 are expressly of non-natural origin and are thus more difficult to evaluate from the toxicological aspect than substances of natural origin, especially if the latter occur in foodstuffs or compositions for consumption for pleasure or originate from raw materials for obtaining foodstuffs or compositions for consumption for pleasure. EP 1 291 342 describes such substances of natural origin (pyridinium-betaines); needless to say, the sweet taste is not influenced selectively by them, but also other taste directions, such as umami or saltiness. Furthermore, the substances disclosed can be purified only with a high expenditure.

US Provisional Application 60/702,943 and the documents based on this (Symrise) recommends the use of hesperetin as an intensifier of the sweet taste of reduced-sugar formulations for nutrition or consumption for pleasure. Needless to say, a disadvantage of the use of hesperetin is sometimes its low solubility, in particular- in clear, aqueous uses (such as e.g. clear cola or lemon carbonated drinks) and the comparatively weak intensification of sweetness in acid foodstuffs and compositions for consumption for pleasure.

It is therefore desirable to discover substances which, in low concentrations, effectively intensify sweet taste impression of sweet substances, preferably the sweet taste impression of reduced-sugar foodstuffs and compositions for consumption for pleasure, in particular of reduced-sugar acidic and/or clear aqueous foodstuffs and compositions for consumption for pleasure, without adversely influencing the remaining aroma profile. It is likewise desirable to discover substances which, in low concentrations, effectively intensify sweet odour impressions of aroma substances which cause a sweet odour impression.

It was the primary object of the present invention to discover substances which (a) are suitable for modifying or reducing the unpleasant taste impression of unpleasantly tasting substances (and preferably in particular show a bitterness-masking effect against a large number of bitter principles), (b) can be used widely, (c) are readily accessible and/or to discover substances which (d) are suitable selectively for intensifying the sweet taste of a sweet-tasting substance and/or the sweet odour impression of an aroma substance which causes a sweet odour impression, preferably without adversely influencing the remaining aroma profile, (e) can also be used widely in acid or clear (aqueous) formulations and preferably (f) occur naturally, in particular in foodstuffs or compositions for consumption for pleasure or the corresponding raw materials for formulation thereof, or are formed during the preparation of foodstuffs or compositions for consumption for pleasure.

The object described is achieved according to the invention by the use
- of a hydroxyphenylalkadione derivative of the formula (I) 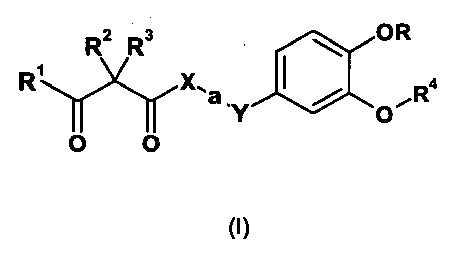 wherein, for X, a and Y:
   X is a -CH₂-, -NH- or -O- group,
   Y is a -CH₂- group and
   a is a single bond
   or
   X and Y are in each case a -CH- group and
   a is a double bond in the Z or E configuration,
   wherein, for R¹, R² and R³
   - R¹: denotes hydrogen, an aliphatic radical having 1 to 4 C atoms or a -O-R⁵, -S-R⁵ or -NR⁵R⁶ group and
   - R² and R³: independently of one another denote hydrogen or an alkyl having 1, 2, 3, 4 or 5 C atoms which is optionally substituted by one or more oxo groups or R⁷-O- groups
   or
   - R¹ and R²: together form an aliphatic ring which contains a total of 4 to 10 carbon atoms and 0 or 1 oxygen atom, wherein the ring constituent represented by R¹ and R² is optionally substituted by one or more oxo groups and/or R⁷-O- groups and/or aliphatic radicals having 1 to 4 C atoms and
   - R³: denotes hydrogen or an alkyl having 1, 2, 3, 4 or 5 C atoms which is optionally substituted by one or more oxo groups or R⁷-O- groups
   or
   - R² and R³: together form an aliphatic ring which contains a total of 4 to 10 carbon atoms and 0 or 1 oxygen atom, wherein the ring constituent represented by R² and R³ is optionally substituted by one or more oxo groups and/or R⁷-O- groups and/or aliphatic radicals having 1 to 4 C atoms and
   - R¹: denotes hydrogen, an aliphatic radical having 1 to 4 C atoms or a -O-R⁵, -S-R⁵ or -NR⁵R⁶ group,
   wherein, for R⁴ and R:
   - R⁴ and R: independently of one another denote hydrogen, methyl or ethyl
   and wherein, for R⁵, R⁶ and R⁷_{:}
   - R⁵ R⁶ and R⁷: independently of one another denote hydrogen or an aliphatic radical having 1 to 4 C atoms,
- a salt of such a hydroxyphenylalkadione of the formula (I),
- a mixture comprising or consisting of two or more different hydroxyphenylalkadiones of the formula (I), wherein X, a, Y, R, R¹, R², R³, R⁴, R⁵, R⁶ and R⁷ in each case have the meaning given above,
- a mixture comprising or consisting of salts of two or more different hydroxyphenylalkadiones of the formula (I), wherein X, a, Y, R, R¹, R², R³, R⁴, R⁵, R⁶ and R⁷ in each case have the meaning given above,
   or
- a mixture comprising or consisting of a hydroxyphenylalkadione of the formula (I) or two or more different hydroxyphenylalkadiones of the formula (I),
wherein X, a, Y, R, R¹, R², R³, R⁴, R⁵, R⁶ and R⁷ in each case have the meaning given above, and a salt of a hydroxyphenylalkadione of the formula (I) or two or more salts of different hydroxyphenylalkadiones of the formula (I), wherein X, a, Y, R, R¹, R², R³, R⁴, R⁵, R⁶ and R⁷ in each case have the meaning given above,
for modifying, masking or reducing the unpleasant flavour impression of an unpleasantly tasting substance and/or for intensifying the sweet taste of a sweet-tasting substance and/or the sweet odour impression of an aroma substance which causes a sweet odour impression.

The aliphatic radical having 1 to 4 C atoms which is optionally present in R¹, R⁵, R⁶ and/or R⁷ preferably denotes methyl, ethyl, 1-propyl or 2-propyl. If an aliphatic radical having 1 to 4 C atoms is present in several radicals R¹, R⁵, R⁶ and/or R⁷, each of these aliphatic radicals is chosen independently of the others. These above definitions likewise apply to the preferred embodiments.

If an alkyl having 1, 2, 3, 4 or 5 C atoms which is optionally substituted by one or more oxo groups or R⁷-O- groups is present in the radicals R² and/or R³, this is preferably chosen from the group consisting of: methyl, ethyl, 1-propyl, 2-propyl, 1-oxomethyl, 1-oxoethyl, 1-methyloxymethyl, 1-methyloxyethyl, 1-oxopropyl. If corresponding radicals are present in both groups R² and R³, these are chosen independently of one another. These above definitions likewise apply to the preferred embodiments.

If R¹ and R² together form an aliphatic ring which is five-, six- or seven-membered in total, this preferably contains 4 to 7 carbon atoms and 0 or 1 oxygen atom. In this context, the ring constituent represented by R¹ and R² is optionally substituted by one or more oxo groups and/or R⁷-O- groups and/or aliphatic radicals having 1 to 4 C atoms. Particularly preferred (part) structures can be seen from the following figure: 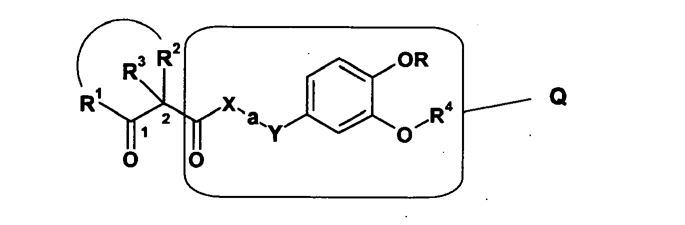 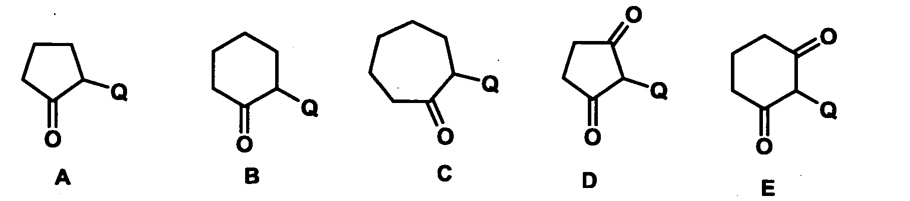 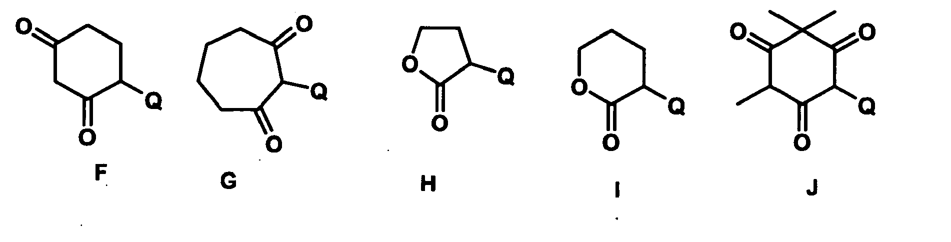 (Part) structures A to J are groupings which are in each case linked with the structural constituent Q via the 2 position. The meaning of the radicals R¹, R² and R³ of the formula (I) within the particular (part) structure A to J can be seen, in particular, by comparison of the particular (part) structure with the formula representation shown above. These above definitions likewise apply to the preferred embodiments.

The following (part) structures are thus particularly preferred:
- Cyclopentan-1-one (A): R³ = H; R¹ and R² together form a ring of 5 carbon atoms having 5 members in total, wherein the ring constituent represented by R¹ and R² is not substituted.
- Cyclohexan-1-one (B): R¹ and R² together form a an aliphatic ring which contains 6 carbon atoms and has 6 members in total, wherein the ring constituent represented by R¹ and R² is not substituted.
- Cycloheptan-1-one (C): R¹ and R² together form a ring which contains 7 carbon atoms and has 7 members in total, wherein the ring constituent represented by R¹ and R² is not substituted.
- Cyclopentane-1,3-dione (D): R¹ and R² together form an aliphatic ring which contains 5 carbon atoms and has 6 members in total, wherein the ring constituent represented by R¹ and R² is substituted by an oxo group.
- Cydohexa-1,3-dione (E): R¹ and R² together form an aliphatic ring which contains 6 carbon atoms and has 6 members in total, wherein the ring constituent represented by R¹ and R² is substituted by an oxo group.
- Cyclohexa-1,5-dione (F): R¹ and R² together form an aliphatic ring which contains 6 carbon atoms and has 6 members in total, wherein the ring constituent represented by R¹ and R² is substituted by an oxo group.
- Cydoheptane-1,3-dione (G): R¹ and R² together form a ring which contains 7 carbon atoms and has 7 members in total, wherein the ring constituent represented by R¹ and R² is substituted by an oxo group.
- 5-Oxacyclopentan-1-one (H): R¹ and R² together form a ring which contains 4 carbon atoms and 1 oxygen atom and has 5 members in total, wherein the ring constituent represented by R¹ and R² is not substituted.
- 6-Oxa-cydohexan-1-one (I): R¹ and R² together form a ring which contains 5 carbon atoms and 1 oxygen atom and has 6 members in total, wherein the ring constituent represented by R¹ and R² is not substituted.
- 4,6,6-Trimethyl-cyclohexane-1,3,5-trione (J): R¹ and R² together form a ring which contains 6 carbon atoms and has 6 members in total, wherein the ring constituent represented by R¹ and R² is substituted by 2 oxo groups and a total of 3 methyl radicals.

The oxo group present in position 1 of the particular (part) structure does not belong to the ring constituent represented by R¹ and R² and is present in every compound of the formula (I) to be employed according to the invention. These above definitions likewise apply to the preferred embodiments.

Unpleasantly tasting substances in the context of the invention are:
(a) substances which taste bitter, astringent, cardboardy, dusty, dry, floury, rancid and/or metallic and
(b) substances which have a bitter, astringent, cardboardy, dusty, dry, floury, rancid or metallic after-taste.

The abovementioned unpleasantly tasting substances can have still further as a rule not unpleasant taste and/or odour qualities. As further taste qualities which are not unpleasant in the sense of the present invention there may be mentioned e.g. the spicy, umami, sweet, salty, acid, piquant, cooling, warming, burning or tingling impressions.

Substances which taste bitter, astringent, cardboardy, dusty, dry, floury, rancid and/or metallic are, for example: xanthine alkaloids, xanthines (caffeine, theobromine, theophylline), alkaloids (quinines, brucine, strychnine, nicotine), phenolic glycosides (e.g. salicin, arbutin), flavonoid glycosides (e.g. hesperidin, naringin), chalcones or chalcone glycosides, hydrolysable tannins (gallic or ellagic acid esters of carbohydrates, e.g. pentagalloylglucose), non-hydrolysable tannins (optionally galloylated catechols or epicatechols and oligomers thereof, e.g. proanthyocyanidines or procyanidines, thearubigin), flavones (e.g. quercetin, taxifolin, myricetin), other polyphenols (γ-oryzanol, caffeic acid or esters thereof), terpenoid bitter principles (e.g. limonoids, such as limonin or nomilin from citrus fruits, lupolones and humolones from hops, iridoids, secoiridoids), absinthe from wormwood, amarogentin from gentian, metallic salts (potassium chloride, sodium sulfate, magnesium sulfate), pharmaceutical active compounds (e.g. fluoroquinolone antibiotics, paracetamol, aspirin, β-lactam antibiotics, ambroxol, propylthiouracil [PROP], guaifenesin), vitamins (for example vitamin H, vitamins from the B series, such as vitamin B1, B2, B6, B12, niacin, pantothenic acid), denatonium benzoate, sucralose octaacetate, potassium chloride; magnesium salts, iron salts, aluminium salts, zinc salts, urea, unsaturated fatty acids, in particular unsaturated fatty acids in emulsions, amino acids (e.g. leucine, isoleucine, valine, tryptophan, proline, histidine, tyrosine, lysine or phenylalanine), peptides (in particular peptides with an amino acid from the group consisting of leucine, isoleucine, valine, tryptophan, proline or phenylalanine on the N or C terminus).

Substances which have a bitter, astringent, cardboardy, chalky, dusty, dry, floury, rancid or metallic after-taste can be aroma or flavouring substances having a not unpleasant primary taste (for example sweet, salty, spicy, acid) and/or odour and belong e.g. to the group consisting of sweeteners, sugar substitutes or the aroma substances. Examples which may be mentioned are: aspartame, neotame, superaspartame, saccharin, sucralose, tagatose, monellin, stevioside, rebaudioside, hemandulcin, thaumatin, miraculin, glycyrrhizin, glycyrrhetic acid or derivatives thereof, cyclamate or the pharmaceutically acceptable salts of the abovementioned compounds.

Sweet-tasting substances (including natural sources of these substances) in the context of this invention can be, for example, sweet-tasting carbohydrates or sugars (e.g. sucrose (synonymous with saccharose), trehalose, lactose, maltose, melicitose, raffinose, palatinose, lactulose, D-fructose, D-glucose, D-galactose, L-rhamnose, D-sorbose, D-mannose, D-tagatose, D-arabinose, L-arabinose, D-ribose, D-glyceraldehyde, maltodextrin) or plant formulations comprising chiefly these carbohydrates (e.g. from sugar beet *(Beta vulgaris ssp.,* sugar fractions, sugar syrup, molasses), from sugar cane (*Saccharum officinarum ssp.,* e.g. molasses, sugar syrups), from sugar maple *(Acer ssp.),* from agave (agave thick juice), synthetic/enzymatic hydrolysates of starch or sucrose (e.g. invert sugar syrup, highly concentrated fructose syrups from maize starch), fruit concentrates (e.g. from apples or pears, apple leaf, pear leaf)), sugar alcohols (e.g. erythritol, threitol, arabitol, ribitol, xylitol, sorbitol, mannitol, dulcitol, lactitol), proteins (e.g. miraculin, monellin, thaumatin, curculin, brazzein), sweeteners (magap, sodium cyclamate, acesulfame K, neohesperidin dihydrochalcone, saccharin sodium salt, aspartame, superaspartame, neotame, alitame, sucralose, stevioside, rebaudioside, lugduname, carrelame, sucrononate, sucrooctate, monatin, phyllodulcin), certain sweet-tasting amino acids (glycine, D-leucine, D-threonine, D-asparagine, D-phenylalanine, D-tryptophan, L-proline), other sweet-tasting low molecular weight substances (e.g. hemandulcin, dihydrochalcone glycosides, glycyrrhizin, glycyrrhetic acid ammonium salt or other glycyrrhetic acid derivatives), extracts from liquorice *(Glycyrrhizza glabra ssp.),* extracts from *Lippia dulcis,* extracts or individual substances from *Momordica ssp.* (in particular *Momordica grosvenori* [Luo Han Guo] and the mogrosides obtained therefrom), from *Hydrangea dulcis* or from *Stevia ssp.* (e.g. *Stevia rebaudiana).*

A preferred use according to the invention of a compound of the formula (I) is that
wherein, for R⁴ and R:
R⁴ and R independently of one another denote hydrogen, methyl or ethyl,
wherein one radical R⁴ or R denotes hydrogen or both radicals R⁴ and R denote hydrogen.

Corresponding statements of course also apply to the corresponding salts and mixtures.

A particularly preferred use according to the invention of a compound of the formula (I) is that
wherein R denotes hydrogen.

Corresponding statements of course also apply to the corresponding salts and mixtures.

A preferred use according to the invention of a compound of the formula (I) is furthermore that
wherein one, several or all of the aliphatic radicals having 1 to 4 C atoms which are optionally present as R¹, R⁵, R⁶ and/or R⁷ denote methyl, ethyl, 1-propyl or 2-propyl.

Corresponding statements of course also apply to the corresponding salts and mixtures.

A particularly preferred use according to the invention of a compound of the formula (I) is that
wherein the alkyl having 1, 2, 3, 4 or 5C atoms which is optionally substituted by one or more oxo groups or R⁷-O- groups is chosen from the group consisting of:
methyl, ethyl, 1-propyl, 2-propyl, 1-oxomethyl, 1-oxoethyl, 1-methyloxymethyl, 1-methyloxyethyl, 1-oxopropyl.

Corresponding statements of course also apply to the corresponding salts and mixtures.

A very particularly preferred use according to the invention of a compound of the formula (I) is furthermore that
wherein R¹ and R² together form an aliphatic ring which is five-, six- or seven-membered in total and contains 4 to 7 carbon atoms and 0 or 1 oxygen atom,
wherein the ring constituent represented by R¹ and R² is optionally substituted by one or more oxo groups and/or R⁷-O- groups and/or aliphatic radicals having 1 to 4 C atoms.

Corresponding statements of course also apply to the corresponding salts and mixtures.

A very particularly preferred use according to the invention of a compound of the formula (I) is furthermore that
wherein, for X, a and Y:
X is a -CH₂- or -NH- group,
Y is a -CH₂- group and
a is a single bond
or
X and Y are in each case a -CH- group and
a is a double bond in the E configuration,
wherein, for R¹, R² and R³
- R¹: denotes an aliphatic radical having 1 to 4 C atoms or a -O-R⁵ group,
- R²: denotes hydrogen or an alkyl having 1, 2, 3, 4 or 5 C atoms which is optionally substituted by one or more oxo groups or R⁷-O- groups and
- R³: denotes hydrogen,
or
- R¹ and R²: together form an aliphatic ring which contains a total of 5 to 7 carbon atoms and 0 or 1 oxygen atom, wherein the ring constituent represented by R¹ and R² is optionally substituted by one or more oxo groups and/or R⁷-O- groups and
- R³: denotes hydrogen.
and wherein, for R⁵ and R⁷:
- R⁵ and R⁷: independently of one another denote hydrogen or an all phatic radical having 1 to 4 C atoms.

Corresponding statements of course also apply to the corresponding salts and mixtures.

A particularly preferred use of compounds of the formula (I) is that
wherein, for R¹ and R²:
- R¹: denotes methyl, ethyl, 1-propyl, 2-propyl or a -O-R⁵ group and
- R²: denotes hydrogen or an alkyl having 1, 2, 3, 4 or 5 C atoms which is optionally substituted by one or more oxo groups
or
- R¹ and R²: together form an aliphatic ring which contains a total of 5 to 7 carbon atoms and 0 or 1 oxygen atom, wherein the ring constituent represented by R¹ and R² is optionally substituted by one or more oxo groups
and wherein, for R⁵:
- R⁵: denotes hydrogen, methyl or ethyl.

Corresponding statements of course also apply to the corresponding salts and mixtures.

A very particularly preferred use is that
- of a hydroxyphenylalkadione of the formula (I) chosen from the group consisting of
   (5*E*)-6-(4-hydroxy-3-methoxyphenyl)hex-5-ene-2,4-dione,
   (6*E*)-7-(4-hydroxy-3-methoxyphenyl)hept-6-ene-3,5-dione,
   (5*E*)-6-(3,4-dihydroxyphenyl)hex-5-ene-2,4-dione,
   6-(4-hydroxy-3-methoxyphenyl)hexane-2,4-dione,
   7-(4-hydroxy-3-methoxyphenyl)heptane-3.5-dione,
   5-(4-hydroxy-3-methoxyphenyl)pentan-3-onoic acid,
   5-(4-hydroxy-3-methoxyphenyl)pentan-3-onoic acid ethyl ester,
   2-acetyl-5-(4-hydroxy-3-methoxyphenyl)pentan-3-onoic acid ethyl ester,
   2-(3-[4-hydroxy-3-methoxyphenyl]propionylycyclopentane-1,3₋dione,
   (5*E*)-6-(3-hydroxy-4-methoxyphenyl)hex-5-ene-2,4-dione and
   6-(3-hydroxy-4-methoxyphenyl)hexane-2,4-dione
- a salt of a hydroxyphenylalkadione of the formula (I) chosen from the said groups,
- a mixture comprising or consisting of two or more hydroxyphenylalkadiones of the formula (I) chosen from the said groups,
- a mixture comprising or consisting of salts of two or more hydroxyphenylalkadiones of the formula (I) chosen from the said groups,
   or
- a mixture comprising or consisting of a hydroxyphenylalkadione of the formula (I) chosen from the said groups or two or more hydroxyphenylalkadiones of the formula (I) chosen from the said groups and a salt of a hydroxyphenylalkadione of the formula (I) chosen from the said groups or two or more salts of hydroxyphenylalkadiones of the formula (I) chosen from the said groups.

The structures of the preferred compounds (1) - (11) are given below for illustration.

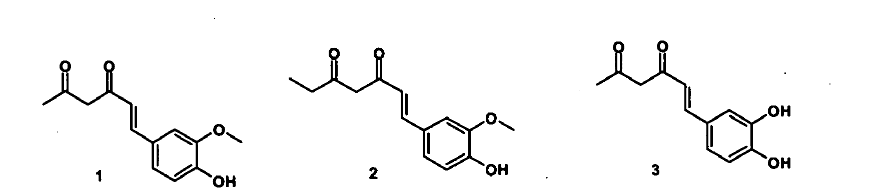

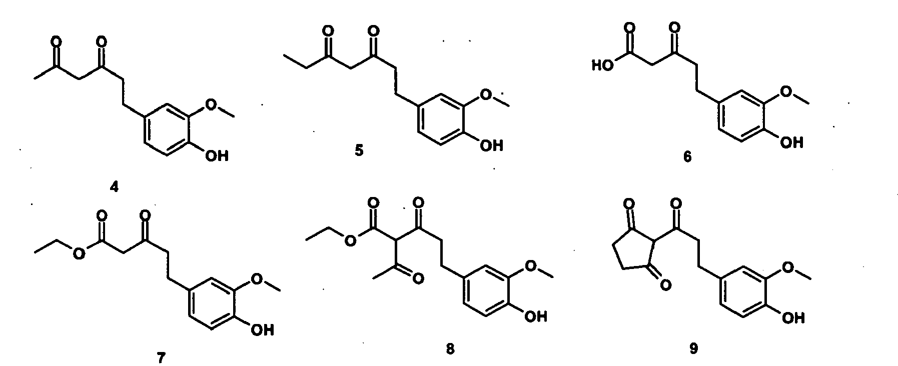

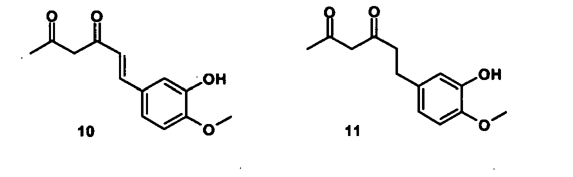

In salts of a compound of the above formula (I) (wherein that stated above continues to apply in respect of the preferred meanings of the radicals and variables) to be used according to the invention, one, several or all of the hydroxyl groups of compound (I) are deprotonated. A corresponding amount of counter-cations is then present, these preferably being chosen from the group consisting of: singly positively charged cations of the first main and sub-group, ammonium ions, trialkylammonium ions, doubly positively charged cations of the second main and sub-group and triply positively charged cations of the third main and sub-group, and mixtures thereof.

It goes without saying that the number of hydroxyl groups in the underlying hydroxyphenylalkadione derivative is decisive for the maximum degree of deprotonation and thus also for the amount of counter-cations present. For example, if two hydroxyl groups in total are present in the underlying hydroxyphenylalkadione derivative, in the case of complete deprotonation of the hydroxyl groups a doubly negatively charged anion is present, so that a corresponding number of positive charges must be provided by the counter-cation(s).

Particularly preferred cations are Na⁺, K⁺, NH₄⁺, Ca²⁺, Mg²⁺, Al³⁺ and Zn²⁺.

The various hydroxyphenylalkadione derivatives according to the invention and salts thereof can of course in each case be used according to the invention by themselves or as mixtures.

A preferred use according to the invention is that in which a compound of the formula (I), a corresponding salt or a corresponding mixture in combination with one, two or more unpleasantly tasting substances is present.

A preferred use according to the invention is also that in which a compound of the formula (I), a corresponding salt or a corresponding mixture in combination with one, two or more sweet- tasting substances or one, two or more aroma substances which cause a sweet odour impression is present. The combination of two or more flavour correctants is then present.

The compounds of the formula (I) (hydroxyphenylalkadione derivatives) to be used according to the invention can be, in the case where at least one of the radicals R² or R³ represents a hydrogen atom, in the form of their tautomers, the ratio of amounts of dione tautomer to keto-enol tautomer being determined by intrinsic and extrinsic physicochemical parameters. The possible tautomeric forms are illustrated by an example where R³ = H:

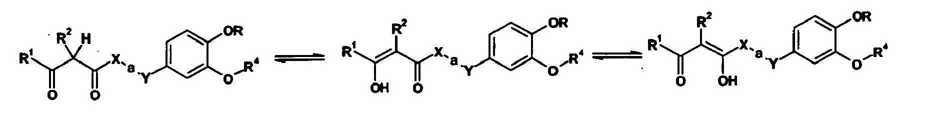

In the context of the present text, also for preferred embodiments, a "compound of the formula (I)" is also to be understood as meaning the particular tautomers.

The use according to the invention of the compounds of the formula (I) (hydroxyphenylalkadione derivatives) is novel.

(5*E*)-6-(4-Hydroxy-3-methoxyphenyl)hex-5-ene-2,4-dione (compound 1) was described as an intermediate product for a synthesis in J. Nat. Prod., vol. 61, year 1998, 609 et seq.

The synthesis and the antioxidative action of hispolone (compound 3) from a parasitic fungus was described in Indian Journal of Chemistry, vol. 41B, April 2002, 875 et seq.

6-(4-Hydroxy-3-methoxyphenyl)hexane-2,4-dione (compound 4), (6*E*)-7-(4-hydroxy-3-methoxyphenyl)hept-6-ene-3,5-dione (compound 2) and 7-(4-hydroxy-3-methoxyphenyl)heptane-3,5-dione (compound 5) were mentioned in EP 245.825 as intermediate products for the synthesis of lipoxygenase inhibitors. (5E)-6-(3-Hydroxy-4-methoxyphenyl)hex-5-ene-2,4-dione (compound 10) was likewise mentioned in EP 245,825 as an intermediate product for the synthesis of lipoxygenase inhibitors.

The compound 6-(3-hydroxy-4-methoxyphenyl)hexane-2,4-dione (compound 11) is novel.

The synthesis of 2-(3-[4-hydroxy-3-methoxyphenyl]propionyl)-cyclopentane-1,3-dione (compound 9), which was found to be a weak antihypertensive agent, was described in DE 2 161,528.

A use of the abovementioned compounds as an aroma or flavouring substance or for influencing aroma or taste was not described or suggested in any of the cases mentioned.

The present invention also relates to the novel compounds of the formula (I), as described above, that stated above in respect of the preferred meaning of the radicals and variables applying accordingly. In this respect, the present invention relates in particular to the compounds
5-(4-hydroxy-3-methoxyphenyl)pentan-3-onoic acid (compound 6),
5-(4-hydroxy-3-methoxyphenyl)pentan-3-onoic acid ethyl ester (compound 7),
2-acetyl-5-(4-hydroxy-3-methoxyphenyl)pentan-3-onoic acid ethyl ester (compound 8),
6-(3-hydroxy-4-methoxyphenyl)hexane-2,4-dione
and
- a salt of such a hydroxyphenylalkadione,
- a mixture comprising or consisting of two, three or four such hydroxyphenylalkadiones,
- a mixture comprising or consisting of salts of two, three or four such hydroxyphenylalkadiones,
or
- a mixture comprising or consisting of one such hydroxyphenylalkadione or two, three or four such hydroxyphenylalkadiones and a salt of one such hydroxyphenylalkadione or two, three or four salts of such hydroxyphenylalkadiones.

It has been found, surprisingly, that the hydroxyphenylalkadione derivatives of the formula (I) used according to the invention can also reduce or even completely suppress the unpleasant taste impression, in particular the bitter taste impression, of a large number of substances, in particular of methylxanthines, such as e.g. caffeine, alkaloids, such as e.g. quinine, flavonoids, such as e.g. naringin, phenols, such as e.g. salicin, pharmaceutical active compounds, such as e.g. denatonium benzoate or β-lactam antibiotics, in very low concentrations, it being particularly advantageous that the hydroxyphenylalkadione derivatives used according to the invention have virtually no intrinsic taste and do not adversely influence the further as a rule not unpleasant taste qualities, in particular even positively influence the sweet taste of sweet substances.

In particular, in utilizing the latter property, it is possible to prepare a ready-to-eat formulation according to the invention having a greatly reduced content of sweet-tasting substances and a content of hydroxyphenylalkadione derivatives of the formula (I) to be used according to the invention which has the same sweetness intensity compared with the original ready-to-eat comparison formulation containing the usual content of sweet-tasting substances.

As already mentioned, one aspect of the present invention is the use of a hydroxyphenylalkadione derivative of the formula (I) or of a corresponding salt or mixture for modifying, masking or reducing the unpleasant taste impression of an unpleasantly tasting substance and/or for intensifying the sweet taste of sweet-tasting substances or the sweet odour impression of aroma substances which cause a sweet odour impression, i.e. as a flavour correctant.

Preferably, the hydroxyphenylalkadione derivative of the formula (I) to be used according to the invention, the salt or the mixture is employed in a formulation for nutrition, oral care or consumption for pleasure or oral pharmaceutical formulation or cosmetic formulation for application in the region of the head, the formulation conventionally comprising one or more unpleasantly tasting substances and/or one or more sweet-tasting substances or aroma substances which have a sweet odour impression..

A further aspect of the present invention relates to such formulations. Formulations according to the invention for nutrition, oral care or consumption for pleasure or cosmetic formulations according to the invention for application in the region of the head preferably comprise (a)
- 0.000001 wt.% to 95 wt.%, based on the total weight of the formulation, of a hydroxyphenylalkadione of the formula (I) 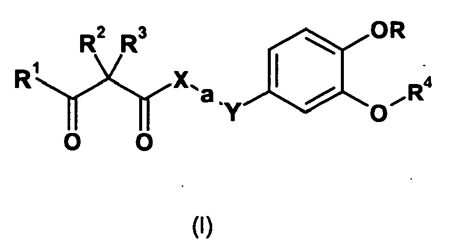 wherein, for X, a and Y:
   X is a -CH₂-, -NH- or -O- group,
   Y is a -CH₂- group and
   a is a single bond
   or
   X and Y are in each case a -CH- group and
   a is a double bond in the Z or E configuration,
   wherein, for R¹, R² and R³
   - R¹: denotes hydrogen, an aliphatic radical having 1 to 4 C atoms or a -O-R⁵, -S-R⁵ or -NR⁵R⁶ group and
   - R² and R³: independently of one another denote hydrogen or an alkyl having 1, 2, 3, 4 or 5 C atoms which is optionally substituted by one or more oxo groups or R⁷-O- groups
   or
   - R¹ and R²: together form an aliphatic ring which contains a total of 4 to 10 carbon atoms and 0 or 1 oxygen atom, wherein the ring constituent represented by R¹ and R² is optionally substituted by one or more oxo groups and/or R⁷-O- groups and/or aliphatic radicals having 1 to 4 C atoms and
   - R³: denotes hydrogen or an alkyl having 1, 2, 3, 4 or 5 C atoms which is optionally substituted by one or more oxo groups or R⁷-O- groups
   or
   - R² and R³: together form an aliphatic ring which contains a total of 4 to 10 carbon atoms and 0 or 1 oxygen atom, wherein the ring constituent represented by R² and R³ is optionally substituted by one or more oxo groups and/or R⁷-O- groups and/or aliphatic radicals having 1 to 4 C atoms and
   - R¹: denotes hydrogen, an aliphatic radical having 1 to 4 C atoms or a -O-R⁵, -S-R⁵ or -NR⁵R⁶ group,
   wherein, for R⁴ and R:
   - R⁴ and R: independently of one another denote hydrogen, methyl or ethyl
   and wherein, for R⁵, R⁶ and R⁷:
   - R⁵ R⁶ and R⁷: independently of one another denote hydrogen or an aliphatic radical having 1 to 4 C atoms or
- a mixture comprising or consisting of two or more different hydroxyphenylalkadiones of the formula (I), wherein X, a, Y, R, R¹, R², R³, R⁴, R⁵, R⁶ and R⁷ in each case have the meaning given above,
- a mixture comprising or consisting of salts of two or more different hydroxyphenylalkadiones of the formula (I), wherein X, a, Y, R, R¹, R², R³, R⁴, R⁵, R⁶ and R⁷ in each case have the meaning given above,
   or
- a mixture comprising or consisting of a hydroxyphenylalkadione of the formula (I) or two or more different hydroxyphenylalkadiones of the formula (I),
wherein X, a, Y, R, R¹, R², R³, R⁴, R⁵, R⁶ and R⁷ in each case have the meaning given above, and a salt of a hydroxyphenylalkadione of the formula (I) or two or more salts of different hydroxyphenylalkadiones of the formula (I), wherein X, a, Y, R, R¹, R², R³, R⁴, R⁵, R⁶ and R⁷ in each case have the meaning given above,
and
(b) one, two or more unpleasantly tasting substances,
(c) one, two or more sweet-tasting substances
and/or
(d) one, two or more aroma substances which cause a sweet odour impression.

Compounds of the formula (I) can also be the preferred embodiments of compounds of the formula (I).

An oral pharmaceutical formulation according to the invention preferably comprises (a)
- 0.000001 wt.% to 10 wt.%, based on the total weight of the formulation, of a hydroxyphenylalkadione of the formula (I) 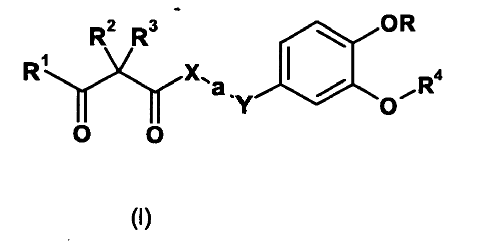 wherein, for X, a and Y:
   X is a -CH₂-, -NH- or -O- group,
   Y is a -CH₂- group and
   a is a single bond
   or
   X and Y are in each case a -CH- group and
   a is a double bond in the Z or E configuration,
   wherein, for R¹, R² and R³
   - R¹: denotes hydrogen, an aliphatic radical having 1 to 4 C atoms or a -O-R⁵, -S-R⁵ or -NR⁵R⁶ group and
   - R² and R³: independently of one another denote hydrogen or an alkyl having 1, 2, 3, 4 or 5 C atoms which is optionally substituted by one or more oxo groups or R⁷-O- groups
   - R¹ and R²: together form an aliphatic ring which contains a total of 4 to 10 carbon atoms and 0 or 1 oxygen atom, wherein the ring constituent represented by R¹ and R² is optionally substituted by one or more oxo groups and/or R⁷-O- groups and/or aliphatic radicals having 1 to 4 C atoms and
   - R³: denotes hydrogen or an alkyl having 1, 2, 3, 4 or 5 C atoms which is optionally substituted by one or more oxo groups or R⁷-O- groups
   or
   - R² and R³: together form an aliphatic ring which contains a total of 4 to 10 carbon atoms and 0 or 1 oxygen atom, wherein the ring constituent represented by R² and R³ is optionally substituted by one or more oxo groups and/or R⁷-O- groups and/or aliphatic radicals having 1 to 4 C atoms and
   - R¹: denotes hydrogen, an aliphatic radical having 1 to 4 C atoms or a -O-R⁵, -S-R⁵ for -NR⁵R⁶ group,
   wherein, for R⁴ and R:
   - R⁴ and R: independently of one another denote hydrogen, methyl or ethyl
   and wherein, for R⁵, R⁶ and R⁷:
   - R⁵ R⁶ and R⁷: independently of one another denote hydrogen or an aliphatic radical having 1 to 4 C atoms,
- a mixture comprising or consisting of two or more different hydroxyphenylalkadiones of the formula (I), wherein X, a, Y, R, R¹, R², R³, R⁴, R⁵, R⁶ and R⁷ in each case have the meaning given above,
- a mixture comprising or consisting of salts of two or more different hydroxyphenylalkadiones of the formula (I), wherein X, a, Y, R, R¹, R², R³, R⁴, R⁵, R⁶ and R⁷ in each case have the meaning given above,
   or
- a mixture comprising or consisting of a hydroxyphenylalkadione of the formula (I) or two or more different hydroxyphenylalkadiones of the formula (I),
wherein X, a, Y, R, R¹, R², R³, R⁴, R⁵, R⁶ and R⁷ in each case have the meaning given above, and a salt of a hydroxyphenylalkadione of the formula (I) or two or more salts of different hydroxyphenylalkadiones of the formula (I), wherein X, a, Y, R, R¹, R², R³, R⁴, R⁵, R⁶ and R⁷ in each case have the meaning given above,
and:
(b) one, two or more unpleasantly tasting substances
   and/or
(c) one, two or more sweet-tasting substances
   and/or
(d) one, two or more aroma substances which cause a sweet odour impression
   and
(e) one, two or more pharmaceutical active compounds which can be administered orally.

Compounds of the formula (I) can also be the preferred embodiments of compounds of the formula (I).

Formulations according to the invention comprising one, two or more unpleasantly tasting substances (b), the amount of the unpleasantly tasting substance (b) being sufficient to be perceived as an unpleasant taste in a comparison formulation which comprises no compound of the formula (I), salt or mixture as described above under (a) but is of otherwise identical composition, and the amount of the compound of the formula (I), salt or mixture as described above under (a) in the formulation being sufficient to mask sensorially the unpleasant taste impression of the unpleasantly tasting substance or to reduce it in comparison with the comparison formulation, are particularly relevant. Compounds of the formula (I) can also be the preferred embodiments of compounds of the formula (I). Formulations according to the invention comprising one, two or more sweet - tasting substances (c) and/or one, two or more aroma substances (d) which cause a sweet odour impression, the total amount of component (a) in the formulation being sufficient to over-proportionally intensify sensorially the sweet taste impression of the sweet-tasting substance(s) (c) or the sweet odour impression of the aroma substance(s) (d) which cause a sweet odour impression are furthermore relevant. Compounds of the formula (I) can also be the preferred embodiments of compounds of the formula (I).

Formulations according to the invention can be in the form of semi-finished goods, in the form of an odoriferous, aroma or flavouring substance composition or in the form of a spice mixture.

In the context of the invention, the formulations for nutrition or consumption for pleasure are e.g. baked goods (e.g. bread, dry biscuits, cakes, other baked products), confectionery (e.g. chocolate, chocolate bar products, other bar products, fruit gum, hard and soft caramels, chewing gum), alcoholic or non-alcoholic drinks (e.g. coffee, tea, wine, wine-containing drinks, beer, beer-containing drinks, liqueurs, schnapps, brandies, fruit-containing carbonated drinks, isotonic drinks, refreshing drinks, nectars, fruit and vegetable juices, fruit or vegetable juice formulations), instant drinks (e.g. instant cocoa drinks, instant tea drinks, instant coffee drinks), meat products (e.g. ham, fresh sausage or uncooked sausage formulations, seasoned or marinated fresh or salted meat products), eggs or egg products (dried egg, egg white, egg yolk), cereal products (e.g. breakfast cereals, muesli bars, precooked ready-made rice products), dairy products (e.g. milk drinks, milk ice, yoghurt, kefir, fresh cheese, soft cheese, hard cheese, dried milk powder, whey, butter, buttermilk, products containing partly or completely hydrolysed milk protein), products from soya protein or other soya bean fractions (e.g. soya milk and products produced therefrom, soya lecithin-containing formulations, fermented products, such as tofu or tempe or products produced therefrom), fruit formulations (e.g. preserves, fruit-flavoured ice-cream, fruit sauces, fruit fillings), vegetable formulations (e.g. ketchup, sauces, dried vegetables, frozen vegetables, precooked vegetables, cooked vegetables), ni b-bles (e.g. baked or fried potato crisps or potato paste products, extrudates basted on maize or peanuts), fat- and oil-based products or emulsions thereof (e.g. mayonnaise, remoulade, dressings), other ready-made dishes and soups (e.g. dried soups, instant soups, precooked soups), spices, spice mixtures and, in particular, sprinkling spices ( seasonings), which are used, for example, in the snacks sector. The formulations in the context of the invention can also be used as semi-finished goods for the preparation of further formulations for nutrition or consumption for pleasure. The formulations in the context of the invention can also be in the form of capsules, tablets (non-coated and coated tablets, e.g. coatings which are resistant to gastric juice), lacquered tablets, granules, pellets, solid mixtures, dispersions in liquid phases, emulsions, powders, solutions, pastes or other formulations as food supplements which can be swallowed or chewed.

In the context of the invention, formulations for oral care are, in particular, oral and/or dental care compositions, such as toothpastes, tooth gels, tooth powders, mouthwashes, chewing gums and other oral care compositions.

Oral pharmaceutical formulations in the context of the invention are formulations which e.g. are in the form of capsules, tablets (non-coated and coated tablets, e.g. coatings which are resistant to gastric juice), lacquered tablets, granules, pellets, solid mixtures, dispersions in liquid phases, emulsions, powders, solutions, pastes or other formulations which can be swallowed or chewed, and are used as medicaments which are available only on prescription or are available only from pharmacies or other medicaments or as food supplements.

Cosmetic formulations for application in the region of the head are, in particular, those which include an unpleasantly tasting substance and may come into contact with the oral cavity even when applied properly to the skin, that is to say, for example - as already mentioned, cosmetic formulations for application in the region of the head, such as soaps, other cleansing or care compositions for the face region, face creams or lotions or ointments, sunscreen agents, beard cleansing or care compositions, shaving foams, soaps or gels, lipsticks or other lip cosmetics or lip care compositions.

Further conventional active compounds, base substances, auxiliary substances and additives for formulations for nutrition, oral care or consumption for pleasure or oral pharmaceutical formulations can be present in amounts of from 5 to 99.999999 wt.%, preferably 10 to 80 wt.%, based on the total weight of the formulation. The formulations can furthermore contain water in an amount of up to 99.999999 wt.%, preferably 5 to 80 wt.%, based on the total weight of the formulation.

The formulations according to the invention comprising one or more of the compounds of the formula (I) according to the invention or their salts or mixtures are prepared according to a preferred embodiment by incorporating the compounds of the formula (I) or their salts or mixtures as solids, as a solution or in the form of a mixture with a solid or liquid carrier substance, into a base formulation for nutrition, oral care or consumption for pleasure or an oral pharmaceutical base formulation. Advantageously, formulations according to the invention in the form of a solution can also be converted into a solid formulation by spray drying. Compounds of the formula (I) can also be the preferred embodiments of compounds of the formula (I).

According to another aspect, the invention relates to a formulation as described above comprising one, two or more sweet-tasting substances (c) and/or one, two or more aroma substances which cause a sweet odour impression, the total amount of component (a) in the formulation being sufficient to over-proportionally intensify sensorially the sweet taste impression of the sweet-tasting substance(s) (c) or the sweet odour impression of the aroma substance(s) (d) which cause a sweet odour impression. Compounds of the formula (I) can also be the preferred embodiments of compounds of the formula (I).

According to a further preferred embodiment, for the preparation of formulations according to the invention, the compounds of the formula (I) according to the invention or their salts or mixtures and optionally other constituents of the formulation according to the invention can also be incorporated beforehand into emulsions, into liposomes, e.g. starting from phosphatidylcholine, into microspheres, into nanospheres or also into capsules, granules or extrudates of a matrix which is suitable for foodstuffs and compositions for consumption for pleasure, e.g. of starch, starch derivatives, cellulose or cellulose derivatives (e.g. hydroxypropylcellulose), other polysaccharides (e.g. alginate), natural fats, natural waxes (e.g. beeswax, carnauba wax) or of proteins, e.g. gelatine. Compounds of the formula (I) can also be the preferred embodiments of compounds of the formula (I).

In a further preferred preparation process, the hydroxyphenylalkadione derivatives (compound of the formula (I)) or their salts or mixtures are complexed beforehand with one or more suitable complexing agents, for example with cycloglycans, e.g. cyclofructans, cyclodextrins or cyclodextrin derivatives, preferably α-, γ- and β-cyclodextrin, and employed in this complexed form.

A formulation according to the invention in which the matrix is chosen such that hydroxyphenylalkadione derivatives are released from the matrix in delayed form, so that a long-lasting aroma action is obtained, is particularly preferred.

Further constituents which can be used for formulations according to the invention for nutrition or consumption for pleasure are conventional base substances, auxiliary substances and additives for foodstuffs or compositions for consumption for pleasure, e.g. water, mixtures of fresh or processed, plant or animal base substances or raw materials (e.g. raw, roasted, dried, fermented, smoked and/or boiled meat, bone, cartilage, fish, vegetables, fruit, herbs, nuts, vegetable or fruit juices or pastes or mixtures thereof), digestible or non-digestible carbohydrates (e.g. sucrose, maltose, fructose, glucose, dextrins, amylose, amylopectin, inulin, xylans, cellulose), sugar alcohols (e.g. sorbitol), natural or hydrogenated fats (e.g. tallow, lard, palm fat, coconut fat, hydrogenated plant fat), oils (e.g. sunflower oil, groundnut oil, maize germ oil, olive oil, fish oil, soya oil, sesame oil), fatty acids or salts thereof (e.g. potassium stearate), proteinogenic or non-proteinogenic amino acids and related compounds (e.g. taurine), peptides, native or processed proteins (e.g. gelatine), enzymes (e.g. peptidases), nucleic acids, nucleotides, flavour correctants for unpleasant taste impressions, flavour correctants for further as a rule not unpleasant taste impressions, taste-modulating substance (e.g. inositol phosphate, nucleotides, such as guanosine monophosphate, adenosine monophosphate or other substances, such as sodium glutamate or 2-phenoxypropionic acid), emulsifiers (e.g. lecithins, diacylglycerols), stabilizers (e.g. carrageenan, alginate), preservatives (e.g. benzoic acid, sorbic acid), antioxidants (e.g. tocopherol, ascorbic acid), chelating agents (e.g. citric acid), organic or inorganic acidifying agents (e.g. malic acid, acetic acid, citric acid, tartaric acid, phosphoric acid), additional bitter principles (e.g. quinine, caffeine, limonin, amarogentin, humolones, lupolones, catechols, tannins), sweeteners (e.g. saccharin, cyclamate, aspartame, neotame), mineral salts (e.g. sodium chloride, potassium chloride, magnesium chloride, sodium phosphates), substances which prevent enzymatic browning (e.g. sulfite, ascorbic acid), essential oils, plant extracts, natural or synthetic dyestuffs or coloured pigments (e.g. carotenoids, flavonoids, anthocyans, chlorophyll and derivatives thereof), spices, substances having a trigeminal action (that is to say cause piquant, pungent, prickling, tickling, astringent, warm or cold effects) or plant extracts containing such substances having a trigeminal action, synthetic, natural or nature-identical aroma substance or odoriferous substances as well as flavour correctants.

Dental care compositions (formulations which serve as a basis for oral care) which comprise the compounds of the formula (I) to be used according to the invention, their salts or mixtures in general comprise an abrasive system (abrasive or polishing agent), such as e.g. silicas, calcium carbonates, calcium phosphates, aluminium oxides and/or hydroxyapatites, surface-active substances, such as e.g. sodium lauryl sulfate, sodium lauryl sarcosinate and/or cocamido-propylbetaine, moisture-retaining agents, such as e.g. glycerol and/or sorbitol, thickeners, such as e.g. carboxymethylcellulose, polyethylene glycols, carrageenan and/or Laponite^{®}, sweeteners, such as e.g. saccharin, flavour correctants for unpleasant taste impressions, flavour correctants for further, as a rule not unpleasant taste impressions, taste-modulating substances (e.g. inositol phosphate, nucleotides, such as guanosine monophosphate, adenosine monophosphate or other substances, such as sodium glutamate or 2-phenoxypropionic acid), cooling active compounds, such as e.g. menthol, menthol derivatives (e.g.
L-menthol, L-menthyl lactate, L-menthyl alkyl carbonates, menthone ketals, menthanecarboxylic acid amides), 2,2,2-trialkylacetic acid amides (e.g. 2,2-diisopropylpropionic acid methylamide), icilin derivatives, stabilizers and active compounds, such as e.g. sodium fluoride, sodium monofluorophosphate, tin difluoride, quaternary ammonium fluorides, zinc citrate, zinc sulfate, tin pyrophosphate, tin dichloride, mixtures of various pyrophosphates, triclosan, cetylpyridinium chloride, aluminium lactate, potassium citrate, potassium nitrate, potassium chloride, strontium chloride, hydrogen peroxide, aromas and/or sodium bicarbonate, or odour correctants.

Chewing gums (as a further example of formulations for oral care) which comprise hydroxyphenylalkadione derivatives to be used according to the invention, their salts or mixtures in general comprise a chewing gum base, i.e. a chewing composition which becomes plastic on chewing, various types of sugars, sugar substitutes, sweeteners, sugar alcohols, flavour correctants for unpleasant taste impressions, flavour correctants for further, as a rule not unpleasant taste impressions, taste-modulating substances (e.g. inositol phosphate, nucleotides, such as guanosine monophosphate, adenosine monophosphate or other substances, such as sodium glutamate or 2-phenoxypropionic acid), the cooling active compounds, humectants, thickeners, emulsifiers, aromas and stabilizers or odour correctants mentioned in the preceding section.

All the conventional further active compounds, base substances, auxiliary substances and additives for oral pharmaceutical formulations can be used as constituents for oral pharmaceutical formulations according to the invention. Unpleasantly tasting pharmaceutical active compounds which can be formulated for oral use can also be used, in particular, as active compounds. The active compounds, base substances, auxiliary substances and additives can be converted into the oral administration forms in a manner known per se. This is regularly effected using inert, non-toxic, pharmaceutically suitable auxiliary substances. These include, inter alia, carrier substances (e.g. microcrystalline cellulose), solvents (e.g. liquid polyethylene glycols), emulsifiers (e.g. sodium dodecyl sulfate), dispersing agents (e.g. polyvinylpyrrolidone), synthetic and natural biopolymers (e.g. albumin), stabilizers (e.g. antioxidants, such as ascorbic acid), dyestuffs (e.g. inorganic pigments, such as iron oxides) and odour correctants as well as flavour correctants which do not relate to the bitter taste.

Preferably, the formulations according to the invention can also comprise an aroma composition in order to round off and refine the taste and/or odour of the formulation. Suitable aromatic compositions comprise e.g. synthetic, natural or nature-identical aroma, odoriferous and flavouring substances as well as suitable auxiliary and carrier substances. In this context, it is regarded as particularly advantageous that a bitter or metallic taste impression which arises from aroma or odoriferous substances contained in the formulations according to the invention can be masked or reduced and therefore the overall aroma or taste profile is improved. An improvement in the taste profile can furthermore also result from the intensification according to the invention of the sweet taste or of the sweet odour impression.

Formulations according to the invention which are in the form of semi-finished goods can serve to mask or reduce the unpleasant taste impression and/or to intensify the sweet taste or odour of finished goods formulations which are prepared using the semi-finished goods formulation.

Formulations according to the invention which preferably serve as semi-finished goods as a rule comprise 0.0001 wt.% to 95 wt.%, preferably 0.001 to 80 wt.%, but in particular 0.01 wt.% to 50 wt.%, based on the total weight of the formulation, of compounds of the formula (I) to be used according to the invention, salts thereof or mixtures thereof. Formulations according to the invention which are in the form of semi-finished goods can serve to mask or reduce the unpleasant taste impression and/or to intensify the sweet taste or odour of finished goods formulations which are prepared using the semi-finished goods formulation.

In a particularly preferred embodiment of the invention, the (a) hydroxyphenylalkadione derivatives according to the invention, their salts or mixtures are in the formulations according to the invention in combination with one or further substances for modifying, masking or reducing the unpleasant taste impression of an unpleasantly tasting substance comprising (b) one, two or more sweet-tasting substances and/or (c) one, two or more aroma substances which cause a sweet odour impression, the total amount of component (a) in the formulation being sufficient to over-proportionally intensify sensorially the sweet taste impression of the sweet-tasting substance(s) (b) or the sweet odour impression of the aroma substance(s) (c) which cause a sweet odour impression. In particular, the combination of the hydroxyphenylalkadione derivatives to be employed according to the invention with other flavour correctants for unpleasant, in particular bitter taste impressions and/or sweet-tasting substances is preferred. Compounds of the formula (I) can also be the preferred embodiments of compounds of the formula (I).

The further flavour correctants can be chosen from the following list, without limiting the invention therewith. Nucleotides (e.g. adenosine 5'-monophosphate, cytidine 5'-monophosphate) or pharmaceutically acceptable salts thereof, lactisols, sodium salts (e.g. sodium chloride, sodium lactate, sodium citrate, sodium acetate, disodium gluconate), hydroxyflavanones (e.g. eriodictyol, homoeriodictyol or sodium salts thereof), in particular according to EP 1 258 200, hydroxybenzoic acid amides (e.g. 2,4-dihydroxybenzoic acid vanillylamide, 2,4-dihydroxybenzoic acid *N*-(4-hydroxy-3-methoxybenzyl)amide, 2,4,6-trihydroxybenzoic acid *N*-(4-hydroxy-3-methoxybenzyl)amide, 2-hydroxy-benzoic acid *N*-4-(hydroxy-3-methoxybenzyl)amide, 4-hydroxybenzoic acid *N*-(4-hydroxy-3-methoxybenzyl)amide, 2,4-dihydroxybenzoic acid *N*-(4-hydroxy-3-methoxybenzyl)amide monosodium salt, 2,4-dihydroxybenzoic acid *N*-2-(4-hydroxy-3-methoxyphenyl)ethylamide, 2,4-dihydroxybenzoic acid *N*-(4-hydroxy-3-ethoxybenzyl)amide, 2,4-dihydroxybenzoic acid N-(3,4-dihydroxybenzyl)amide and 2-hydroxy-5-methoxy-N-[2-(4-hydroxy-3-methoxyphenyl)ethyl]amide (aduncamide), 4-hydroxybenzoic acid vanillylamide), hydroxydeoxybenzoins (e.g. 2-(4-hydroxy-3-methoxyphenyl)-1-(2,4,6-trihydroxyphenyl)ethanone, 1-(2,4-dihydroxyphenyl)-2-(4-hydroxy-3-methoxyphenyl)ethanone, 1-(2-hydroxy-4-methoxyphenyl)-2-(4-hydroxy-3-methoxyphenyl)ethanone), hesperetin, amino acids (e.g. gamma-aminobutyric acid) or mixtures of whey proteins with lecithins.

A further aspect of the present invention relates to a process for modifying, masking or reducing an unpleasant taste impression of an unpleasantly tasting substance, with the following step:
- mixing of one or more unpleasantly tasting substances (component (b)) with a total amount of a component (a)
   - 0.000001 wt.% to 95 wt.%, based on the total weight of the formulation, of a hydroxyphenylalkadione of the formula (I) 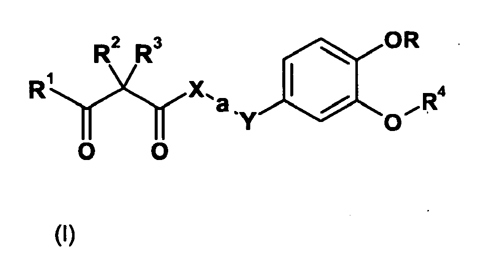 wherein, for X, a and Y:
      X is a -CH₂-, -NH- or -O- group,
      Y is a -CH₂- group and
      a is a single bond
      or
      X and Y are in each case a -CH- group and
      a is a double bond in the Z or E configuration,
      wherein, for R¹, R² and R³
      - R¹: denotes hydrogen, an aliphatic radical having 1 to 4 C atoms or a -O-R⁵, -S-R⁵ or -NR⁵R⁶ group and
      - R² and R³: independently of one another denote hydrogen or an alkyl having 1, 2, 3, 4 or 5 C atoms which is optionally substituted by one or more oxo groups or R⁷-O- groups
      or
      - R¹ and R²: together form an aliphatic ring which contains a total of 4 to 10 carbon atoms and 0 or 1 oxygen atom, wherein the ring constituent represented by R¹ and R² is optionally substituted by one or more oxo groups and/or R⁷-O- groups and/or aliphatic radicals having 1 to 4 C atoms and
      - R³: denotes hydrogen or an alkyl having 1, 2, 3, 4 or 5 C atoms which is optionally substituted by one or more oxo groups or R⁷-O- groups
      or
      - R² and R³: together form an aliphatic ring which contains a total of 4 to 10 carbon atoms and 0 or 1 oxygen atom, wherein the ring constituent represented by R² and R³ is optionally substituted by one or more oxo groups and/or R⁷-O- and/or aliphatic radicals having 1 to 4 C atoms and
      - R¹: denotes hydrogen, an aliphatic radical having 1 to 4 C atoms or a -O-R⁵, -S-R⁵ or -NR⁵R⁶ group,
      wherein, for R⁴ and R:
      - R⁴ and R: independently of one another denote hydrogen, methyl or ethyl
      and wherein, for R⁵, R⁶and R⁷:
      R⁵ R⁶ and R⁷ independently of one another denote hydrogen or an aliphatic radical having 1 to 4 C atoms or
   - a mixture comprising or consisting of two or more different hydroxyphenylalkadiones of the formula (I), wherein X, a, Y, R, R¹, R², R³, R⁴, R⁵, R⁶ and R⁷ in each case have the meaning given above,
   - a mixture comprising or consisting of salts of two or more different hydroxyphenylalkadiones of the formula (I), wherein X, a, Y, R, R¹, R², R³, R⁴, R⁵, R⁶ and R⁷ in each case have the meaning given above,
      or
   - a mixture comprising or consisting of a hydroxyphenylalkadione of the formula (I) or two or more different hydroxyphenylalkadiones of the formula (I), wherein X, a, Y, R, R¹, R², R³, R⁴, R⁵, R⁶ and R⁷ in each case have the meaning given above, and a salt of a hydroxyphenylalkadione of the formula (I) or two or more salts of different hydroxyphenylalkadiones of the formula (I), wherein X, a, Y, R, R¹, R², R³, R⁴, R⁵, R⁶ and R⁷ in each case have the meaning given above,
   wherein the amount of component (b) in the formulation is sufficient to be perceived as an unpleasant taste in a comparison formulation which comprises no component (b) but is otherwise of identical composition, and the amount of component (a) in the formulation is sufficient to mask sensorially the unpleasant taste impression of the unpleasantly tasting substance (b) or to reduce it in comparison with the comparison formulation.

One aspect of the present invention furthermore relates to a process for intensifying the sweet taste of a sweet-tasting substance or the sweet odour impression of an aroma substance which causes a sweet odour impression, with the following step:
- mixing of one or more sweet-tasting substances (component (c)) or one or more aroma substances (component (d)) which cause(s) a sweet odour impression with a total amount of a component (a)
   - 0.000001 wt.% to 95 wt.%, based on the total weight of the formulation, of a hydroxyphenylalkadione of the formula (I) 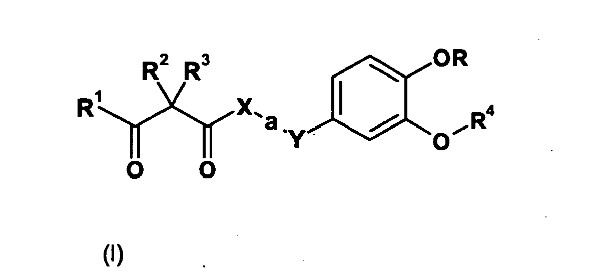 wherein, for X, a and Y:
      X is a -CH₂-, -NH- or -O- group,
      Y is a -CH₂- group and
      a is a single bond
      or
      X and Y are in each case a -CH- group and
      a is a double bond in the Z or E configuration,
      wherein, for R¹, R² and R³
      - R¹: denotes hydrogen, an aliphatic radical having 1 to 4 C atoms or a -O-R⁵, -S-R⁵ or -NR⁵R⁶ group and
      - R² and R³: independently of one another denote hydrogen or an alkyl having 1, 2, 3, 4 or 5 C atoms which is optionally substituted by one or more oxo groups or R⁷-O- groups
      or
      - R¹ and R²: together form an aliphatic ring which contains a total of 4 to 10 carbon atoms and 0 or 1 oxygen atom, wherein the ring constituent represented by R¹ and R² is optionally substituted by one or more oxo groups and/or R⁷-O- groups and/or aliphatic radicals having 1 to 4 C atoms and
      - R³: demotes hydrogen or an alkyl having 1, 2, 3, 4 or 5 C atoms which is optionally substituted by one or more oxo groups or R⁷-O- groups
      or
      - R² and R³: together form an aliphatic ring which contains a total of 4 to 10 carbon atoms and 0 or 1 oxygen atom, wherein the ring constituent represented by R² and R³ is optionally substituted by one or more oxo groups and/or R⁷-O- groups and/or aliphatic radicals having 1 to 4 C atoms and
      - R¹: denotes hydrogen, an aliphatic radical having 1 to 4 C atoms or a -O-R⁵, -S-R⁵ or -NR⁵R⁶ group,
      wherein, for R⁴ and R:
      - R⁴ and R: independently of one another denote hydrogen, methyl or ethyl
      and wherein, for R⁵, R⁶ and R⁷:
      - R⁵ R⁶ and R⁷: independently of one another denote hydrogen or an aliphatic radical having 1 to 4 C atoms or
   - a mixture comprising or consisting of two or more different hydroxyphenylalkadiones of the formula (I), wherein X, a, Y, R, R¹, R², R³, R⁴, R⁵, R⁶ and R⁷ in each case have the meaning given above,
   - a mixture comprising or consisting of salts of two or more different hydroxyphenylalkadiones of the formula (I), wherein X, a, Y, R, R¹, R², R³, R⁴, R⁵, R⁶ and R⁷ in each case have the meaning given above,
   or
- a mixture comprising or consisting of a hydroxyphenylalkadione of the formula (I) or two or more different hydroxyphenylalkadiones of the formula (I); wherein X, a, Y, R, R¹, R², R³, R⁴, R⁵, R⁶ and R⁷ in each case have the meaning given above, and a salt of a hydroxyphenylalkadione of the formula (I) or two or more salts of different hydroxyphenylalkadiones of the formula (I), wherein X, a, Y, R, R¹, R², R³; R⁴, R⁵, R⁶ and R⁷ in each case have the meaning given above,
   the total amount of component (a) in the formulation being sufficient to intensify sensorially the sweet taste impression of the sweet-tasting substance(s) (c) or the sweet odour impression of the aroma substance(s) (d) which cause a sweet odour impression.

### Examples

The examples serve only for illustration of the invention, without limiting this thereto.

### Example 1: (5E)-6-(4-Hydroxy-3-methoxyphenyl)hex-5-ene-2,4-dione (compound 1)

Boron trioxide (3.76 g) and acetyl acetone (7.58 g) are stirred at 100 °C for one hour and then cooled to 0 °C. Tributyl borate (23.0 g) is dissolved in dry ethyl acetate (50 ml) and the solution is added to the mixture. Vanillin (3.76 g) in dry ethyl acetate (30 ml) and then butylamine (0.5 ml) in dry ethyl acetate (3 ml) are added dropwise at 0 °C. The mixture is warmed slowly to room temperature and stirred for a further 3 days. Hydrochloric acid (0.4 mol/l, 50 ml, 60 °C) is added and the phases are separated. The organic phase is washed with water, dried over sodium sulfate, filtered and evaporated in vacuo. The crude product (6.6 g) is chromatographed over silica gel 60 with the eluent n-hexane/ethyl acetate 1:1 (v/v).
Yield 2.97 g (51 %, based on vanillin) of a yellowish oil.
HPLC-MS (RP-18 phase, APCI +): *m*/*z* = 235.19 (100 %, [M+H]⁺).
¹H-NMR (400 MHz, CDCl₃, internal standard TMS, the compound is chiefly in the keto-enol form): δ = 7.53 (1H, d, *J* = 15.8 Hz, H-6), 7,51* (approx. 5 % H, d, *J* = 16.1 Hz), 7.11* (approx. 5 % H, ddd, *J* = 8.2 Hz, *J* = 2 Hz, *J* = 0.5 Hz), 7.09 (1 H, ddd, *J* = 8.2 Hz, *J* = 1.9 Hz, *J* = 0.5 Hz, H-6'), 7.05* (5 % H, d, *J* = 2 Hz), 7.02 (1 H, d, *J* = 1.9 Hz, H-2'), 6.93* (5 % H, d, *J* = 8.2 Hz), 6.92 (1 H, d, *J* = 8.2 Hz, H-5'), 6.63* (approx. 5 % H, d, *J* = 16.1 Hz), 6.32 (1H, d, *J* = 15.8 Hz, H-4), 5.91 (1 H, bs, OH), 5.62 (1 H, s, H-3), 3.94 (3H, s, O-CH₃), 2.16 (3H, s, H-1) ppm.
¹³C-NMR (100 MHz; CDCl₃, internal standard TMS): δ = 197.03 (C, C-2), 177.93 (C, C-4), 147.72 (C, C-3' or C-4'), 146.79 (C, C-4' or C-3'), 140.06 (CH, C-6), 127.67 (CH, C-1'), 122.65 (CH, C-6'), 120.32 (CH, C-5), 114.82 (CH, C-5'), 109.50.(CH, C-2'), 100.71 (CH, C-3), 55.95 (CH₃, O-CH₃), 26.58 (CH₃, C-1) ppm.

### Example 2: 6-(4-Hydroxy-3-methoxyphenyl)hexane-2,4-dione (compound 4)

(5*E*)-6-(4-Hydroxy-3-methoxyphenyl)hex-5-ene-2,4-dione from Example 1 (2.28 g) was hydrogenated with hydrogen with palladium/active charcoal (10 %, moist, 0.23 g) in 100 ml under normal pressure at room temperature for 2 hours (H₂ uptake of 410 ml). The mixture was filtered and the filtrate was evaporated in vacuo. The product was chromatographed over silica gel 60 with the eluent *n*-hexane/ethyl acetate 2:1 (v/v).
Yield: 1.16 g (50 % of theory) of a colourless oil.
HPLC-MS (RP-18 phase, ESI+): *m*/*z* = 236.92 (100 %, [M+H]⁺), 219.08 (94 %, [M-HO]⁺).
HRMS: calc. for C₁₃H₁₆O₄236.1049, found 236.1041.
¹H-NMR (400 MHz, CDCl₃, internal standard TMS, main component enol form, secondary component* keto form): δ = 6.83 (1 H, dd, *J* = 7.2 Hz, *J* = 0.8 Hz, H-5'), 6.82* (10 % H, m, H-5'), 6.69 (1 H, m, H-2'), 6.67 (1 H, ddm, *J* = 7.2 Hz, *J* = 1.6 Hz, H-6'), 5.53 (1H, s, OH), 5.47 (1H, s, H-3), 3.88* (10 % H, s, O-CH₃), 3.86 (3H, s, O-CH₃), 3.54* (10 % 2H, s, H-3), 2.86 (2H, dd, *J* = 8.4 Hz, *J* = 7.2 Hz), 2.82* (10 2H, ddd, *J* = 6.8 Hz, *J* = 4.4 Hz, *J* approx. 1 Hz), 2.559* (10 % 2H, m), 2.558 (2H, dd, *J* = 8.4 Hz, 8.4 Hz), 2.20* (10 % 3H, H-1, s), 2.03 (3H, s, H-1) ppm.
¹³C-NMR (100 MHz; CD₃OD, internal standard TMS): δ = 203.38* (C, C-4), 193.27 (C, C-4), 191.18 (C, C-2), 146.42 (C, C-3'), 144.00 (C-4'), 132.62 (C-1'), 120.82 (C-6'), 114.38- (CH₂, C-5'), 114.34 (CH₂, C-5'), 111.01* (CH₂, C-2'), 110.96 (CH₂, C-2'), 100.13 (CH₂, C-3), 58.13* (CH₃, O-CH₃), 55.88 (CH₃, O-CH₃), 45.53* (CH₂, C-3), 40.40 (CH₂, C-5), 31.29 (CH₂, C-6), 29.15* (CH₂), 24.86 (CH₃, C-1) ppm.

### Example 3: (5E)-6-(4-Hydroxy-3-methoxyphenyl)hex-5-ene-2,4-dione (compound 10)

The compound 10 was prepared analogously to compound 1 according to Example 1 from boric acid anhydride (7.0 g), acetylacetone (14.3 g), tributyl borate (42.6 g), isovanillin (7.05 g) and butylamine (2 ml), and recrystallized in ethyl acetate for purification.
Yield: 4.95 g of yellow-orange crystals (purity > 95 %, HPLC-MS). For analysis, a portion of the product was chromatographed over silica gel 60 with the eluent n-hexane/ethyl acetate 1:1 (v/v) (99.7 % GC)..
¹H-NMR (400 MHz, CDCl₃, internal standard TMS, enol form): δ = 7.51 (1H, d, *J* = 15.79 Hz, H-6), 7.14 (1H, d, *J* = 2.1 Hz, H-2'), 7.02 (1H, ddd, *J* = 8.3 Hz, *J* = 2.1 Hz, *J* = 0.4 Hz, H-6'), 6.85 (1 H, d, *J* = 8.3 Hz, H-5'), 5.69 (1 H, s, OH), 6.32 (1 H, d, *J* = 15.8 Hz, H5), 5.62 (1 H, s, H-3), 3.92 (3H, s, O-CH₃), 2.16 (3H, s, H-1) ppm.
¹³C-NMR (100 MHz; CDCl₃, internal standard TMS): δ = 197.30 (C, C-2), 177.67 (C, C-4), 148.31 (C, C-4'), 145.91 (C, C-3'), 139.72 (CH, C-6), 128.76 (CH, C-1'), 121.86 (CH, C-6'), 120.92 (CH, C-5), 112.67 (CH, C-5'), 110.89 (CH, C-2'), 100.89 (CH, C-3), 56.02 (CH₃, O-CH₃), 26.91 (CH₃, C-1) ppm.

### Example 4: 6-(3-Hydroxy-4-methoxyphenyl)hexane-2,4-dione (compound 11)

The compound 11 wars reduced with hydrogen analogously to compound 4 according to Example 2 from compound 10 (2.34 g) from Example 3 and the crude product (69 % GC) was chromatographed over silica gel with the eluent n-hexane/ethyl acetate 2:1 (v/v).
Yield: 1.0 g (99.0 % GC) of colourless crystals
MS (EI): *m*/*z* = 236 (M⁺, 43 %), 150 (14 %), 137 (100 %), 85 (15 %), 43 (17 %), 28 (14 %).
¹H-NMR (400 MHz, CDCl₃, internal standard TMS, main component enol form): δ = 6.76 (1H, d, *J* = 8.2 Hz, H-5'), 6.77 (1H, d, *J* = 2,1 Hz, H-2'), 6.62 (1 H, ddm, *J* = 8.2 Hz, *J* = 2.1 Hz, H-6'), 5.60 (1H, s, OH), 5.47 (1H, s, H-3), 3.861 (3H, s, O-CH₃), 3.856* (approx. 5 % H, s, O-CH₃), 2.85 (2H, m, H-6), 2.55 (2H, dd, *J* = 9 Hz, 9 Hz), 2.20* (approx. 5 % 3H, H-1, s), 2.04 (3H, s, H-1) ppm.
¹³C-NMR (100 MHz; CD₃OD, internal standard TMS, *keto form): δ = 193.30 (C, C-4), 191.09 (C, C-2), 145.56 (C, C-3'), 145.03 (C, C-4'), 134.00 (C, C-1'), 119.68* (CH, C-6'), 119.65 (CH, C-6'), 114.38* (CH₂, C-5'), 114.34 (CH₂, C-5'), 110.75* (CH, C-2'), 110.70 (CH, C-2'), 100.13 (CH, C-3), 58.07* (CH₃, O-CH₃), 56.00 (CH₃, O-CH₃), 45.34* (CH₂, C-3), 40.10 (CH₂, C-5), 30.89 (CH₂, C-6), 28.84* (CH₂), 24.85 (CH₃, C-1) ppm.

### Use Example 1: Bitterness reduction in a bitter principle solution

In order to quantify the reduction in the bitter impression, the bitterness of an aqueous bitter principle solution and of a sample which contained the same amount of bitter principle and a varying amount of the compound given by way of example was determined by a group of experts (classification 0 [not bitter] to 10 [extremely bitter]). The evaluation was made as a calculation of the reduction (in %) of the bitter impression from the average values of the estimations of the bitter principle solution and of the solutions containing bitter principle and compound given by way of example. 2,4-Dihydrobenzoic acid (2,4-DHB) was used as a comparison from US 5,643,941. HED means homoeriodictyol sodium salt according to EP 1 258 200 B1.

**Table: Bitterness of a bitter principle solution and of a solution containing bitter principle and a compound given by way of example (2,4-DHB = 2,4-dihydroxybenzoic acid, HED = homoeriodictyol sodium salt according to EP 1 258 200 B1). "Testers positive" means the number of testers who were able to detect masking; the 95 % confidence intervals are stated as errors; p<x means the significance according to the Student t test method (cf. statistics textbooks), n.s. means not significant.**

| **Substance** | **Bitter principle** | **Testers** | | **Bitter impression (1-10)** | | **% reduction in the bitter impression** |
|---|---|---|---|---|---|---|
| | | **total** | **positive** | **none** | **with** | |
| 100 ppm 2,4-DHB | 500 ppm caffeine | 15 | 9 | 5.1 ± 1.0 | 5.0 ± 1.0 | 3 %, n.s. |
| 100 ppm compound 1, Example 1 | 500 ppm caffeine | 12 | 9 | 6.4 ± 0.9 | 5.1 ± 1.0 | 21 %, p<0.01 |
| 100 ppm compound 4, Example 2 | 500 ppm caffeine | 17 | 15 | 6.0 ± 0.8 | 3.9 ± 0.8 | 34 %, p<0.001 |
| 100 ppm compound 4, Example 2 | 5 ppm quinine hydrochloride | 16 | 10 | 3.3 ± 0.9 | 1.3 ± 0.8 | 21 %, p<0.05 |
| 50 ppm compound 4 and 250 ppm HED | 500 ppm caffeine | 16 | 13 | 5.1 ± 1.1 | 3.1 ± 0.8 | 39 %, p<0.05 |
| 100 ppm compound 10, Example 3 | 500 ppm caffeine | 16 | 12 | 5.9 ± 1.0 | 4.6 ± 0.9 | 23 % p<0.005 |
| 100 ppm compound 11, Example 4 | 500 ppm caffeine | 15 | 11 | 5.1 ± 1.1 | 4.2 ± 0.8 | 17 %, n.s. |

### Use Example 2: Spray-dried formulation as semi-finished goods for aromatization of finished goods

| **Constituent** | **Amount employed in wt.%** |
|---|---|
| Drinking water | 60.8 % |
| Maltodextrin from wheat | 24.3 % |
| Gum arabic | 6.1 % |
| 6-(4-Hydroxy-3-methoxyphenyl)hexane-2,4-dione (compound 4 from Example 2) | 8.8 % |

The drinking water is initially introduced into a container and the maltodextrin and the gum arabic are dissolved therein. The 6-(4-hydroxy-3-methoxyphenyl)hexane-2,4-dione (Example 2; compound 4) is then emulsified into the carrier substance solution with a Turrax. The temperature of the spray solution should not fall below 30 °C. The mixture is then spray dried (theory temperature intake: 185 - 195°C, theory temperature exit: 70 - 75 °C). The spray-dried semi-finished goods contain approx. 18 -22 % of the active compound from Example 2.

Analogously to this, spray-dried formulations can be prepared as semi-finished goods with compounds from Example 1 (compound 1), Example 3 (compound 10) and Example 4 (compound 11).

### Use Example 3: Combination with sweeteners

0.5 g of one of the spray-dried goods from Use Example 2 is added to 90 g sucrose and 10 g tagatose and the components are mixed. The product can be employed e.g. as a sweetener for coffee or tea.

### Use Example 4: Black tea formulation

| **Constituent** | **Amount employed in wt.%** |
|---|---|
| Black tea, Ceylon, leaf goods | 94.00 % |
| Semi-finished goods from Use Example 2 containing approx. 18 - 22 % 6-(4-hydroxy-3-methoxyphenyl)hexane-2,4-dine (compound 4 from Example 2) | 6 % |

The tea and the semi-finished goods are mixed and the mixture is packed into tea-bags of filter paper. For use, one tea-bag is brewed in 100 - 250 ml of boiling water and allowed to infuse for 2 - 5 min.

### Use Example 5: Black tea formulation in combination with homoeriodictyol sodium salt

| **Constituent** | **Amount employed in wt.%** |
|---|---|
| Black tea, Ceylon, leaf goods | 94.00 % |
| Semi-finished goods from Use Example 2 containing approx. 18 - 22 % 6-(4-hydroxy-3-methoxyphenyl)hexane-2,4-dine (compound 4 from Example 2) | 3 % |
| Semi-finished goods from homoeriodictyol sodium salt according to EP 1258200 B1, spray-dried analogously to Use Example 2 | 3 % |

The tea and the semi-finished goods are mixed and the mixture is packed into tea-bags of filter paper. For use, one tea-bag is brewed in 100 - 250 ml of boiling water and allowed to infuse for 2 - 5 min.

### Use Example 6: Use in a soya drink

The compound (5*E*)6-(4-hydroxy-3-methoxyphenyl)hex-5-ene-2,4-dione from Example 1 was predissolved in ethanol and the solution was added to a soya milk from a local supermarket. The mixture was stirred together with the milk aroma in a glass beaker.

| **Constituent** | **Amount employed in wt.%** |
|---|---|
| Soya milk (local supermarket) | 99.8 % |
| Milk aroma | 0.1 % |
| 10 % (5*E*)-6-(4-hydroxy-3-methoxyphenyl)hex-5-ene-2,4-dione (compound 1 from Example 1) in ethanol | 0.1 % |

### Use Example 7: Use in a soya drink in combination with y-aminobutyric acid

γ-Aminobutyric acid was predissolved in water and 6-(4-hydroxy-3-methoxyphenyl)hexane-2,4-dione (Example 2) was predissolved in ethanol and the solutions were added to a soya milk from a local supermarket. The mixture was stirred together with the milk aroma in a glass beaker.

| **Constituent** | **Amount employed in wt.%** |
|---|---|
| Soya milk (local supermarket) | 99.7 % |
| Milk aroma | 0.1 % |
| 10 % 6-(4-hydroxy-3-methoxyphenyl)hexane-2,4-dione (compound 4 from Example 2) in ethanol | 0.1 % |
| 1 % γ-aminobutyric acid in water | 0.1 % |

### Use Example 8: Use in a chewing gum

| **Part** | **Constituent** | **Amount employed in wt.%** |
|---|---|---|
| A | Chewing gum base, Company "Jagum T" | 30.00 |
| B | Sorbitol, powdered | 39.00 |
| | Isomalt^{®} (Palatinit GmbH) | 9.50 |
| | Xylitol | 2.00 |
| | Mannitol | 3.00 |
| | Aspartame^{®} | 0.10 |
| | Acesulfame^{®} K | 0.10 |
| | Emulgum^{®} (Colloides Naturels, Inc.) | 0.30 |
| C | Sorbitol, 70 % | 14.00 |
| | Glycerol | 1.00 |
| D | Aroma containing 1 % 6-(4-hydroxy-3-methoxyphenyl)hexane-2,4-dione (compound 4 from Example 2), based on the total weight of the aroma | 1 |

Parts A to D are mixed, and kneaded intensively. The crude mass can be processed e.g. in the form of thin strips to give ready-for-consumption chewing gums.

### Use Example 9: Use in a toothpaste

| **Part** | **Constituent** | **Amount employed in wt.%** |
|---|---|---|
| A | Demineralized water | 22.00 |
| | Sorbitol (70 %) | 45.00 |
| | Solbrol^{®} M, sodium salt (Bayer AG, p-hydroxybenzoic acid alkyl ester) | 0.15 |
| | Trisodium phosphate | 0.10 |
| | Saccharin 450 | 0.20 |
| | Sodium monofluorophosphate | 1.12 |
| | Polyethylene glycol 1500 | 5.00 |
| B | Sident 9 (abrasive silicon dioxide) | 10.00 |
| | Sident 22 S (thickening silicon dioxide) | 8.00 |
| | Sodium carboxymethylcellulose | 0.90 |
| | Titanium dioxide | 0.50 |
| C | Demineralized water | 4.53 |
| | Sodium lauryl sulfate | 1.50 |
| D | Aroma containing 1 % 6-(4-hydroxy-3-methoxyphenyl)hexane-2,4-dione (compound 4 from Example 2), based on the total weight of the aroma | 1 |

The constituents of parts A and B are each initially introduced into the mixing vessel in themselves and stirred together thoroughly in vacuo at 25 - 30 °C for 30 min. Part C is weighed out and added to the premixture; D is added and the mixture is stirred thoroughly in vacuo at 25 - 30 °C for 30 min. After release, the toothpaste is finished and can be transferred to containers.

### Use Example 10: Intensification of the sweet impression

In order to quantify the intensification of the sweet impression, the sweetness of a solution containing 5 wt.% of sucrose and of a sample which contained 5 wt.% of sucrose and 100 ppm of the test compound was determined by a group of experts (classification 0 [not sweet] to 10 [extremely sweet]). The evaluation was made as a calculation of the intensification (in %) of the sweet impression from the average values of the estimations of the sucrose solution and of the solution containing sucrose and compound 2.

**Table: Sweetness (a) of a sucrose solution and (b) of a solution containing sucrose and test substance; the standard deviations are stated as errors. In the last column, the total number of testers is stated against the number of testers who found the test solution sweeter.**

| **Test substance** | **Sweet impression (1-10) of a 5 % strength sucrose solution** | | **% intensification of the sweet impression** |
|---|---|---|---|
| | **none** Test substance | **with** Test substance | |
| 6-(4-Hydroxy-3-methoxyphenyl)hex ane-2,4-dione (compound 4 from Example 2) | 4.7 ± 1.4 | 5.3 ± 1.6 | + 13 % (15/9) |
| 6-(3-Hydroxy-4-methoxyphenyl)hex ane-2,4-dione (compound 11 from Example 4) | 5.2 ± 1.2 | 6.6 ± 1.2 | + 28 % (16/13) p<0.005 |

### Use Example 11: Use in a chocolate

The spray-dried formulation from Use Example 2 was incorporated into the chocolate, which was melted at 40 °C, and the liquid composition was poured into a slab mould and cooled by a temperature control programme known to the person skilled in the art, eating chocolate being obtained.

| **Constituent** | **Amount employed in wt.%** |
|---|---|
| Bitter chocolate, min. 85 % cocoa (commercial product) | 99.8 % |
| Spray-dried formulation from Use Example 2 | 0.2 % |

The chocolate prepared in this way was described by the trained persons skilled in the art as less bitter, less astringent and overall more rounded-off.

### Use Example 12: Use in a reduced-sugar refreshing drink

Comparison formulation having a normal sugar content (sucrose content) (A)

Comparison formulation having a reduced sugar content (sucrose content) (B)

Formulation having a reduced sugar content (sucrose content) and compound 11 from Example 4 (C)

| | **Content** | | |
|---|---|---|---|
| **Constituent** | Formulation A | Formulation B | Formulation C |
| **Water** | 89.85 % | 91.85 % | 91.85 % |
| Sucrose | 10.0 % | 8.0 % | 8.0 % |
| Citric acid | 0.15 % | 0.2 % | 0.2 % |
| Compound 11 from Example 4 | - | - | 0.01 % |

The solid substances were initially introduced into the mixing vessel and topped up with water and dissolved. With formulation C, it was possible to achieve the sweetness of comparison formulation A containing 10 % sucrose.

## Claims

1. Use
- of a hydroxyphenylalkadione derivative of the formula (I) 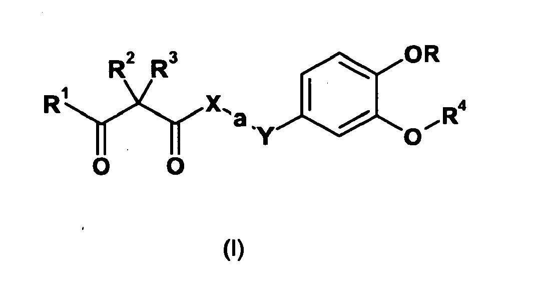 wherein, for X, a and Y:
X is a -CH₂-, -NH- or -O- group,
Y is a -CH₂- group and
a is a single bond
or
X and Y are in each case a -CH- group and
a is a double bond in the Z or E configuration,
wherein, for R¹, R² and R³
R¹ denotes hydrogen, an aliphatic radical having 1 to 4 C atoms or a -O-R⁵, -S-R⁵ or -NR⁵R⁶ group and
R² and R³ independently of one another denote hydrogen or an alkyl having 1, 2, 3, 4 or 5 C atoms which is optionally substituted by one or more oxo groups or R⁷-O- groups
or
R¹ and R² together form an aliphatic ring which contains a total of 4 to 10 carbon atoms and 0 or 1 oxygen atom, wherein the ring constituent represented by R¹ and R² is optionally substituted by one or more oxo groups and/or R⁷-O- groups and/or aliphatic radicals having 1 to 4 C atoms and
R³ denotes hydrogen or an alkyl having 1, 2, 3, 4 or 5 C atoms which is optionally substituted by one or more oxo groups or R⁷-O- groups
or
R² and R³ together form an aliphatic ring which contains a total of 4 to 10 carbon atoms and 0 or 1 oxygen atom, wherein the ring constituent represented by R² and R³ is optionally substituted by one or more oxo groups and/or R⁷-O- groups and/or aliphatic radicals having 1 to 4 C atoms and
R¹ denotes hydrogen, an aliphatic radical having 1 to 4 C atoms or a -O-R⁵, -S-R⁵ or -NR⁵R⁶ group,
wherein, for R⁴ and R:
R⁴ and R independently of one another denote hydrogen, methyl or ethyl
and wherein, for R⁵, R⁶ and R⁷:
R⁵ R⁶ and R⁷ independently of one another denote hydrogen or an aliphatic radical having 1 to 4 C atoms,
- a salt of such a hydroxyphenylalkadione of the formula (I),
- a mixture comprising or consisting of two or more different hydroxyphenylalkadiones of the formula (I), wherein X, a, Y, R, R¹, R², R³, R⁴, R⁵, R⁶ and R⁷ in each case have the meaning given above,
- a mixture comprising or consisting of salts of two or more different hydroxyphenylalkadiones of the formula (I), wherein X, a, Y, R, R¹, R², R³, R⁴, R⁵, R⁶ and R⁷ in each case have the meaning given above,
or
- a mixture comprising or consisting of a hydroxyphenylalkadione of the formula (I) or two or more different hydroxyphenylalkadiones of the formula (I), wherein X, a, Y, R, R¹, R², R³, R⁴, R⁵, R⁶ and R⁷ in each case have the meaning given above, and a salt of a hydroxyphenylalkadione of the formula (I) or two or more salts of different hydroxyphenylalkadiones of the formula (I), wherein X, a, Y, R, R¹, R², R³, R⁴, R⁵, R⁶ and R⁷ in each case have the meaning given above,
for modifying, masking or reducing the unpleasant taste impression of an unpleasantry tasting substance.
and/or
for intensifying the sweet taste of a sweet-tasting substance and/or the sweet odour impression of an aroma substance which causes a sweet odour impression.

2. Use according to claim 1,
wherein, for R⁴ and R:
R⁴ and R independently of one another denote hydrogen, methyl or ethyl, wherein one radical R⁴ or R denotes hydrogen or both radicals R⁴ and R denote hydrogen.

3. Use according to claim 1 or 2,
wherein R denotes hydrogen.

4. Use according to one of the preceding claims, wherein one, several or all of the aliphatic radicals having 1 to 4 C atoms which are optionally present as R¹, R⁵, R⁶ and/or R⁷ denote methyl, ethyl, 1-propyl or 2-propyl.

5. Use according to one of the preceding claims, wherein the alkyl having 1, 2, 3, 4 or 5 C atoms which is optionally substituted by one or more oxo groups or R⁷-O- groups is chosen from the group consisting of: methyl, ethyl, 1-propyl, 2-propyl, 1-oxomethyl, 1-oxoethyl, 1-methyloxymethyl, 1-methyloxyethyl, 1-oxopropyl.

6. Use according to one of the preceding claims, wherein
R¹ and R² together form an aliphatic ring which is five-, six- or seven-membered in total and contains 4 to 7 carbon atoms and 0 or 1 oxygen atom, wherein the ring constituent represented by R¹ and R² is optionally substituted by one or more oxo groups and/or R⁷-O- groups and/or aliphatic radicals having 1 to 4 C atoms.

7. Use according to one of the preceding claims,
wherein, for X, a and Y:
X is a -CH₂- or -NH- group,
Y is a -CH₂- group and
a is a single bond
or
X and Y are in each case a -CH- group and
a is a double bond in the E configuration,
wherein, for R¹, R² and R³
R¹ denotes an aliphatic radical having 1 to 4 C atoms or a -O-R⁵ group,
R² denotes hydrogen or an alkyl having 1, 2, 3, 4 or 5 C atoms which is optionally substituted by one or more oxo groups or R⁷-O- groups and
R³ denotes hydrogen,
or
R¹ and R² together form an aliphatic ring which contains a total of 5 to 7 carbon atoms and 0 or 1 oxygen atom, wherein the ring constituent represented by R¹ and R² is optionally substituted by one or more oxo groups and/or R⁷-O- groups and
R³ denotes hydrogen.
and wherein, for R⁵ and R⁷:
R⁵ and R⁷ independently of one another denote hydrogen or an aliphatic radical having 1 to 4 C atoms.

8. Use according to claim 7,
wherein, for R¹ and R²:
R¹ denotes methyl, ethyl, 1-propyl, 2-propyl or a -O-R⁵ group and
R² denotes hydrogen or an alkyl having 1, 2, 3, 4 or 5 C atoms which is optionally substituted by one or more oxo groups
or
R¹ and R² together form an aliphatic ring which contains a total of 5 to 7 carbon atoms and 0 or 1 oxygen atom, wherein the ring constituent represented by R¹ and R² is optionally substituted by one or more oxo groups and
and wherein, for R⁵_{:}
R⁵ denotes hydrogen, methyl or ethyl.

9. Use according to one of the preceding claims
- of a hydroxyphenylalkadione of the formula (I) chosen from the group consisting of
(5*E*)-6-(4-hydroxy-3-methoxyphenyl)hex-5-ene-2,4-dione,
(6*E*)-7-(4-hydroxy-3-methoxyphenyl)hept-6-ene-3,5-dione,
(5*E*)-6-(3,4-dihydroxyphenyl)hex-5-ene-2,4-dione,
6-(4-hydroxy-3-methoxyphenyl)hexane-2,4-dione,
7-(4-hydroxy-3-methoxyphenyl)heptane-3.5-dione,
5-(4-hydroxy-3-methoxyphenyl)pentan-3-onoic acid,
5-(4-hydroxy-3-methoxyphenyl)pentan-3-onoic acid ethyl ester,
2-acetyl-5-(4-hydroxy-3-methoxyphenyl)pentan-3-onoic acid ethyl ester,
2-(3-[4-hydroxy-3-methoxyphenyl]propionyl)-cyclopentane-1,3-dione,
(5*E*)6-(3-hydroxy-4-methoxyphenyl)hex-5-ene-2,4-dione and
6-(3-hydroxy-4-methoxyphenyl)hexane-2,4-dione
- a salt of a hydroxyphenylalkadione of the formula (I) chosen from the said groups,
- a mixture comprising or consisting of two or more hydroxyphenylalkadiones of the formula (I) chosen from the said groups,
- a mixture comprising or consisting of salts of two or more hydroxyphenylalkadiones of the formula (I) chosen from the said groups,
or
- a mixture comprising or consisting of a hydroxyphenylalkadione of the formula (I) chosen from the said groups or two or more hydroxyphenylalkadiones of the formula (I) chosen from the said groups and a salt of a hydroxyphenylalkadione of the formula (I) chosen from the said groups or two or more salts of hydroxyphenylalkadiones for the formula (I) chosen from the said groups.

10. Use of a salt of a compound of the formula (I) according to one of the preceding claims, wherein one, several or all of the hydroxyl groups of the compound of the formula (I) are deprotonated and a corresponding amount of counter-cations is present, which are chosen from the group consisting of: singly positively charged cations of the first main and sub-group, ammonium ions, trialkylammonium ions, doubly positively charged cations of the second main and sub-group and triply positively charged cations of the third main and sub-group, and mixtures thereof.

11. Use according to one of the preceding claims, in combination with one, two or more unpleasantly tasting substances.

12. Use according to one of the preceding claims, in combination with one, two or more sweet-tasting substances or one, two and/or more aroma substances which cause a sweet odour impression.

13. Use according to one of the preceding claims in a formulation for nutrition, oral care or consumption for pleasure or an oral pharmaceutical formulation or cosmetic formulation for application in the region of the head.

14. Formulation chosen from the group consisting of formulations for nutrition, oral care or consumption for pleasure or cosmetic formulation for application in the region of the head, comprising the following components:
(a)
- 0.000001 wt.% to 95 wt.%, based on the total weight of the formulation, of a hydroxyphenylalkadione of the formula (I) 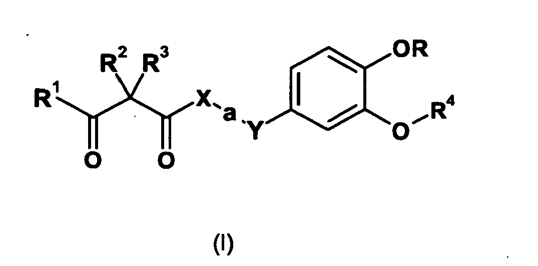 wherein, for X, a and Y:
X is a -CH₂-, -NH- or -O- group,
Y is a -CH₂- group and
a is a single bond
or
X and Y are in each case a -CH- group and
a is a double bond in the Z or E configuration,
wherein, for R¹, R² and R³
R¹ denotes hydrogen, an aliphatic radical having 1 to 4 C atoms or a -O-R⁵, -S-R⁵ or -NR⁵R⁶ group and
R² and R³ independently of one another denote hydrogen or an alkyl having 1, 2, 3, 4 or 5 C atoms which is optionally substituted by one or more oxo groups or R⁷-O- groups
or
R¹ and R² together form an aliphatic ring which contains a total of 4 to 10 carbon atoms and 0 or 1 oxygen atom, wherein the ring constituent represented by R¹ and R² is optionally substituted by one or more oxo groups and/or R⁷-O- groups and/or aliphatic radicals having 1 to 4 C atoms and
R³ denotes hydrogen or an alkyl having 1, 2, 3, 4 or 5 C atoms which is optionally substituted by one or more oxo groups or R⁷-O- groups
or
R² and R³ together form an aliphatic ring which contains a total of 4 to 10 carbon atoms and 0 or 1 oxygen atom, wherein the ring constituent represented by R² and R³ is optionally substituted by one or more oxo groups and/or R⁷-O- groups and/or aliphatic radicals having 1 to 4 C atoms and
R¹ denotes hydrogen, an aliphatic radical having 1 to 4 C atoms or a -O-R⁵, -S-R⁵ or -NR⁵R⁶ group,
wherein, for R⁴ and R:
R⁴ and R independently of one another denote hydrogen, methyl or ethyl
and wherein, for R⁵, R⁶ and R⁷:
R⁵ R⁶ and R⁷ independently of one another denote hydrogen or an aliphatic radical having 1 to 4 C atoms or
- a mixture comprising or consisting of two or more different hydroxyphenylalkadiones of the formula (I), wherein X, a, Y, R, R¹, R², R³, R⁴, R⁵, R⁶ and R⁷ in each case have the meaning given above,
- a mixture comprising or consisting of salts of two or more different hydroxyphenylalkadiones of the formula (I), wherein X, a, Y, R, R¹, R², R³, R⁴, R⁵, R⁶ and R⁷ in each case have the meaning given above,
or
- a mixture comprising or consisting of a hydroxyphenylalkadione of the formula (I) or two or more different hydroxyphenylalkadiones of the formula (I), wherein X, a, Y, R, R¹, R², R³, R⁴, R⁵, R⁶ and R⁷ in each case have the meaning given above, and a salt of a hydroxyphenylalkadione of the formula (I) or two or more salts of different hydroxyphenylalkadiones of the formula (I), wherein X, a, Y, R, R¹, R², R³, R⁴, R⁵, R⁶ and R⁷ in each case have the meaning given above,
and:
(b) one, two or more unpleasantly tasting substances
and/or
(c) one, two or more further sweet-tasting substances
and/or
(d) one, two or more aroma substances which cause a sweet odour impression.

15. Oral pharmaceutical compositions, comprising
(a)
- 0.000001 wt.% to 10 wt.%, based on the total weight of the formulation, of a hydroxyphenylalkadione of the formula (I) 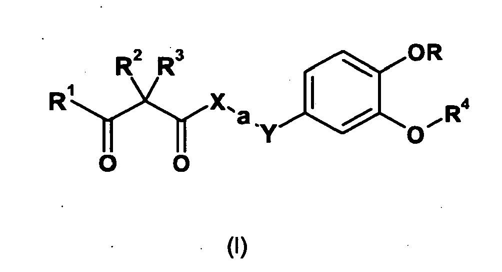 wherein, for X, a and Y:
X is a -CH₂-, -NH- or -O- group,
Y is a -CH₂- group and
a is a single bond
or
X and Y are in each case a -CH- group and
a is a double bond in the Z or E configuration,
wherein, for R¹, R² and R³
R¹ denotes hydrogen, an aliphatic radical having 1 to 4 C atoms or a -O-R⁵, -S-R⁵ or -NR⁵R⁶ group and
R² and R³ independently of one another denote hydrogen or an alkyl having 1, 2, 3, 4 or 5 C atoms which is optionally substituted by one or more oxo groups or R⁷-O- groups
or
R¹ and R² together form an aliphatic ring which contains a total of 4 to 10 carbon atoms and 0 or 1 oxygen atom, wherein the ring constituent represented by R¹ and R² is optionally substituted by one or more oxo groups and/or R⁷-O- groups and/or aliphatic radicals having 1 to 4 C atoms and
R³ denotes hydrogen or an alkyl having 1, 2, 3, 4 or 5 C atoms which is optionally substituted by one or more oxo groups or R⁷-O- groups
or
R² and R³ together form an aliphatic ring which contains a total of 4 to 10 carbon atoms and 0 or 1 oxygen atom, wherein the ring constituent represented by R² and R³ is optionally substituted by one or more oxo groups and/or R⁷-O- groups and/or aliphatic radicals having 1 to 4 C atoms and
R¹ denotes hydrogen, an aliphatic radical having 1 to 4 C atoms or a -O-R⁵, -S-R⁵ or -NR⁵R⁶ group,
wherein, for R⁴ and R:
R⁴ and R independently of one another denote hydrogen, methyl or ethyl
and wherein, for R⁵, R⁶ and R⁷:
R⁵ R⁶ and R⁷ independently of one another denote hydrogen or an aliphatic radical having 1 to 4 C atoms,
- a mixture comprising or consisting of two or more different hydroxyphenylalkadiones of the formula (I), wherein X, a, Y, R, R¹, R², R³, R⁴, R⁵, R⁶ and R⁷ in each case have the meaning given above,
- a mixture comprising or consisting of salts of two or more different hydroxyphenylalkadiones of the formula (I), wherein X, a, Y, R, R¹, R², R³, R⁴, R⁵, R⁶ and R⁷ in each case have the meaning given above,
or
- a mixture comprising or consisting of a hydroxyphenylalkadione of the formula (I) or two or more different hydroxyphenylalkadiones of the formula (I), wherein X, a, Y, R, R¹, R², R³, R⁴, R⁵, R⁶ and R⁷ in each case have the meaning given above, and a salt of a hydroxyphenylalkadione of the formula (I) or two or more salts of different hydroxyphenylalkadiones of the formula (I), wherein X, a, Y, R, R¹, R², R³, R⁴, R⁵, R⁶ and R⁷ in each case have the meaning given above,
and:
(b) one, two or more unpleasantly tasting substances
and/or
(c) one, two or more sweet-tasting substances
and/or
(d) one, two or more aroma substances which cause a sweet odour impression
and
(e) one, two or more pharmaceutical active compounds which can be administered orally.

16. Formulation according to claim 14 or 15, comprising one, two or more unpleasantly tasting substances (b), the amount of the unpleasantly tasting substance (b) being sufficient to be perceived as an unpleasant taste in a comparison formulation which comprises no component (a) but is of otherwise identical composition, and component (a) in the formulation being sufficient to mask sensorially or modify the unpleasant taste impression of the unpleasantly tasting substance or to reduce it in comparison with the comparison formulation.

17. Formulation according claim 14 or 15, comprising one, two or more sweet-tasting substances (c) and/or one, two or more aroma substances (d) which cause a sweet odour impression, the total amount of component (a) in the formulation being sufficient to over-proportionally intensify sensorially the sweet taste impression of the sweet-tasting substance(s) (c) or the sweet odour impression of the aroma substance(s) (d) which cause a sweet odour impression.

18. Formulation according to claim 14 or 15, **characterized in that** it is in the form of semi-finished goods, in the form of an odoriferous, aroma or flavouring substance composition or in the form of a spice mixture.

19. Formulation according to one of claims 16 to 18, furthermore comprising one or more further substances for modifying, masking or reducing the unpleasant taste impression of an unpleasantly tasting substance and/or for intensifying the sweet taste of a sweet-tasting substance and/or the sweet odour impression of an aroma substance which causes a sweet odour impression.

20. 5-(4-hydroxy-3-methoxyphenyl)pentan-3-onoic acid,
5-(4-hydroxy-3-methoxyphenyl)pentan-3-onoic acid ethyl ester,
2-acetyl-5-(4-hydroxy-3-methoxyphenyt)pentan-3-onoic acid ethyl ester and
6-(3-hydroxy-4-methoxyphenyl)hexane-2,4-dione
and
- a salt of such a hydroxyphenylalkadione,
- a mixture comprising or consisting of two, three or four such hydroxyphenylalkadiones,
- a mixture comprising or consisting of salts of two, three or four such hydroxyphenylalkadiones,
or
- a mixture comprising or consisting of
one such hydroxyphenylalkadione or two, three or four such hydroxyphenylalkadiones and
a salt of one such hydroxyphenylalkadione or two, three or four salts of such hydroxyphenylalkadiones.

21. Process for modifying, masking or reducing an unpleasant taste impression of an unpleasantly tasting substance, with the following step:
- mixing of one or more unpleasantly tasting substances (component (b)) with a total amount of a component (a)
- 0.000001 wt.% to 95 wt.%, based on the total weight of the formulation, of a hydroxyphenylalkadione of the formula (I) 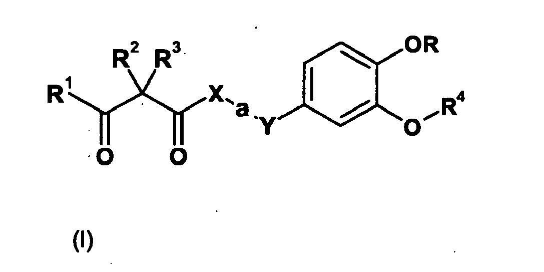
wherein, for X, a and Y:
X is a -CH₂-, -NH- or -O- group,
Y is a -CH₂- group and
a is a single bond
or
X and Y are in each case a -CH- group and
a is a double bond in the Z or E configuration,
wherein, for R¹, R² and R³
R¹ denotes hydrogen, an aliphatic radical having 1 to 4 C atoms or a -O-R⁵, -S-R⁵ or -NR⁵R⁶ group and
R² and R³ independently of one another denote hydrogen or an alkyl having 1, 2, 3, 4 or 5 C atoms which is optionally substituted by one or more oxo groups or R⁷-O- groups
or
R¹ and R² together form an aliphatic ring which contains a total of 4 to 10 carbon atoms and 0 or 1 oxygen atom, wherein the ring constituent represented by R¹ and R² is optionally substituted by one or more oxo groups and/or R⁷-O- groups and/or aliphatic radicals having 1 to 4 C atoms and
R³ denotes hydrogen or an alkyl having 1, 2, 3, 4 or 5 C atoms which is optionally substituted by one or more oxo groups or R⁷-O- groups
R² and R³ together form an aliphatic ring which contains a total of 4 to 10 carbon atoms and 0 or 1 oxygen atom, wherein the ring constituent represented by R² and R³ is optionally substituted by one or more oxo groups and/or R⁷-O- groups and/or aliphatic radicals having 1 to 4 C atoms and
R¹ denotes hydrogen, an aliphatic radical having 1 to 4 C atoms or a -O-R⁵, -S-R⁵ or -NR⁵R⁶group,
wherein, for R⁴ and R:
R⁴ and R independently of one another denote hydrogen, methyl or ethyl
and wherein, for R⁵, R⁶ and R⁷:
R⁵ R⁶ and R⁷ independently of one another denote hydrogen or an aliphatic radical having 1 to 4 C atoms or
- a mixture comprising or consisting of two or more different hydroxyphenylalkadiones of the formula (I), wherein X, a, Y, R, R¹, R², R³, R⁴, R⁵, R⁶ and R⁷ in each case have the meaning given above,
- a mixture comprising or consisting of salts of two or more different hydroxyphenylalkadiones of the formula (I), wherein X, a, Y, R, R¹, R², R³, R⁴, R⁵, R⁶ and R⁷ in each case have the meaning given above,
or
- a mixture comprising or consisting of a hydroxyphenylalkadione of the formula (I) or two or more different hydroxyphenylalkadiones of the formula (I), wherein X, a, Y, R, R¹, R², R³, R⁴, R⁵, R⁶ and R⁷ in each case have the meaning given above, and a salt of a hydroxyphenylalkadione of the formula (I) or two or more salts of different hydroxyphenylalkadiones of the formula (I),
wherein X, a, Y, R, R¹, R², R³, R⁴, R⁵, R⁶ and R⁷ in each case have the meaning given above,
the amount of component (b) in the formulation being sufficient to be perceived as an unpleasant taste in a comparison formulation which comprises no component (b) but is otherwise of identical composition, and the amount of component (a) in the formulation being sufficient to mask sensorially the unpleasant taste impression of the unpleasantly tasting substance (b) or to reduce it in comparison with the comparison formulation.

22. Process for intensifying the sweet taste of a sweet-tasting substance or the sweet odour impression of an aroma substance which causes a sweet odour impression, with the following step:
- mixing of one or more sweet-tasting substances (component (c)) or one or more aroma substances (component (d)) which cause(s) a sweet odour impression with a total amount of a component (a)
- 0.000001 wt.% to 95 wt.%, based on the total weight of the formulation, of a hydroxyphenylalkadione of the formula (I) 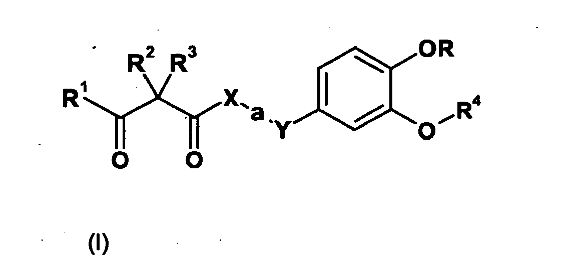 wherein, for X, a and Y:
X is a -CH₂-, -NH- or -O- group,
Y is a -CH₂- group and
a is a single bond
or
X and Y are in each case a -CH- group and
a is a double bond in the Z or E configuration,
wherein, for R¹, R² and R³
R¹ denotes hydrogen, an aliphatic radical having 1 to 4 C atoms or a -O-R⁵, -S-R⁵ or -NR⁵R⁶ group and
R² and R³ independently of one another denote hydrogen or an alkyl having 1, 2, 3, 4 or 5 C atoms which is optionally substituted by one or more oxo groups or R⁷-O- groups
or
R¹ and R² together form an aliphatic ring which contains a total of 4 to 10 carbon atoms and 0 or 1 oxygen atom, wherein the ring constituent represented by R¹ and R² is optionally substituted by one or more oxo groups and/or R⁷-O- groups and/or aliphatic radicals having 1 to 4 C atoms and
R³ denotes hydrogen or an alkyl having 1, 2, 3, 4 or 5 C atoms which is optionally substituted by one or more oxo groups or R⁷-O- groups
or
R² and R³ together form an aliphatic ring which contains a total of 4 to 10 carbon atoms and 0 or 1 oxygen atom, wherein the ring constituent represented by R² and R³ is optionally substituted by one or more oxo groups and/or R⁷-O- groups and/or aliphatic radicals having 1 to 4 C atoms and
R¹ denotes hydrogen, an aliphatic radical having 1 to 4 C atoms or a -O-R⁵, -S-R⁵ or -NR⁵R⁶ group,
wherein, for R⁴ and R:
R⁴ and R independently of one another denote hydrogen, methyl or ethyl
and wherein, for R⁵, R⁶ and R⁷:
R⁵ R⁶ and R⁷ independently of one another denote hydrogen or an aliphatic radical having 1 to 4 C atoms or
- a mixture comprising or consisting of two or more different hydroxyphenylalkadiones of the formula (I), wherein X, a, Y, R, R¹, R², R³, R⁴, R⁵, R⁶ and R⁷ in each case have the meaning given above,
- a mixture comprising or consisting of salts of two or more different hydroxyphenylalkadiones of the formula (I), wherein X, a, Y, R, R¹, R², R³, R⁴, R⁵, R⁶ and R⁷ in each case have the meaning given above,
or
- a mixture comprising or consisting of a hydroxyphenylalkadione of the formula (I) or two or more different hydroxyphenylalkadiones of the formula (I), wherein X, a, Y, R, R¹, R², R³, R⁴, R⁵, R⁶ and R⁷ in each case have the meaning given above, and a salt of a hydroxyphenylalkadione of the formula (I) or two or more salts of different hydroxyphenylalkadiones of the formula (I), wherein X, a, Y, R, R¹, R², R³, R⁴, R⁵, R⁶ and R⁷ in each case have the meaning given above, the total amount of component (a) in the formulation being sufficient to intensify sensorially the sweet taste impression of the sweet-tasting substance(s) (c) or the sweet odour impression of the aroma substance(s) (d) which cause a sweet odour impression.

## Patentansprüche

1. Verwendung
- eines Hydroxyphenylalkadion-Derivates der Formel (I) 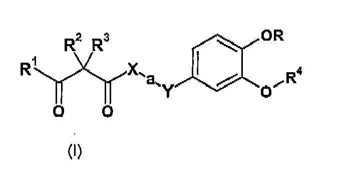
wobei für X, a und Y gilt:
X ist eine Gruppe-CH₂-, -NH- oder -O-,
Y ist eine Gruppe -CH₂- und
a ist eine Einfachbindung
oder
X und Y sind jeweils eine Gruppe -CH- und
a ist eine in Z- oder E-Konfiguration vorliegende Doppelbindung,
wobei für R¹, R² und R³ gilt:
R¹ bedeutet Wasserstoff, aliphatischer Rest mit 1 bis 4 C-Atomen oder eine Gruppe -O-R⁵, -S-R⁵ oder -NR⁵R⁶ und
R² und R³ bedeuten unabhängig voneinander Wasserstoff oder ein Alkyl mit 1, 2, 3, 4 oder 5 C-Atomen, das gegebenenfalls durch eine oder mehrere Oxo-Gruppen oder Gruppen R⁷-O- substituiert ist
oder
R¹ und R² bilden zusammen einen aliphatischen Ring, der insgesamt 4 bis 10 Kohlenstoffatome und 0 oder 1 Sauerstoffatom umfasst, wobei der durch R¹ und R² dargestellte Ringbestandteil gegebenenfalls durch eine oder mehrere Oxo-Gruppen und/oder Gruppen R⁷-O- und/oder aliphatische Reste mit 1 bis 4 C-Atomen substituiert ist und
R³ bedeutet Wasserstoff oder ein Alkyl mit 1, 2, 3, 4 oder 5 C-Atomen, das gegebenenfalls durch eine oder mehrere Oxo-Gruppen oder Gruppen R⁷-O- substituiert ist
oder
R² und R³ bilden zusammen einen aliphatischen Ring, der insgesamt 4 bis 10 Kohlenstoffatome und 0 oder 1 Sauerstoffatom umfasst, wobei der durch R² und R³ dargestellte Ringbestandteil gegebenenfalls durch eine oder mehrere Oxo-Gruppen und/oder Gruppen R⁷-O-, und/oder aliphatische Reste mit 1 bis 4 C-Atomen substituiert ist und
R¹ bedeutet Wasserstoff, ein aliphatischer Rest mit 1 bis 4 C-Atomen oder eine Gruppe-O-R⁵, -S-R⁵ oder -NR⁵R⁶,
wobei für R⁴ und R gilt:
R⁴ und R bedeuten unabhängig voneinander Wasserstoff, Methyl oder Ethyl und wobei für R⁵, R⁶ und R⁷ gilt:
R⁵, R⁶ und R⁷ bedeuten unabhängig voneinander Wasserstoff oder einen aliphatischen Rest mit 1 bis 4 C-Atomen,
- eines Salz eines solchen Hydroxyphenylalkadions der Formel (I),
- einer Mischung umfassend oder bestehend aus zwei oder mehreren verschiedenen Hydroxyphenylalkadionen der Formel (I), wobei X, a, Y, R, R¹, R², R³, R⁴, R⁵, R⁶ und R⁷ jeweils die oben gegebene Bedeutung haben,
- einer Mischung umfassend oder bestehend aus Salzen von zwei oder mehreren verschiedenen Hydroxyphenylalkadionen der Formel (I), wobei X, a, Y, R, R¹, R², R³, R⁴, R⁶, R⁶ und R⁷ jeweils die oben gegebene Bedeutung haben,
oder
- einer Mischung umfassend oder bestehend aus einem Hydroxyphenylalkadion der Formel (I) oder zwei oder mehreren verschiedenen Hydroxyphenylalkadionen der Formel (I), wobei X, a, Y, R, R¹, R², R³, R⁴, R⁵, R⁶ und R⁷ jeweils die oben gegebene Bedeutung haben, und einem Salz eines Hydroyphenylalkadions der Formel (I) oder zwei oder mehreren Salzen von verschiedenen Hydroxyphenylalkadionen der Formel (I), wobei X, a, Y, R, R¹, R², R³, R⁴, R⁶, R⁶ und R⁷ jeweils die oben gegebene Bedeutung haben.
zur Veränderung, Maskierung oder Verminderung des unangenehmen Geschmackseindrucks eines unangenehm schmeckenden Stoffes
und/oder
zur Intensivierung des süßen Geschmacks eines süß schmeckenden Stoffes und/oder des süßen Geruchseindrucks eines Aromastoffes, der einen süßen Geruchseindruck vermittelt.

2. Verwendung nach Anspruch 1,
wobei für R⁴ und R gilt:
R⁴ und R bedeuten unabhängig voneinander Wasserstoff, Methyl oder Ethyl,
wobei ein Rest R⁴ oder R bedeutet Wasserstoff oder beide Reste R⁴ und R bedeuten Wasserstoff.

3. Verwendung nach Anspruch 1 oder 2, wobei R Wasserstoff bedeutet.

4. Verwendung nach einem der vorangehenden Ansprüche, wobei einer, mehrere oder alle der aliphatischen Reste mit 1 bis 4 C-Atomen, die gegebenenfalls als R¹, R⁵, R⁶ und/oder R⁷ vorliegen, Methyl, Ethyl, 1-Propyl oder 2-Propyl bedeuten.

5. Verwendung nach einem der vorangehenden Ansprüche, wobei das Alkyl mit 1, 2, 3, 4 oder 5 C-Atomen, das gegebenenfalls durch eine oder mehrere Oxo-Gruppen oder Gruppen R⁷-O- substituiert ist, ausgewählt ist aus der Gruppe bestehend aus: Methyl, Ethyl, 1-Propyl, 2-Propyl, 1-Oxomethyl, 1-Oxoethyl. 1-Methyloxymethyl, 1-Methyloxyethyl, 1-Oxopropyl.

6. Verwendung nach einem der vorangehenden Ansprüche, wobei
R¹ und R² zusammen einen insgesamt fünf-, sechs- oder siebengliedrigen aliphatischen Ring bilden, der 4 bis 7 Kohlenstoffatome und 0 oder 1 Sauerstoffatom umfasst, wobei der durch R¹ und R² dargestellte Ringbestandteil gegebenenfalls durch eine oder mehrere Oxo-Gruppen und/oder Gruppen R⁷-O- und/oder aliphatische Reste mit 1 bis 4 C-Atomen substituiert ist.

7. Verwendung nach einem der vorangehenden Ansprüche,
wobei für X, a und Y gilt:
X ist eine Gruppe -CH₂- oder -NH-,
Y ist eine Gruppe -CH₂- und
a ist eine Einfachbindung
oder
X und Y sind jeweils eine Gruppe -CH- und
a ist eine in E-Konfiguration vorliegende Doppelbindung,
wobei für R¹, R² und R³ gilt:
R¹ bedeutet ein aliphatischer Rest mit 1 bis 4 C-Atomen oder eine Gruppe -O-R⁵,
R² bedeutet Wasserstoff oder ein Alkyl mit 1, 2, 3, 4 oder 5 C-Atomen, das gegebenenfalls durch eine oder mehrere Oxo-Gruppen oder Gruppen R⁷-O- substituiert ist, und
R³ bedeutet Wasserstoff,
oder
R¹ und R² bilden zusammen einen aliphatischen Ring, der insgesamt 5 bis 7 Kohlenstoffatome und 0 oder 1 Sauerstoffatom umfasst, wobei der durch R¹ und R² dargestellte Ringbestandteil gegebenenfalls durch eine oder mehrere Oxo-Gruppen und/oder Gruppen R⁷-O- substituiert ist und
R³ bedeutet Wasserstoff
und wobei für R⁵ und R⁷ gilt:
R⁵ und R⁷ bedeuten unabhängig voneinander Wasserstoff oder ein aliphatischer Rest mit 1 bis 4 C-Atomen.

8. Verwendung nach Anspruch 7,
wobei für R¹ und R² gilt:
R¹ bedeutet Methyl, Ethyl, 1-Propyl, 2-Propyl oder eine Gruppe -O-R⁶ und
R² bedeutet Wasserstoff oder ein Alkyl mit 1, 2, 3, 4 oder 5 C-Atomen, das gegebenenfalls durch eine oder mehrere Oxo-Gruppen substituiert ist
oder
R¹ und R² bilden zusammen einen aliphatischen Ring, der insgesamt 5 bis 7 Kohlenstoffatome und 0 oder 1 Sauerstoffatom umfasst, wobei der durch R¹ und R² dargestellte Ringbestandteil gegebenenfalls durch eine oder mehrere Oxo-Gruppen substituiert ist
und wobei für R⁶ gilt:
R⁵ bedeutet Wasserstoff, Methyl oder Ethyl.

9. Verwendung nach einem der vorangehenden Ansprüche
- eines Hydroxyphenylalkadions der Formel (I) ausgewählt aus der Gruppe bestehend aus
(5*E*)-6-(4-Hydroxy-3-methoxyphenyl)hex-5-en-2,4-dion,
(6*E*)-7-(4-Hydroxy-3-methoxyphenyl)hept-6-en-3,5-dion,
(5*E*)-6-(3,4-Dihydroxyphenyl)hex-5-en-2,4-dion,
6-(4-Hydroxy-3-methoxyphenyl)hexan-2,4-dion,
7-(4-Hydroxy-3-methoxyphenyl)heptan-3,5-dion,
5-(4-Hydroxy-3-methoxyphenyl)pentan-3-onsäure,
5-(4-Hydroxy-3-methoxyphenyl)pentan-3-onsäureethylester,
2-Acetyl-5-(4-hydroxy-3-methoxyphenyl)pentan,3-onsäureethylester,
2-(3-[4-Hydroxy-3-methoxyphenyl]propionyl)-cyclopentan-1,3-dion,
(5*E*)-6-(3-Hydroxy-4-methoxyphenyl)hex-5-en-2,4-dion,
6-(3-Hydroxy-4-methoxyphenyl)hexan-2,4-dion
- eines Salzes eines Hydroxyphenylalkadions der Formel (I) ausgewählt aus den besagten Gruppen,
- einer Mischung umfassend oder bestehend aus zwei oder mehreren Hydroxyphenylalkadionen der Formel (I) ausgewählt aus den besagten Gruppen,
- einer Mischung umfassend oder bestehend aus Salzen zweier oder mehrerer Hydroxyphenylalkadionen der Formel (I) ausgewählt aus den besagten Gruppen, oder
- einer Mischung umfassend oder bestehend aus einem Hydroxyphenylalkadion der Formel (I) ausgewählt aus den besagten Gruppen oder zwei oder mehreren Hydroxyphenylalkadionen der Formel (I) ausgewählt aus den besagten Gruppen und einem Salz eines Hydroxyphenylalkadions der Formel (I) ausgewählt aus den besagten Gruppen oder zwei oder mehreren Salzen von Hydroxyphenylalkadionen der Formel (I) ausgewählt aus den besagten Gruppen.

10. Verwendung eines Salzes einer Verbindung der Formel (I) nach einem der vorrangehenden Ansprüche wobei eine, mehrere oder sämtliche Hydroxy-Gruppen der Verbindung der Formel (I) deprotoniert sind und eine entsprechende Menge von Gegenkationen vorliegt, die ausgewählt sind aus der Gruppe bestehend aus; einfach positiv geladene Kationen der ersten Haupt- und Nebengruppe, Ammoniumionen, Trialkylammoniumionen, zweifach positiv geladene Kationen der zweiten Haupt- und Nebengruppe und dreifach positiv geladene Kationen der dritten Haupt- und Nebengruppe, und deren Mischungen.

11. Verwendung nach einem der vorangehenden Ansprüche, in Kombination mit einem, zwei oder mehreren unangenehm schmeckenden Stoffen.

12. Verwendung nach einem der vorangehenden Ansprüche, in Kombination mit einem, zwei oder mehreren süß schmeckenden Stoffen oder einem, zwei oder mehreren Aromastoffen, die einen süßen Geruchseindruck vermitteln.

13. Verwendung nach einem der vorangehenden Ansprüche in einer der Ernährung, der Mundpflege oder dem Genuss dienenden oder oralen pharmazeutischen oder zur Applikation im Bereich des Kopfes kosmetischen Zubereitung.

14. Zubereitung ausgewählt aus der Gruppe bestehend aus der Ernährung, der Mundpflege oder dem Genuss dienenden oder zur Applikation im Bereich des Kopfes kosmetischen Zubereitungen, umfassend die folgenden Komponenten:
(a)
- 0,000001 Gew.-% bis 95 Gew.-%, bezogen auf das Gesamtgewicht der Zubereitung, eines Hydroxyphenylalkadions der Formel (I) 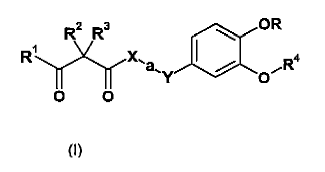 wobei für X, a und Y gilt:
X ist eine Gruppe -CH₂-, -NH- oder -O-,
Y ist eine Gruppe -CH₂- und
a ist eine Einfachbindung
oder
X und Y sind jeweils eine Gruppe -CH- und
a ist eine in Z- oder E-Konfiguration vorliegende Doppelbindung,
wobei für R¹, R² und R³ gilt:
R¹ bedeutet Wasserstoff, ein aliphatischer Rest mit 1 bis 4 C-Atomen oder eine Gruppe -O-R⁵, -S-R⁵ oder -NR⁵R⁶ und
R² und R³ bedeuten unabhängig voneinander Wasserstoff oder ein Alkyl mit 1, 2, 3, 4 oder 5 C-Atomen, das gegebenenfalls durch eine oder mehrere Oxo-Gruppen oder Gruppen R⁷-O- substituiert ist
oder
R¹ und R² bilden zusammen einen aliphatischen Ring, der insgesamt 4 bis 10 Kohlenstoffatome und 0 oder 1 Sauerstoffatom umfasst, wobei der durch R¹ und R² dargestellte Ringbestandteil gegebenenfalls durch eine oder mehrere Oxo-Gruppen und/oder Gruppen R⁷-O- und/oder aliphatische Reste mit 1 bis 4 C-Atomen substituiert ist und
R³ bedeutet Wasserstoff oder ein Alkyl mit 1, 2, 3, 4 oder 5 C-Atomen, das gegebenenfalls durch eine oder mehrere Oxo-Gruppen oder Gruppen R⁷-O- substituiert ist
oder
R² und R³ bilden zusammen einen aliphatischen Ring, der insgesamt 4 bis 10 Kohlenstoffatome und 0 oder 1 Sauerstoffatom umfasst, wobei der durch R² und R³ dargestellte Ringbestandteil gegebenenfalls durch eine oder mehrere Oxo-Gruppen und/oder Gruppen R⁷-O- und/oder aliphatische Reste mit 1 bis 4 C-Atomen substituiert ist und
R¹ bedeutet Wasserstoff, ein aliphatischer Rest mit 1 bis C-Atomen oder eine Gruppe -O-R⁵, -S-R⁵ oder -NR⁵R⁶,
wobei für R⁴ und R gilt:
R⁴ und R bedeuten unabhängig voneinander Wasserstoff, Methyl oder Ethyl und wobei für R⁵, R⁶ und R⁷ gilt:
R⁵, R⁶ und R⁷ bedeuten unabhängig voneinander Wasserstoff oder ein aliphatischer Rest mit 1 bis 4 C-Atomen oder
- Mischung umfassend oder bestehend aus zwei oder mehreren verschiedenen Hydroxyphenylalkadionen der Formel (I), wobei X, a, Y, R, R¹, R², R³, R⁴, R⁵, R⁶ und R⁷ jeweils die oben gegebene Bedeutung haben,
- Mischung umfassend oder bestehend aus Salzen zweier oder mehrerer verschiedener Hydroxyphenylalkadionen der Formel (I), wobei X, a, Y, R, R¹, R², R³, R⁴, R⁵, R⁶ und R⁷ jeweils die oben gegebene Bedeutung haben,
oder
- Mischung umfassend oder bestehend aus einem Hydroxyphenylalkadion der Formel (I) oder zwei oder mehreren verschiedenen Hydroxyphenylalkadionen der Formel (I),
wobei X, a, Y, R, R¹, R², R³, R⁴, R⁵, R⁶ und R⁷ jeweils die oben gegebene Bedeutung haben, und einem Salz eines Hydroyphenylalkadions der Formel (I) oder zwei oder mehreren Salzen von verschiedenen Hydroxyphenylalkadionen der Formel (I), wobei X, a, Y, R, R¹, R², R³, R⁴, R⁵, R⁶ und R⁷ jeweils die oben gegebene Bedeutung haben,
und
(b) ein, zwei oder mehrere unangenehm schmeckende Stoffe
und/oder
(c) ein, zwei oder mehrere weitere süß schmeckende Stoffe
und/oder
(d) ein, zwei oder mehrere Aromastoffe, die einen süßen Geruchseindruck vermitteln.

15. Orale pharmazeutische Zubereitung, umfassend
(a)
- 0,000001 Gew.-% bis 10 Gew.%, bezogen auf das Gesamtgewicht der Zubereitung, eines Hydroxyphenylalkadions der Formel (I) 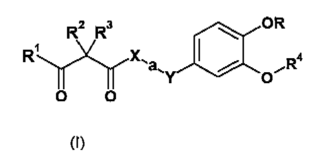 wobei für X, a und Y gilt:
X ist eine Gruppe -CH₂-, -NH- oder -O-,
Y ist eine Gruppe -CH₂- und
a ist eine Einfachbindung
oder
X und Y sind jeweils eine Gruppe -CH- und
a ist eine in Z- oder E-Konfiguration vorliegende Doppelbindung,
wobei für R¹, R² und R³ gilt:
R¹ bedeutet Wasserstoff, ein aliphatischer Rest mit 1 bis 4 C-Atomen oder eine Gruppe -O-R⁵, -S-R⁵ oder -NR⁵R⁶ und
R² und R³ bedeuten unabhängig voneinander Wasserstoff oder ein Alkyl mit 1, 2, 3, 4 oder 5 C-Atomen, das gegebenenfalls durch eine oder mehrere Oxo-Gruppen oder Gruppen R⁷-O- substituiert ist
oder
R¹ und R² bilden zusammen einen aliphatischen Ring, der insgesamt 4 bis 10 Kohlenstoffatome und 0 oder 1 Sauerstoffatom umfasst, wobei der durch R¹ und R² dargestellte Ringbestandteil gegebenenfalls durch eine oder mehrere Oxo-Gruppen und/oder Gruppen R⁷-O- und/oder aliphatische Reste mit 1 bis 4 C-Atomen substituiert ist und
R³ bedeutet Wasserstoff oder ein Alkyl mit 1, 2, 3, 4 oder 5 C-Atomen, das gegebenenfalls durch eine oder mehrere Oxo-Gruppen oder Gruppen R⁷-O- substituiert ist
oder
R² und R³ bilden zusammen einen aliphatischen Ring, der insgesamt 4 bis 10 Kohlenstoffatome und 0 oder 1 Sauerstoffatom umfasst, wobei der durch R² und R³ dargestellte Ringbestandteil gegebenenfalls durch eine oder mehrere Oxo-Gruppen und/oder Gruppen R⁷-O- und/oder aliphatische Reste mit 1 bis 4 C-Atomen substituiert ist und
R¹ bedeutet Wasserstoff, ein aliphatischer Rest mit 1 bis 4 C-Atomen oder eine Gruppe -O-R⁶, -S-R⁵ oder -NR⁵R⁶,
wobei für R⁴ und R gilt:
R⁴ und R bedeuten unabhängig voneinander Wasserstoff, Methyl oder Ethyl und wobei für R⁵, R⁶ und R⁷ gilt:
R⁵, R⁶ und R⁷ bedeuten unabhängig voneinander Wasserstoff oder ein aliphatischer Rest mit 1 bis 4 C-Atomen,
- Mischung umfassend oder bestehend aus zwei oder mehreren verschiedenen Hydroxyphenylalkadionen der Formel (I), wobei X, a, Y, R, R¹, R², R³, R⁴, R⁵, R⁶ und R⁷ jeweils die oben gegebene Bedeutung haben,
- Mischung umfassend oder bestehend aus Salzen zweier oder mehrerer verschiedener Hydroxyphenylalkadionen der Formel (I), wobei X, a, Y, R, R¹, R², R³, R⁴, R⁵, R⁶ und R⁷ jeweils die oben gegebene Bedeutung haben,
oder
- Mischung umfassend oder bestehend aus einem Hydroxyphenylalkadion der Formel (I) oder zwei oder mehreren verschiedenen Hydroxyphenylalkadionen der Formel (I),
wobei X, a, Y, R, R¹, R², R³, R⁴, R⁵, R⁶ und R⁷ jeweils die oben gegebene Bedeutung haben, und einem Salz eines Hydroyphenylalkadions der Formel (I) oder zwei oder mehreren Salzen von verschiedenen Hydroxyphenylalkadionen der Formel (I), wobei X, a, Y, R, R¹, R², R³, R⁴, R⁵, R⁶ und R⁷ jeweils die oben gegebene Bedeutung haben,
und
(b) ein, zwei oder mehrere unangenehm schmeckende Stoffe
und/oder
(c) ein, zwei oder mehrere süß schmeckende Stoffe
und/oder
(d) ein, zwei oder mehrere Aromastoffe, die einen süßen Geruchseindruck vermitteln
und
(e) ein, zwei oder mehrere pharmazeutische wirksame Komponente, die oral verabreicht werden können.

16. Zubereitung nach einem der Ansprüche 14 oder 15, umfassend einen, zwei oder mehrere unangenehm schmeckende Stoffe (b), wobei die Menge des unangenehm schmeckenden Stoffes (b) ausreicht, um in einer Vergleichszubereitung, die keine Verbindung (a) umfasst, aber ansonsten identisch zusammengesetzt ist, als unangenehmer Geschmack wahrgenommen zu werden, und die Menge der Verbindung (a) in der Zubereitung ausreicht, um den unangenehmen Geschmackseindruck des unangenehm schmeckenden Stoffes sensorisch zu maskieren oder zu ändern oder im Vergleich mit der Vergleichszubereitung zu vermindern.

17. Zubereitung nach einem der Ansprüche 14 oder 15, umfassend einen, zwei oder mehrere süß schmeckende Stoffe (c) und/oder einen, zwei oder mehrere Aromastoffe (d), die einen süßen Geruchseindruck vermitteln, wobei die Gesamtmenge der Verbindung (a) in der Zubereitung ausreicht, um den süßen Geschmackseindruck der/des süß schmeckenden Stoffe(s) (c) oder den süßen Geruchseindruck der/des Aromastoffe(s) (d), die/der einen süßen Geruchseindruck vermitteln/vermittelt, überproportional sensorisch zu verstärken.

18. Zubereitung nach einem der Ansprüche 14 oder 15, **dadurch gekennzeichnet, dass** sie als Halbfertigware, als Riech-, Aroma- oder Geschmacksstoffkomposition oder als Würzmischung vorliegt.

19. Zubereitung nach einem der Ansprüche 16 bis 18, weiter umfassend eine oder mehrere weitere Substanz zum Verändern, Maskieren oder Vermindern des unangenehmen Geschmackseindrucks eines unangenehm schmeckenden Stoffes und/oder zum Verstärken des süßen Geschmacks eines süß schmeckenden Stoffes und/oder des süßen Geruchseindrucks eines Aromastoffes, der einen süßen Geruchseindruck vermittelt.

20. 5-(4-Hydroxy-3-methoxyphenyl)pentan-3-onsäure,
5-(4-Hydroxy-3-methoxyphenyl)pentan-3-onsäureethylester,
2-Acetyl-5-(4-hydroxy-3-methoxyphenyl)pentan-3-onsäureethylester und
6-(3-Hydroxy-4-methoxyphenyl)hexan-2,4-dion
und
- Salz eines dieser Hydroxyphenylalkadione,
- Mischung umfassend oder bestehend aus zwei, drei oder vier dieser Hydroxyphenylalkadione,
- Mischung umfassend oder bestehend aus Salzen zweier, dreier oder vierer dieser Hydroxyphenylalkadione,
oder
- Mischung umfassend oder bestehend aus
einem dieser Hydroxyphenylalkadione oder zwei, drei oder vier dieser Hydroxyphenylalkadione und
einem Salz eines dieser Hydroxyphenylalkadione oder zwei, drei oder vier Salzen dieser Hydroxyphenylalkadione.

21. Verfahren zur Veränderung, Maskierung oder Vermindern eines unangenehmen Geschmackseindrucks eines unangenehm schmeckenden Stoffes mit dem folgenden Schritt:
- Mischen eines oder mehrerer unangenehm schmeckender Stoffe (Verbindung (b)) mit einer Gesamtmenge einer Verbindung (a)
- 0,000001 Gew.-% bis 95 Gew.%, bezogen auf das Gesamtgewicht der Zubereitung, eines Hydroxyphenylalkadions der Formel (I) 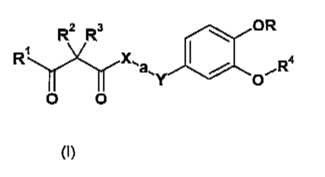 wobei für X, a und Y gilt:
X ist eine Gruppe -CH₂-, -NH- oder -O-,
Y ist eine Gruppe -CH₂- und
a ist eine Einfachbindung
oder
X und Y sind jeweils eine Gruppe -CH- und
a ist eine in Z- oder E-Konfiguration vorliegende Doppelbindung,
wobei für R¹, R² und R³ gilt:
R¹ bedeutet Wasserstoff, ein aliphatischer Rest mit 1 bis 4 C-Atomen oder eine Gruppe -O-R⁵, -S-R⁵ oder -NR⁵R⁶ und
R² und R³ bedeuten unabhängig voneinander Wasserstoff oder ein Alkyl mit 1, 2, 3, 4 oder 5 C-Atomen, das gegebenenfalls durch eine oder mehrere Oxo-Gruppen oder Gruppen R⁷-O- substituiert ist oder
R¹ und R² bilden zusammen einen aliphatischen Ring, der insgesamt 4 bis 10 Kohlenstoffatome und 0 oder 1 Sauerstoffatom umfasst, wobei der durch R¹ und R² dargestellte Ringbestandteil gegebenenfalls durch eine oder mehrere Oxo-Gruppen und/oder Gruppen R⁷-O- und/oder aliphatische Reste mit 1 bis 4 C-Atomen substituiert ist und
R³ bedeutet Wasserstoff oder ein Alkyl mit 1, 2, 3, 4 oder 5 C-Atomen, das gegebenenfalls durch eine oder mehrere Oxo-Gruppen oder Gruppen R⁷-O- substituiert ist
oder
R² und R³ bilden zusammen einen aliphatischen Ring, der insgesamt 4 bis 10 Kohlenstoffatome und 0 oder 1 Sauerstoffatom umfasst, wobei der durch R² und R³ dargestellte Ringbestandteil gegebenenfalls durch eine oder mehrere Oxo-Gruppen und/oder Gruppen R⁷-O- und/oder aliphatische Reste mit 1 bis 4 C-Atomen substituiert ist und
R¹ bedeutet Wasserstoff, ein aliphatischer Rest mit 1 bis 4 C-Atomen oder eine Gruppe -O-R⁵, -S-R⁵ oder -NR⁵R⁶,
wobei für R⁴ und R gilt:
R⁴ und R bedeuten unabhängig voneinander Wasserstoff, Methyl oder Ethyl und wobei für R⁵, R⁶ und R⁷ gilt:
R⁵, R⁶ und R⁷ bedeuten unabhängig voneinander Wasserstoff oder ein aliphatischer Rest mit 1 bis 4 C-Atomen oder,
- Mischung umfassend oder bestehend aus zwei oder mehreren verschiedenen Hydroxyphenylalkadionen der Formel (I), wobei X, a, Y, R, R¹, R², R³, R⁴, R⁵, R⁶ und R⁷ jeweils die oben gegebene Bedeutung haben,
- Mischung umfassend oder bestehend aus Salzen zweier oder mehrerer verschiedener Hydroxyphenylalkadionen der Formel (I), wobei X, a, Y, R, R¹, R², R³, R⁴, R⁵, R⁶ und R⁷ jeweils die oben gegebene Bedeutung haben,
oder
- Mischung umfassend oder bestehend aus einem Hydroxyphenylalkadion der Formel (I) oder zwei oder mehreren verschiedenen Hydroxyphenylalkadionen der Formel (I), wobei X, a, Y, R, R¹, R², R³, R⁴, R⁵, R⁶ und R⁷ jeweils die oben gegebene Bedeutung haben, und einem Salz eines Hydroyphenylalkadions der Formel (I) oder zwei oder mehreren Salzen von verschiedenen Hydroxyphenylalkadionen der Formel (I), wobei X, a, Y, R, R¹, R², R³, R⁴, R⁵, R⁶ und R⁷ jeweils die oben gegebene Bedeutung haben,
wobei die Menge der Verbindung (b) in der Zubereitung ausreicht, um gegenüber einer Vergleichszubereitung, die keine Verbindung (b) umfasst, aber ansonsten identisch zusammengesetzt ist, als unangenehmer Geschmack wahrgenommen zu werden, und wobei die Menge der Verbindung (a) in der Zubereitung ausreicht, um den unangenehmen Geschmackseindruck des unangenehm schmeckenden Stoffes (b) sensorisch zu maskieren oder im Vergleich mit der Vergleichszubereitung zu vermindern.

22. Verfahren zur Verstärkung des süßen Geschmacks eines süß schmeckenden Stoffes oder des süßen Geruchseindrucks eines Aromastoffes, der einen süßen Geruchseindruck vermittelt, mit dem folgenden Schritt:
- Mischen eines oder mehrerer süß schmeckender Stoffe (Verbindung (c)) oder eines oder mehrerer Aromastoffe (Verbindung (d)), der/die einen süßen Geruchseindruck vermittelt/vermitteln, mit einer Gesamtmenge einer Verbindung (a)
- 0,000001 Gew.% bis 95 Gew.-%, bezogen auf das Gesamtgewicht der Zubereitung, eines Hydroxyphenylalkadions der Formel (I) 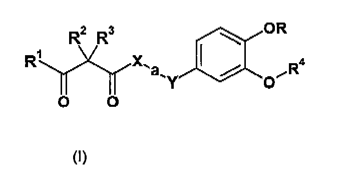 wobei für X, a und Y gilt:
X ist eine Gruppe -CH₂-, -NH- oder -O-,
Y ist eine Gruppe -CH₂- und
a ist eine Einfachbindung
oder
X und Y sind jeweils eine Gruppe -CH- und
a ist eine in Z- oder E-Konfiguration vorliegende Doppelbindung,
wobei für R¹, R² und R³ gilt:
R¹ bedeutet Wasserstoff, ein aliphatischer Rest mit 1 bis 4 C-Atomen oder eine Gruppe -O-R⁵, -S-R⁵ oder -NR⁵R⁶ und
R² und R³ bedeuten unabhängig voneinander Wasserstoff oder ein Alkyl mit 1, 2, 3, 4 oder 5 C-Atomen, das gegebenenfalls durch eine oder mehrere Oxo-Gruppen oder Gruppen R⁷-O- substituiert ist
oder
R¹ und R² bilden zusammen einen aliphatischen Ring, der insgesamt 4 bis 10 Kohlenstoffatome und 0 oder 1 Sauerstoffatom umfasst, wobei der durch R¹ und R² dargestellte Ringbestandteil gegebenenfalls durch eine oder mehrere Oxo-Gruppen und/oder Gruppen R⁷-O- und/oder aliphatische Reste mit 1 bis 4 C-Atomen substituiert ist und
R³ bedeutet Wasserstoff oder ein Alkyl mit 1, 2, 3, 4 oder 5 C-Atomen, das gegebenenfalls durch eine oder mehrere Oxo-Gruppen oder Gruppen R⁷-O- substituiert ist
oder
R² und R³ bilden zusammen einen aliphatischen Ring, der insgesamt 4 bis 10 Kohlenstoffatome und 0 oder 1 Sauerstoffatom umfasst, wobei der durch R² und R³ dargestellte Ringbestandteil gegebenenfalls durch eine oder mehrere Oxo-Gruppen und/oder Gruppen R⁷-O- und/oder aliphatische Reste mit 1 bis 4 C-Atomen substituiert ist und
R¹ bedeutet Wasserstoff, ein aliphatischer Rest mit 1 bis 4 C-Atomen oder eine Gruppe -O-R⁵, -S-R⁵ oder -NR⁵R⁶,
wobei für R⁴ und R gilt:
R⁴ und R bedeuten unabhängig voneinander Wasserstoff, Methyl oder Ethyl und wobei für R⁵, R⁶ und R⁷ gilt:
R⁵, R⁶ und R⁷ bedeuten unabhängig voneinander Wasserstoff oder ein aliphatischer Rest mit 1 bis 4 C-Atomen oder,
- Mischung umfassend oder bestehend aus zwei oder mehreren verschiedenen Hydroxyphenylalkadionen der Formel (I), wobei X, a, Y, R, R¹, R², R³, R⁴, R⁵, R⁶ und R⁷ jeweils die oben gegebene Bedeutung haben,
- Mischung umfassend oder bestehend aus Salzen zweier oder mehrerer verschiedener Hydroxyphenylalkadionen der Formel (I), wobei X, a, Y, R, R¹, R², R³, R⁴, R⁵, R⁶ und R⁷ jeweils die oben gegebene Bedeutung haben,
oder
- Mischung umfassend oder bestehend aus einem Hydroxyphenylaikadion der Formel (I) oder zwei oder mehreren verschiedenen Hydroxyphenylaikadionen der Formel (I), wobei X, a, Y, R, R¹, R², R³, R⁴, R⁵, R⁶ und R⁷ jeweils die oben gegebene Bedeutung haben, und einem Salz eines Hydroyphenylalkadions der Formel (I) oder zwei oder mehreren Salzen von verschiedenen Hydroxyphenylalkadionen der Formel (I), wobei X, a, Y, R, R¹, R², R³, R⁴, R⁵, R⁶ und R⁷ jeweils die oben gegebene Bedeutung haben,
wobei die Gesamtmenge der Verbindung (a) in der Zubereitung ausreicht, um den süßen Geschmackseindruck der/des süß schmeckenden Stoffe(s) (c) oder den süßen Geruchseindruck der/des Aromastoffe(s) (d), die/der einen süßen Geruchseindruck vermitteln/vermittelt, sensorisch zu verstärken.

## Revendications

1. Utilisation
- d'un dérivé d'hydroxyphénylalcanedione de formule (I) 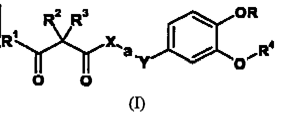 dans laquelle, pour X, a et Y:
X est un groupe -CH₂-, -NH- ou -O-,
Y est un groupe -CH₂-, et
a est une liaison simple,
ou
X et Y sont dans chaque cas un groupe -CH- et
a est une double liaison dans la configuration Z ou E, pour R¹, R² et R³:
R¹ désigne un atome d'hydrogène, un radical aliphatique de 1 à 4 atomes de carbone ou un groupe -O-R⁵, -S-R⁵ ou -NR⁵R⁶ et
R² et R³ désignent, indépendamment l'un de l'autre, un atome d'hydrogène ou un groupe alkyle ayant 1, 2, 3, 4 ou 5 atomes de carbone, qui est éventuellement substitué par un ou plusieurs groupes oxo ou groupes R⁷-O-
ou
R¹ et R² forment ensemble un cycle aliphatique qui contient en tout 4 à 10 atomes de carbone et 0 ou 1 atome d'oxygène, le constituant cyclique représenté par R¹ et R² étant éventuellement substitué par un ou plusieurs groupes oxo et/ou groupes R⁷-O- et/ou radicaux aliphatiques de 1 à 4 atomes de carbone, et
R³ désigne un atome d'hydrogène ou un groupe alkyle de 1, 2, 3, 4, ou 5 atomes de carbone qui est éventuellement substitué par un ou plusieurs groupes oxo ou groupes R⁷-O-,
ou
R² et R³ forment ensemble un cycle aliphatique qui contient en tout 4 à 10 atomes de carbone et 0 ou 1 atome d'oxygène, le constituant cyclique représenté par R² et R³ étant éventuellement substitué par un ou plusieurs groupes oxo et/ou groupes R⁷-O- et/ou radicaux aliphatiques de 1 à 4 atomes de carbone, et
R¹ désigne un atome d'hydrogène ou un radical aliphatique de 1 à 4 atomes de carbone ou un groupe -O-R⁵, -S-R⁵ ou -NR⁵R⁶,
pour R⁴ et R:
R⁴ et R désignent indépendamment l'un de l'autre un atome d'hydrogène, un groupe méthyle ou éthyle,
et, pour R⁵, R⁶ et R⁷:
R⁵, R⁶ et R⁷, indépendamment les uns des autres, désignent un atome d'hydrogène ou un radical aliphatique de 1 à 4 atomes de carbone,
- d'un sel d'une telle hydroxyphénylalcanedione de formule (I),
- d'un mélange comprenant, ou constitué par, au moins deux hydroxyphénylalcanediones de formule (I) différentes, dans lesquelles X, a, Y, R, R¹, R², R³, R⁴, R⁵, R⁶ et R⁷ ont dans chaque cas la signification donnée ci-dessus,
- d'un mélange comprenant, ou constitué par, des sels d'au moins deux hydroxyphénylalcanediones de formule (I) différentes, dans lesquelles X, a, Y, R, R¹, R², R³, R⁴, R⁵, R⁶ et R⁷ ont dans chaque cas la signification donnée ci-dessus,
ou
- d'un mélange comprenant, ou constitué par, une hydroxyphénylalcanedione de formule (I) ou au moins deux hydroxyphénylalcanediones de formule (I) différentes,
dans lesquelles X, a, Y, R, R¹, R², R³, R⁴, R⁵, R⁶ et R⁷ ont dans chaque cas la signification donnée ci-dessus, et un sel d'une hydroxyphénylalcanedione de formule (I) ou au moins deux sels d'hydroxyphénylalcanediones de formule (I) différentes
dans lesquelles X, a, Y, R, R¹, R², R³, R⁴, R⁵, R⁶ et R⁷ ont dans chaque cas la signification donnée ci-dessus,
pour la modification, le masquage ou la réduction de l'impression de goût désagréable d'une substance au goût désagréable, et/ou
pour l'intensification du goût sucré d'une substance au goût sucré et/ou de l'impression d'odeur sucrée d'une substance aromatique qui donne une impression d'odeur sucrée.

2. Utilisation selon la revendication 1,
dans laquelle, pour R⁴ et R:
R⁴ et R désignent indépendamment l'un de l'autre un atome d'hydrogène, un groupe méthyle ou éthyle, l'un des radicaux R⁴ ou R désignant un atome d'hydrogène ou les deux radicaux R⁴ et R désignant un atome d'hydrogène.

3. Utilisation selon la revendication 1 ou 2, dans laquelle R désigne un atome d'hydrogène.

4. Utilisation selon l'une des revendications précédentes, dans laquelle, un, plusieurs ou la totalité des radicaux aliphatiques de 1 à 4 atomes de carbone qui sont éventuellement présents en tant que R¹, R⁵, R⁶ et/ou R⁷ désignent des radicaux méthyle, éthyle, 1-propyle ou 2-propyle.

5. Utilisation selon l'une des revendications précédentes, dans laquelle l'alkyle de 1, 2, 3, 4 ou 5 atomes de carbone qui est éventuellement substitué par un
ou plusieurs groupes oxo ou groupes R⁷-O- est choisi dans le groupe constitué par: méthyle, éthyle, 1-propyle, 2-propyle, 1-oxométhyle, 1-oxoéthyle, 1-méthyloxyméthyle, 1-méthyloxyéthyle, 1-oxopropyle.

6. Utilisation selon l'une des revendications précédentes, dans laquelle R¹ et R² forment ensemble un cycle aliphatique de cinq, six ou sept chaînons en tout et contenant 4 à 7 atomes de carbone et 0 ou 1 atome d'oxygène, le constituant cyclique représenté par R¹ et R² étant éventuellement substitué par un ou plusieurs groupes oxo et/ou groupes R⁷-O- et/ou des radicaux aliphatiques de 1 à 4 atomes de carbone.

7. Utilisation selon l'une des revendications précédentes, dans laquelle, pour X, a et Y:
X est un groupe -CH₂- ou -NH-,
Y est un groupe -CH₂-, et
a est une simple liaison,
ou
X et Y sont dans chaque cas un groupe -CH- et
a est une double liaison en configuration E,
pour R¹, R² et R³:
R¹ désigne un radical aliphatique de 1 à 4 atomes de carbone ou un groupe -O-R⁵,
R² désigne un atome d'hydrogène ou un groupe alkyle de 1, 2, 3, 4 ou 5 atomes de carbone qui est éventuellement substitué par un ou plusieurs groupes oxo ou groupes R⁷-O-, et
R³ désigne un atome d'hydrogène,
R¹ et R² forment ensemble un cycle aliphatique contenant en tout 5 à 7 atomes de carbone et 0 ou 1 atome d'oxygène, le constituant cyclique représenté par R¹ et R² étant éventuellement substitué par un ou plusieurs groupes oxo et/ou groupes R⁷-O-, et
R³ désigne un atome d'hydrogène.
et pour R⁵ et R⁷:
R⁵ et R⁷ indépendamment les uns des autres, désignent un atome d'hydrogène ou un radical aliphatique de 1 à 4 atomes de carbone.

8. Utilisation selon la revendication 7, dans laquelle, pour R¹ et R²:
R¹ désigne un groupe méthyle, éthyle, 1-propyle, 2-propyle ou un groupe -O-R⁵, et
R² désigne un atome d'hydrogène ou un groupe alkyle de 1, 2, 3, 4 ou 5 atomes de carbone qui est éventuellement substitué par un ou plusieurs groupes oxo,
ou
R¹ et R² forment ensemble un cycle aliphatique contenant en tout 5 à 7 atomes de carbone et 0 ou 1 atome d'oxygène, le constituant cyclique représenté par R¹ et R² étant éventuellement substitué par un ou plusieurs groupes oxo,
et, pour R⁵:
R⁵ désigne un atome d'hydrogène ou un groupe méthyle ou éthyle.

9. Utilisation selon l'une des revendications précédentes
- d'une hydroxyphénylalcanedione de formule (I) choisie dans le groupe constitué par
la (5E)-6-(4-hydroxy-3-méthoxyphényl)hex-5-ène-2,4-dione,
la (6E)-7-(4-hydroxy-3-méthoxyphényl)hept-6-éne-3,5-dione,
la (5E)-6-(3,4-dihydroxyphényl)hex-5-éne-2,4-dione,
la 6-(4-hydroxy-3-méthoxyphényl)hexane-2,4-dione,
la 7-(4-hydroxy-3-méthoxyphényl)heptane-3,5-dione,
l'acide 5-(4-hydroxy-3-méthoxyphényl)pentan-3-onoïque,
le 5-(4-hydroxy-3-méthoxyphényl)pentan-3-onoate d'éthyle,
le 2-acétyl-5-(4-hydroxy-3-méthoxyphényl)pentan-3-onoate d'éthyle,
la 2-(3-[4-hydroxy-3-méthoxyphényl]propionyl)-cyclopentane-1,3-dione,
la (5E)-6-(3-hydroxy-4-méthoxyphényl)hex-5-ène-2,4-dione et
la 6-(3-hydroxy-4-méthoxyphényl)hexane-2,4-dione;
- d'un sel d'une hydroxyphénylalcanedione de formule (I) choisie dans ledit groupe,
- d'un mélange comprenant, ou constitué par, au moins deux hydroxyphénylalcanediones de formule (I) choisies dans ledit groupe,
- d'un mélange comprenant, ou constitué par, des sels d'au moins deux hydroxyphénylalcanediones de formule (I) choisies dans ledit groupe, ou
- d'un mélange comprenant, ou constitué par, une hydroxyphénylalcanedione de formule (I) choisie dans ledit groupe ou au moins deux hydroxyphénylalcanediones de formule (I) choisies dans ledit groupe et un sel d'une hydroxyphénylalcanedione de formule (I) choisie dans ledit groupe ou au moins deux sels d'hydroxyphénylalcanediones de formule (I) choisies dans ledit groupe.

10. Utilisation d'un sel d'un composé de formule (I) selon l'une des revendications précédentes, dans laquelle un, plusieurs ou la totalité des groupes hydroxyle du composé de formule (1) sont déprotonés et une quantité correspondante de contre-cations est présente, ces contre-cations étant choisis dans le groupe constitué par des cations à une seule charge positive du premier groupe principal et secondaire, des ions ammonium, des ions trialkylammonium, des cations à deux charges positives du deuxième groupe principal et secondaire et des cations à trois charges positives du troisième groupe principal et secondaire, et leurs mélanges.

11. Utilisation selon l'une des revendications précédentes, en combinaison avec une ou au moins deux substances au goût désagréable.

12. Utilisation selon l'une des revendications précédentes, en combinaison avec une ou au moins deux substances au goût sucré ou une, deux et/ou plusieurs substances aromatiques qui donnent une impression d'odeur sucrée.

13. Utilisation selon l'une des revendications précédentes dans une formulation destinée à l'alimentation, à des soins oraux ou à la consommation de plaisir ou à une formulation pharmaceutique orale ou à une formulation cosmétique à appliquer dans la région de la tête.

14. Formulation choisie dans le groupe constitué par des formulations destinées à l'alimentation, à des soins oraux ou à la consommation de plaisir ou à une formulation cosmétique à appliquer dans la région de la tête, comprenant les constituants suivants:
(a)
- 0,000001 % en masse à 95 % en masse, par rapport à la masse totale de la formulation, d'une hydroxyphénylalcanedione de formule (1) 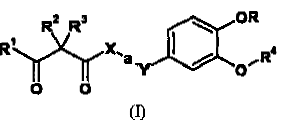 dans laquelle, pour X, a et Y:
X est un groupe -CH₂-, -NH- ou -O-,
Y est un groupe -CH₂-, et
a est une liaison simple,
ou
X et Y sont dans chaque cas un groupe -CH- et
a est une double liaison dans la configuration Z ou E,
pour R¹, R² et R³:
R¹ désigne un atome d'hydrogène, un radical aliphatique de 1 à 4 atomes de carbone ou un groupe -O-R⁵, -S-R⁵ ou -NR⁵R⁶ et
R² et R³ désignent, indépendamment l'un de l'autre, un atome d'hydrogène ou un groupe alkyle ayant 1, 2, 3, 4 ou 5 atomes de carbone, qui est éventuellement substitué par un ou plusieurs groupes oxo ou groupes R⁷-O-
ou
R¹ et R² forment ensemble un cycle aliphatique qui contient en tout 4 à 10 atomes de carbone et 0 ou 1 atome d'oxygène, le constituant cyclique représenté par R¹ et R² étant éventuellement substitué par un ou plusieurs groupes oxo et/ou groupes R⁷-O- et/ou radicaux aliphatiques de 1 à 4 atomes de carbone, et
R³ désigne un atome d'hydrogène ou un groupe alkyle de 1, 2, 3, 4, ou 5 atomes de carbone qui est éventuellement substitué par un ou plusieurs groupes oxo ou groupes R⁷-O-,
ou
R² et R³ forment ensemble un cycle aliphatique qui contient en tout 4 à 10 atomes de carbone et 0 ou 1 atome d'oxygène, le constituant cyclique représenté par R² et R³ étant éventuellement substitué par un ou plusieurs groupes oxo et/ou groupes R⁷-O- et/ou radicaux aliphatiques de 1 à 4 atomes de carbone, et
R¹ désigne un atome d'hydrogène ou un radical aliphatique de 1 à 4 atomes de carbone ou un groupe -O-R⁵, -S-R⁵ ou -NR⁵R⁶,
pour R⁴ et R:
R⁴ et R désignent indépendamment l'un de l'autre un atome d'hydrogène, un groupe méthyle ou éthyle,
et, pour R⁵, R⁶ et R⁷:
R⁵, R⁶ et R⁷, indépendamment les uns des autres, désignent un atome d'hydrogène ou un radical aliphatique de 1 à 4 atomes de carbone,
- d'un mélange comprenant, ou constitué par, au moins deux hydroxyphénylalcanediones de formule (I) différentes, dans lesquelles X, a, Y, R, R¹, R², R³, R⁴, R⁵, R⁶ et R⁷ ont dans chaque cas la signification donnée ci-dessus,
- d'un mélange comprenant, ou constitué par, des sels d'au moins deux hydroxyphénylalcanediones de formule (I) différentes, dans lesquelles X, a, Y, R, R¹, R², R³, R⁴, R⁵, R⁶ et R⁷ ont dans chaque cas la signification donnée ci-dessus,
ou
- d'un mélange comprenant, ou constitué par, une hydroxyphénylalcanedione de formule (I) ou au moins deux hydroxyphénylalcanediones de formule (I) différentes, dans lesquelles X, a, Y, R, R¹, R², R³, R⁴, R⁵, R⁶ et R⁷ ont dans chaque cas la signification donnée ci-dessus, et un sel d'une hydroxyphénylalcanedione de formule (I) ou au moins deux sels d'hydroxyphénylalcanediones différentes dans lesquelles X, a, Y, R, R¹, R², R³, R⁴, R⁵, R⁶ et R⁷ ont dans chaque cas la signification donnée ci-dessus, et
(b) une ou au moins deux substances au goût désagréable, et/ou
(c) une ou au moins deux autres substances au goût sucré, et/ou
(d) une ou au moins deux substances aromatiques qui donnent une impression d'odeur sucrée.

15. Compositions pharmaceutiques orales, comprenant
(a)
- 0,000001 % en masse à 10 % en masse, par rapport à la masse totale de la formulation, d'une hydroxyphénylalcanedione de formule (I) 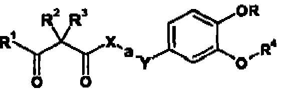 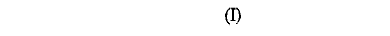 dans laquelle, pour X, a et Y:
X est un groupe -CH₂-, -NH- ou -O-,
Y est un groupe -CH₂-, et
a est une liaison simple,
ou
X et Y sont dans chaque cas un groupe -CH- et
a est une double liaison dans la configuration, Z ou E,
pour R¹, R² et R³:
R¹ désigne un atome d'hydrogène, un radical aliphatique de 1 à atomes de carbone ou un groupe -O-R⁵, -S-R⁵ ou -NR⁵R⁶ et
R² et R³ désignent, indépendamment l'un de l'autre, un atome d'hydrogène ou un groupe alkyle ayant 1, 2, 3, 4 ou 5 atomes de carbone, qui est éventuellement substitué par un ou plusieurs groupes oxo ou groupes R⁷-O-
ou
R¹ et R² forment ensemble un cycle aliphatique qui contient en tout 4 à 10 atomes de carbone et 0 ou 1 atome d'oxygène, le constituant cyclique représenté par R¹ et R² étant éventuellement substitué par un ou plusieurs groupes oxo et/ou groupes R⁷-O- et/ou radicaux aliphatiques de 1 à 4 atomes de carbone, et
R³ désigne un atome d'hydrogène ou un groupe alkyle de 1, 2, 3, 4, ou 5 atomes de carbone qui est éventuellement substitué par un ou plusieurs groupes oxo ou groupes R⁷-O-,
ou
R² et R³ forment ensemble un cycle aliphatique qui contient en tout 4 à 10 atomes de carbone et 0 ou 1 atome d'oxygène, le constituant cyclique représenté par R² et R³ étant éventuellement substitué par un ou plusieurs groupes oxo et/ou groupes R⁷-O- et/ou radicaux aliphatiques de 1 à 4 atomes de carbone, et
R¹ désigne un atome d'hydrogène ou un radical aliphatique de 1 à 4 atomes de carbone ou un groupe -O-R⁵, -S-R⁵ ou -NR⁵R⁶,
pour R⁴ et R:
R⁴ et R désignent indépendamment l'un de l'autre un atome d'hydrogène, un groupe méthyle ou éthyle,
et, pour R⁵, R⁶ et R⁷:
R⁵, R⁶ et R⁷, indépendamment les uns des autres, désignent un atome d'hydroèéne ou un radical aliphatique de 1 à 4 atomes de carbone,
- d'un mélange comprenant, ou constitué par, au moins deux hydroxyphénylalcanediones de formule (I) différentes, dans lesquelles X, a, Y, R, R¹, R², R³, R⁴, R⁵, R⁶ et R⁷ ont dans chaque cas la signification donnée ci-dessus,
- d'un mélange comprenant, ou constitué par, des sels d'au moins deux hydroxyphénylalcanediones de formule (1) différentes, dans lesquelles X, a, Y, R, R¹, R², R³, R⁴, R⁵, R⁶ et R⁷ ont dans chaque cas la signification donnée ci-dessus,
ou
- d'un mélange comprenant, ou constitué par, une hydroxyphénylalcanedione de formule (I) ou au moins deux hydroxyphénylalcanediones de formule (I) différentes, dans lesquelles X, a, Y, R, R¹, R², R³, R⁴, R⁵, R⁶ et R⁷ ont dans chaque cas la signification donnée ci-dessus, et un sel d'une hydroxyphénylalcanedione de formule (I) ou au moins deux sels d'hydroxyphénylalcanediones différentes dans lesquelles X, a, Y, R, R¹, R², R³, R⁴, R⁵, R⁶ et R⁷ ont dans chaque cas la signification donnée ci-dessus, et
(b) une ou au moins deux substances au goût désagréable, et/ou
(c) une ou au moins deux autres substances au goût sucré, et/ou
(d) une ou au moins deux substances aromatiques qui donnent une impression d'odeur sucrée, et
(e) un ou au moins deux composés actifs pharmaceutiques pouvant être administrés par voie orale.

16. Formulation selon la revendication 14 ou 15, comprenant une ou au moins deux substances au goût désagréable (b), la quantité de substance au goût désagréable (b) étant suffisante pour être perçue comme goût désagréable dans une formule comparative qui ne comprend pas de constituant (a), mais qui a par ailleurs une composition identique, et le constituant (a) dans la formulation étant suffisant pour masquer sur le plan sensoriel ou modifier l'impression de goût désagréable de la substance au goût désagréable ou pour la réduire par comparaison à la formulation comparative.

17. Formulation selon la revendication 14 ou 15, comprenant une ou au moins deux substances au goût sucré (c) et/ou une ou au moins deux substances aromatiques (d) qui donnent une impression d'odeur sucrée, la quantité totale de constituant (a) dans la formulation étant suffisante pour intensifier de façon surproportionnée sur le plan sensoriel l'impression de goût sucré de la substance ou des substances au goût sucré (c) ou l'impression d'odeur sucrée de la substance ou des substances (d) donnant une impression d'odeur sucrée.

18. Formulation selon la revendication 14 ou 15, **caractérisée en ce qu'**elle est sous forme de produit semi-fini, sous forme d'une composition de substances odorantes, aromatiques ou aromatisants ou sous forme d'un mélange d'épices.

19. Formulation selon l'une des revendications 16 à 18, comprenant en outre une ou plusieurs autres substances destinées à modifier, masquer ou réduire l'impression de goût désagréable d'une substance au goût désagréable et/ou à intensifier le goût sucré d'une substance au goût sucré et/ou l'impression d'odeur sucrée d'une substance aromatique qui donne une impression de goût sucré.

20. Acide 5-(4-hydroxy-3-méthoxyphényl)pentan-3-onoïque,
5-(4-hydroxy-3-méthoxyphényl)pentan-3-onoate d'éthyle,
2-acétyl-5-(4-hydroxy-3-méthoxyphényl)pentan-3-onoate d'éthyle, et
6-(3-hydroxy-4-méthoxyphényl)hexane-2,4-dione;
et
- un sel d'une telle hydroxyphénylalcanedione,
- un mélange comprenant, ou constitué par, 2, 3 ou 4 de telles hydroxyphénylalcanediones,
- un mélange comprenant, ou constitué par, des sels de 2, 3 ou 4 de telles hydroxyphénylalcanediones, ou
- un mélange comprenant, ou constitué par, une telle hydroxyphénylalcanedione ou deux, trois, ou quatre de telles hydroxyphénylalcanediones, et un sel d'une hydroxyphénylalcanedione ou 2, 3, ou 4 sels de telles hydroxyphénylalcanediones.

21. Procédé pour modifier, masquer ou réduire une impression de goût désagréable d'une substance au goût désagréable, comprenant l'étape suivante:
- mélange d'une ou plusieurs substances au goût désagréable (constituant (b)) avec une quantité totale de constituant (a)
- 0,000001 % en masse à 95 % en masse, par rapport à la masse totale de la formulation, d'une hydroxyphénylalcanedione de formule (I) 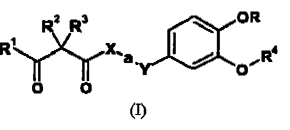 dans laquelle, pour X, a et Y:
X est un groupe -CH₂-, -NH- ou -O-,
Y est un groupe -CH₂-, et
a est une liaison simple,
ou
X et Y sont dans chaque cas un groupe -CH- et
a est une double liaison dans la configuration ou E,
pour R¹, R² et R³:
R¹ désigne un atome d'hydrogène, un radical aliphatique de 1 à 4 atomes de carbone ou un groupe -O-R⁵, -S-R⁵ ou -NR⁵R⁶ et
R² et R³ désignent, indépendamment l'un de l'autre, un atome d'hydrogène ou un groupe alkyle ayant 1, 2, 3, 4 ou 5 atomes de carbone, qui est éventuellement substitué par un ou plusieurs groupes oxo ou groupes R⁷-O-
ou
R¹ et R² forment ensemble un cycle aliphatique qui contient en tout 4 à 10 atomes de carbone et 0 ou 1 atome d'oxygène, le constituant cyclique représenté par R¹ et R² étant éventuellement substitué par un ou plusieurs groupes oxo et/ou groupes R⁷-O- et/ou radicaux aliphatiques de 1 à 4 atomes de carbone, et
R³ désigne un atome d'hydrogène ou un groupe alkyle de 1, 2, 3, 4, ou 5 atomes de carbone qui est éventuellement substitué par un ou plusieurs groupes oxo ou groupes R⁷-O-,
ou
R² et R³ forment ensemble un cycle aliphatique qui contient en tout 4 à 10 atomes de carbone et 0 ou 1 atome d'oxygène, le constituant cyclique représenté par R² et R³ étant éventuellement substitué par un ou plusieurs groupes oxo et/ou groupes R⁷-O- et/ou radicaux aliphatiques de 1 à 4 atomes de carbone, et
R¹ désigne un atome d'hydrogène ou un radical aliphatique de 1 à 4 atomes de carbone ou un groupe -O-R⁵, -S-R⁵ ou -NR⁵R⁶,
pour R⁴ et R:
R⁴ et R désignent indépendamment l'un de l'autre un atome d'hydrogène, un groupe méthyle ou éthyle,
et, pour R⁵, R⁶ et R⁷:
R⁵, R⁶ et R⁷, indépendamment les uns des autres, désignent un atome d'hydrogène ou un radical aliphatique de 1 à 4 atomes de carbone,
- d'un mélange comprenant, ou constitué par, au moins deux hydroxyphénylalcanediones de formule (I) différentes, dans lesquelles X, a, Y, R, R¹, R², R³, R⁴, R⁵, R⁶ et R⁷ ont dans chaque cas la signification donnée ci-dessus,
- d'un mélange comprenant, ou constitué par, des sels d'au moins deux hydroxyphénylalcanediones de formule (I) différentes, dans lesquelles X, a, Y, R, R¹, R², R³, R⁴, R⁵, R⁶ et R⁷ ont dans chaque cas la signification donnée ci-dessus,
ou
- d'un mélange comprenant, ou constitué par, une hydroxyphénylalcanedione de formule (I) ou au moins deux hydroxyphénylalcanediones de formule (I) différentes, dans lesquelles X, a, Y, R, R¹, R², R³, R⁴, R⁵, R⁶ et R⁷ ont dans chaque cas la signification donnée ci-dessus, et un sel d'une hydroxyphénylalcanedione de formule (I) ou au moins deux sels d'hydroxyphénylalcanediones de formule (I) différentes dans lesquelles X, a, Y, R, R¹, R², R³, R⁴, R⁵, R⁶ et R⁷ ont dans chaque cas la signification donnée ci-dessus,
la quantité de constituant (b) dans la formulation étant suffisante pour être perçue comme un goût désagréable dans une formule comparative qui ne comprend pas de constituant (a), mais qui a par ailleurs une composition identique, et la quantité de constituant (a) dans la formulation étant suffisante pour masquer sur le plan sensoriel
ou modifier l'impression de goût désagréable de la substance au goût désagréable (b) ou pour la réduire par comparaison à la formulation comparative.

22. Procédé pour intensifier le goût sucré d'une substance au goût sucré ou l'impression d'odeur sucrée d'une substance aromatique qui donne une impression d'odeur sucrée, comprenant l'étape suivante:
- mélange d'une ou plusieurs substances au goût sucré (constituant (c)), ou d'une ou plusieurs substances aromatiques (constituant (d)) donnant une impression d'odeur sucrée, avec une quantité totale de constituant (a)
- 0,000001 % en masse à 95 % en masse, par rapport à la masse totale de la formulation, d'une hydroxyphénylalcanedione de formule (I) 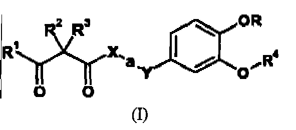
dans laquelle, pour X, a et Y:
X est un groupe -CH₂-, -NH- ou -O-,
Y est un groupe -CH₂-, et
a est une liaison simple,
ou
X et Y sont dans chaque cas un groupe -CH- et
a est une double liaison dans la configuration Z ou E,
dans laquelle, pour R¹, R² et R³,
R¹ désigne un atome d'hydrogène, un radical aliphatique de 1 à 4 atomes de carbone ou un groupe -O-R⁵, -S-R⁵ ou -NR⁵R⁶ et
R² et R³ désignent, indépendamment l'un de l'autre, un atome d'hydrogène ou un groupe alkyle ayant 1, 2, 3, 4 ou 5 atomes de carbone, qui est éventuellement substitué par un ou plusieurs groupes oxo ou groupes R⁷-O-
ou
R¹ et R² forment ensemble un cycle aliphatique qui contient en tout 4 à 10 atomes de carbone et 0 ou 1 atome d'oxygène, le constituant cyclique représenté par R¹ et R² étant éventuellement substitué par un ou plusieurs groupes oxo et/ou groupes R⁷-O- et/ou radicaux aliphatiques de 1 à 4 atomes de carbone, et
R³ désigne un atome d'hydrogène ou un groupe alkyle de 1, 2, 3, 4, ou 5 atomes de carbone qui est éventuellement substitué par un ou plusieurs groupes oxo ou groupes R⁷-O-,
ou
R² et R³ forment ensemble un cycle aliphatique qui contient en tout 4 à 10 atomes de carbone et 0 ou 1 atome d'oxygène, le constituant cyclique représenté par R² et R³ étant éventuellement substitué par un ou plusieurs groupes oxo et/ou groupes R⁷-O- et/ou radicaux aliphatiques de 1 à 4 atomes de carbone, et
R¹ désigne un atome d'hydrogène ou un radical aliphatique de 1 à atomes de carbone ou un groupe -O-R⁵, -S-R⁵ ou -NR⁵R⁶,
pour R⁴ et R:
R⁴ et R désignent indépendamment l'un de l'autre un atome d'hydrogène, un groupe méthyle ou éthyle,
et, pour R⁵, R⁶ et R⁷:
R⁵, R⁶ et R⁷, indépendamment les uns des autres, désignent un atome d'hydrogène ou un radical aliphatique de 1 à atomes de carbone,
- d'un mélange comprenant, ou constitué par, au moins deux hydroxyphénylalcanediones de formule (I) différentes, dans lesquelles X, a, Y, R, R¹, R², R³, R⁴, R⁵, R⁶ et R⁷ ont dans chaque cas la signification donnée ci-dessus,
- d'un mélange comprenant, ou constitué par, des sels d'au moins deux hydroxyphénylalcanediones de formule (I) différentes, dans lesquelles X, a, Y, R, R¹, R², R³, R⁴, R⁵, R⁶ et R⁷ ont dans chaque cas la signification donnée ci-dessus,
ou
- d'un mélange comprenant, ou constitué par, une hydroxyphénylalcanedione de formule (I) ou au moins deux hydroxyphénylalcanedienes de formule (I) différentes, dans lesquelles X, a, Y, R, R¹, R², R³, R⁴, R⁵, R⁶ et R⁷ ont dans chaque cas la signification donnée ci-dessus, et un sel d'une hydroxyphénylalcanedione de formule (I) ou au moins deux sels d'hydroxyphénylalcanediones de formule (I) différentes dans lesquelles X, a, Y, R, R¹, R², R³, R⁴, R⁵, R⁶ et R⁷ ont dans chaque cas la signification donnée ci-dessus,
la quantité totale de constituant (a) dans la formulation étant suffisante pour intensifier sur le plan sensoriel l'impression de goût sucré de la substance ou des substances au goût sucré (c) ou l'impression d'odeur sucrée de la substance ou des substances aromatiques (d) donnant une impression d'odeur sucrée.
